# EUROPEAN PATENT SPECIFICATION

(11) **EP 1 042 279 B1**
(45) Date of publication and mention of the grant of the patent: **02.03.2005**
(21) Application number: 98962586.8
(22) Date of filing: 23.12.1998
(51) Int. Cl.: C07C 275/42, C07D 207/27, A61K 31/17, A61K 31/40

(54) **SUBSTITUTED BETA-ALANINES**
SUBSTITUIERTE BETA-ALANINEN
BETA-ALANINES SUBSTITUEES

(30) Priority: 23.12.1997 GB 9727532; 13.07.1998 US 92602 P
(43) Date of publication of application: 11.10.2000
(73) Proprietor: Aventis Pharma Limited, West Malling, Kent ME19 4AH (GB)
(72) Inventor: ASTLES, Peter, Charles, Dagenham, Essex RM10 7XS (GB); HARRIS, Neil, Victor, Dagenham, Essex RM10 7XS (GB); MORLEY, Andrew, David, Dagenham, Essex RM10 7XS (GB)
(74) Representative: Jones, Stephen Anthony
(86) International application number: PCT/GB1998/003859
(87) International publication number: WO 1999/033789

(56) References cited:
- WO-A-97/36859
- WO-A-97/36862

## Description

This invention is directed to substituted β-alanines. their preparation, pharmaceutical compositions containing these compounds, and their pharmaceutical use in the treatment of disease states capable of being modulated by the inhibition of cell adhesion.

Cell adhesion is a process by which cells associate with each other, migrate towards a specific target or localise within the extra-cellular matrix. Many of the cell-cell and cell-extracellular matrix interactions are mediated by protein ligands (e.g. fibronectin, vitronectin and VCAM-1) and their integrin receptors [e.g. VLA-4 (α₄β₁)]. Recent studies have shown these interactions to play an important part in many physiological (e.g. embryonic development and wound healing) and pathological conditions (e.g. tumour-cell invasion and metastasis, inflammation, atherosclerosis and autoimmune disease).

A wide variety of proteins serve as ligands for integrin receptors. In general, the proteins recognised by integrins fall into one of three classes: extracellular matrix proteins, plasma proteins and cell surface proteins. Extracellular matrix proteins such as collagen fibronectin, fibrinogen, laminin, thrombospondin and vitronectin bind to a number of integrins. Many of the adhesive proteins also circulate in plasma and bind to activated blood cells. Additional components in plasma that are ligands for integrins include fibrinogen and factor X. Cell bound complement C3bi and several transmembrane proteins, such as Ig-like cell adhesion molecule (ICAM-1,2,3) and vascular cell adhesion molecule (VCAM-1), which are members of the Ig superfamily, also serve as cell-surface ligands for some integrins.

Integrins are heterodimeric cell surface receptors consisting of two subunits called α and β. There are at least twelve different α-subunits (α1-α6, α-L, α-M, α-X, α-IIb, α-V and α-E) and at least nine different β (β1-β9) subunits. The integrin family can be subdivided into classes based on the β subunits, which can be associated with one or more α-subunits. The most widely distributed integrins belong to the β1 class, also known as the very late antigens (VLA). The second class of integrins are leukocyte specific receptors and consist of one of three α-subunits (α-L, α-M or α-X) complexed with the β2 protein. The cytoadhesins α-IIbβ3 and α-Vβ3. constitute the third class of integrins.

The present invention principally relates to agents which modulate the interaction of the ligand VCAM-1 with its integrin receptor α4β1 (VLA-4), which is expressed on numerous hematopoietic cells and established cell lines, including hematopoietic precursors, peripheral and cytotoxic T lymphocytes, B lymphocytes, monocytes, thymocytes and eosinophils.

The integrin α4β1 mediates both cell-cell and cell-matrix interactions. Cells expressing α4β1 bind to the carboxy-terminal cell binding domain of the extracellular matrix protein fibronectin, to the cytokine-inducible endothelial cell surface protein VCAM-1, and to each other to promote homotypic aggregation. The expression of VCAM-1 by endothelial cells is upregulated by proinflammatory cytokines such as INF-γ, TNF-α and LI-1β.

Regulation of α4β1 mediated cell adhesion is important in numerous physiological processes, including T-cell proliferation, B-cell localisation to germinal centres, and adhesion of activated T-cells and eosinophils to endothelial cells. Evidence for the involvement of VLA-4/VCAM-1 interaction in various disease processes such as melanoma cell division in metastasis, T-cell infiltration of synovial membranes in rheumatoid arthritis, autoimmune diabetes, collitis and leukocyte penetration of the blood-brain barrier in experimental autoimmune encephalomyelitis, atherosclerosis, peripheral vascular disease, cardiovascular disease and multiple sclerosis, has been accumulated by investigating the role of the peptide CS-1 (the variable region of fibronectin to which α4β1 binds via the sequence Leu-Asp-Val) and antibodies specific for VLA-4 or VCAM-1 in various in vitro and in vivo experimental models of inflammation. For example, in a Streptococcal cell wall-induced experimental model of arthritis in rats, intravenous administration of CS-1 at the initiation of arthritis suppresses both acute and chronic inflammation (S.M.Wahl et al., J.Clin.Invest., 1994, 94, pages 655-662). In the oxazalone-sensitised model of inflammation (contact hypersensitivity response) in mice, intravenous administration of anti-α4 specific monoclonal antibodies significantly inhibited (50-60 % reduction in the ear swelling response) the efferent response (P.L.Chisholm et al. J.Immunol., 1993, 23, pages 682-688).

WO 96/22966 discloses certain compounds that are said to be useful for inhibition and prevention of cell adhesion and cell adhesion-mediated pathologies.

We have now found a group of substituted β-alanines which have valuable pharmaceutical properties, in particular the ability to regulate the interaction of VCAM-1 and fibronectin with the integrin VLA-4 (α4β1).

Thus, in one aspect, the present invention is directed to compounds of general formula (I):- 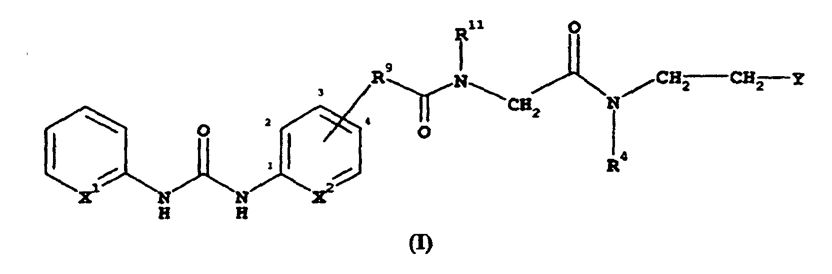 in which :
R⁴ is aryl or heteroaryl, or R⁴ is alkyl, alkenyl, alkynyl each optionally substituted by one or more groups chosen from halo, oxo, R⁵, -C(=O)-R⁷, -NH-C(=O)-R⁷or -C(=O)NY¹Y², or R⁴ is cycloalkenyl, cycloalkyl or heterocycloalkyl each optionally substituted by one or more groups chosen from oxo, R⁶ or -L²-R⁶ {where R⁵ is a carboxyl (or corresponding protected derivative), aryl, cycloalkyl, cycloalkenyl, heteroaryl, heterocycloalkyl, -ZR⁷ or -NY¹Y²; R⁶ is a carboxyl (or corresponding protected derivative), aryl, heteroaryl, heterocycloalkyl, -ZH, -Z¹R⁷ or -NY¹Y²; R⁷ is alkyl, aryl, arylalkyl, cycloalkyl, cycloalkylalkyl, heteroaryl, heteroarylalkyl, heterocycloalkyl or heterocycloalkylalkyl; L² is alkylene; Y¹ and Y² are independently hydrogen, acyl, alkyl [optionally substituted by hydroxy, heterocycloalkyl, or one or more carboxy or -C(=O)-NHR⁸ groups (where R⁸ is hydrogen or C₁₋₄alkyl)], alkylsulphonyl, aryl, arylalkyloxycarbonyl, arylsulphonyl, arylalkyl, heteroaryl, heteroarylalkyl, heterocycloalkyl or heterocycloalkylalkyl; or the group -NY¹Y² may form a 5-7 membered cyclic amine which (i) may be optionally substituted with one or more substituents selected from carboxamido, carboxy, hydroxy, oxo, hydroxyalkyl, HOCH₂CH₂-(OCH₂CH₂)ₘ- (where m is zero, or an integer one or two), or alkyl optionally substituted by carboxy or carboxamido (ii) may also contain a further heteroatom selected from O, N, S or SO₂ and (iii) may also be fused to additional aromatic, heteroaromatic, heterocycloalkyl or cycloalkyl rings to form a bicyclic or tricyclic ring system; Z is O or S; and Z¹ is O or S(O)ₘ};
R⁹ is a straight or branched C₁₋₆alkylene chain, a straight or branched C₂₋₆alkenylene chain or a straight or branched C₂₋₆alkynylene chain;
R¹¹ is a hydrogen atom or a group R⁴;
Y is carboxy;
X¹ and X² each independently represent CR² (wherein R² is hydrogen, halogen, C₁₋₄alkyl or C₁₋₄alkoxy);
and -R⁹-CON(R¹¹)-CH₂-CON(R⁴)-CH₂-CH₂-Y is attached at the ring 3 or 4 position;
wherein said aryl and heteroaryl, whether as a group or part of a group, may be optionally substituted by aryl substituents selected from the group consisting of acyl, acylamino, alkoxy, alkoxycarbonyl, alkylsulphinyl, alkylsulphonyl, alkylthio, aroyl, aroylamino, aryl, arylalkyloxy, arylalkyloxycarbonyl, arylalkylthio, aryloxy, aryloxycarbonyl, arylsulphinyl, arylsulphonyl, arylthio, carboxy, cyano, halo, heteroaroyl, heteroaryl, heteroarylalkyloxy, heteroarylamino, heteroaryloxy, hydroxy, nitro, trifluoromethyl, Y³Y⁴N-, Y³Y⁴NCO-, Y³Y⁴NSO₂-(where Y³ and Y⁴ are independently hydrogen, alkyl, aryl, and arylalkyl), Y³Y⁴N-L³-Z²- (where L³ is C₂₋₆alkylene and Z² is O, NR⁸ or S(O)ₘ), alkylC(=O)-Y³N-, alkylSO₂-Y³N- or alkyl optionally substituted with aryl, heteroaryl, hydroxy, or Y³Y⁴N-,
and their ester prodrugs and pharmaceutically acceptable salts, and solvates (e.g.hydrates) of such compounds and their ester prodrugs.

In the present specification, the term "compounds of the invention", and equivalent expressions, are meant to embrace compounds of general formula (I) as hereinbefore described, which expression includes the prodrugs, the pharmaceutically acceptable salts, and the solvates, e.g. hydrates, where the context so permits. Similarly, reference to intermediates, whether or not they themselves are claimed, is meant to embrace their salts, and solvates, where the context so permits. For the sake of clarity, particular instances when the context so permits are sometimes indicated in the text, but these instances are purely illustrative and it is not intended to exclude other instances when the context so permits.

As used above, and throughout the description of the invention, the following terms, unless otherwise indicated, shall be understood to have the following meanings:-
"Patient" includes both human and other mammals.
"Acidic functional group" means a group with a carboxyl group. The "corresponding protected derivatives" are those where the acidic hydrogen atom has been replaced with a suitable protecting group. For suitable protecting groups see T.W. Greene and P.G.M.Wuts in "Protective Groups in Organic Chemistry" John Wiley and Sons, 1991. Exemplary protected derivatives are esters of carboxy groups.
"Acyl" means an H-CO- or alkyl-CO- group in which the alkyl group is as described herein.
"Acylamino" is an acyt-NH- group wherein acyl is as defined herein.
"Alkenyl" means an aliphatic hydrocarbon group containing a carbon-carbon double bond and which may be straight or branched having 2 to 15 carbon atoms in the chain. Preferred alkenyl groups have 2 to 12 carbon atoms in the chain: and more preferably 2 to 4 carbon atoms in the chain. "Branched", as used herein and throughout the text. means that one or more lower alkyl groups such as methyl, ethyl or propyl are attached to a linear chain; here a linear alkenyl chain. "Lower alkenyl" means 2 to 4 carbon atoms in the chain which may be straight or branched. Exemplary alkenyl groups include ethenyl, propenyl. n-butenyl, i-butenyl, 3-methylbut-2-enyl, n-pentenyl, heptenyl, octenyl, and decenyl.
"Alkenylene" means an aliphatic bivalent radical derived from a straight or branched alkenyl group, in which the alkenyl group is as described herein. Exemplary alkenylene radicals include C₂₋₄alkenylene radicals such as vinylene and propylene.
"Alkoxy" means an alkyl-O- group in which the alkyl group is as described herein. Exemplary alkoxy groups include methoxy, ethoxy, n-propoxy, i-propoxy, n-butoxy and heptoxy.
"Alkoxycarbonyl" means an alkyl-O-CO- group in which the alkyl group is as described herein. Exemplary alkoxycarbonyl groups include methoxy- and ethoxycarbonyl.
"Alkyl" means, unless otherwise specified, an aliphatic hydrocarbon group which may be straight or branched having 1 to 15 carbon atoms in the chain optionally substituted by one or more halogen atoms. Particular alkyl groups have from 1 to 6 carbon atoms. "Lower alkyl" as a group or part of a lower alkoxy group means unless otherwise specified, an aliphatic hydrocarbon group which may be straight or branched having 1 to 4 carbon atoms in the chain. Exemplary alkyl groups include methyl, ethyl, n-propyl, i-propyl, n-butyl, s-butyl, t-butyl, n-pentyl, 3-pentyl, heptyl, octyl, nonyl, decyl and dodecyl.
"Alkylene" means an aliphatic bivalent radical derived from a straight or branched alkyl group, in which the alkyl group is as described herein. Exemplary alkylene radicals include C₁₋₄alkylene radicals such as methylene, ethylene and trimethylene.
"Alkylsulphinyl" means an alkyl-SO- group in which the alkyl group is as previously described. Preferred alkylsulphinyl groups are those in which the alkyl group is C₁₋₄alkyl.
"Alkylsulphonyl" means an alkyl-SO₂- group in which the alkyl group is as previously described. Preferred alkylsulphonyl groups are those in which the alkyl group is C₁₋₄alkyl.
"Alkylsulphonylcarbamoyl" means an alkyl-SO₂-NH-C(=O)- group in which the alkyl group is as previously described. Preferred alkylsulphonylcarbamoyl groups are those in which the alkyl group is C₁₋₄alkyl.
"Alkylthio" means an alkyl-S- group in which the alkyl group is as previously described. Exemplary alkylthio groups include methylthio, ethylthio, isopropylthio and heptylthio.
"Alkynyl" means an aliphatic hydrocarbon group containing a carbon-carbon triple bond and which may be straight or branched having 2 to 15 carbon atoms in the chain. Preferred alkynyl groups have 2 to 12 carbon atoms in the chain; and more preferably 2 to 4 carbon atoms in the chain. Excmplary alkynyl groups include ethynyl, propynyl, n-butynyl, i-butynyl, 3-methylbut-2-ynyl, and n-pentynyl.
"Alkynylene" means an aliphatic bivalent radical derived from a straight or branched alkynyl group, in which the alkynyl group is as described herein. Exemplary alkynylene radicals include C₂₋₄alkynylene radicals such as ethynylene and propynylene.
"Aroyl" means an aryl-CO- group in which the aryl group is as described herein. Exemplary aroyl groups include benzoyl and 1- and 2-naphthoyl.
"Aroylamino" is an aroyl-NH- group wherein aroyl is as previously defined.
"Aryl" as a group or part of a group denotes: (i) an optionally substituted monocyclic or multicyclic aromatic carbocyclic moiety of 6 to 14 carbon atoms or (ii) an optionally substituted partially saturated multicyclic aromatic carbocyclic moiety in which an aryl and a cycloalkyl or cycloalkenyl group are fused together to form a cyclic structure such as a tetrahydronaphthyl, indenyl or indanyl ring. When R⁴ contains an optionally substituted aryl group, this may particularly represent optionally substituted phenyl with one or more aryl group substituents which may be the same or different, where "aryl group substituent" are selected from the group consisting of acyl, acylamino, alkoxy, alkoxycarbonyl, alkylsulphinyl, alkylsulphonyl, alkylthio, aroyl, aroylamino, aryl, arylalkyloxy, arylalkyloxycarbonyl, arylalkylthio, aryloxy, aryloxycarbonyl, arylsulphinyl, arylsulphonyl, arylthio, carboxy, cyano, halo, heteroaroyl, heteroaryl, heteroarylalkyloxy, heteroarylamino, heteroaryloxy, hydroxy, nitro, trifluoromethyl, Y³Y⁴N-, Y³Y⁴NCO-, Y³Y⁴NSO₂-(where Y³ and Y⁴ are independently hydrogen, alkyl, aryl, and arylalkyl), Y³Y⁴N-L³-Z²- (where L³ is C₂₋₆alkylene and Z² is O, NR⁸ or S(O)m), alkylC(=O)-Y³N-, alkylSO₂-Y³N- or alkyl optionally substituted with aryl, heteroaryl, hydroxy, or Y³Y⁴N-.
"Arylalkyl" means an aryl-alkyl- group in which the aryl and alkyl moieties are as previously described. Preferred arylalkyl groups contain a C₁₋₄alkyl moiety. Exemplary arylalkyl groups include benzyl, 2-phenethyl and naphthlenemethyl.
"Arylalkyloxy" means an arylalkyl-O- group in which the arylalkyl groups is as previously described. Exemplary arylalkyloxy groups include benzyloxy and 1- or 2-naphthalenemethoxy.
"Arylalkyloxycarbonyl" means an arylalkyl-O-CO- group in which the arylalkyl groups is as previously described. An exemplary arylalkyloxycarbonyl group is benzyloxycarbonyl.
"Arylalkylthio" means an arylalkyl-S- group in which the arylalkyl group is as previously described. An exemplary arylalkylthio group is benzylthio.
"Arylene" means an optionally substituted bivalent radical derived from an aryl group as defined above. Exemplary arylene groups include optionally substituted phenylene, naphthylene and indanylene. Suitable substituents include one or more "aryl group substituents" as defined above, particularly halogen, methyl or methoxy.
"Aryloxy" means an aryl-O- group in which the aryl group is as previously described. Exemplary aryloxy groups include optionally substituted phenoxy and naphthoxy.
"Aryloxycarbonyl" means an aryl-O-CO- group in which the aryl group is as previously described. Exemplary aryloxycarbonyl groups include phenoxycarbonyl and naphthoxycarbonyl.
"Arylsulphinyl" means an aryl-SO- group in which the aryl group is as previously described.
"Arylsulphonyl" means an aryl-SO₂- group in which the aryl group is as previously described.
"Arylsulphonylcarbamoyl" means an aryl-SO₂-NH-C(=O)- group in which the aryl group is as previously described.
"Arylthio" means an aryl-S- group in which the aryl group is as previously described. Exemplary arylthio groups include phenylthio and naphthylthio.
"Azaheteroaryl" means an aromatic carbocyclic moiety of about 5 to about 10 ring members in which one of the ring members is nitrogen and the other ring members are chosen from carbon. oxygen, sulphur, or nitrogen. Examples of azaheteroaryl groups include pyridyl, pyrimidinyl, quinolinyl, isoquinolinyl, quinazolinyl, imidazolyl, and benzimidazolyl.
"Cycloalkenyl" means a non-aromatic monocyclic or multicyclic ring system containing at least one carbon-carbon double bond and having 3 to 10 carbon atoms. Exemplary monocyclic cycloalkenyl rings include C₃₋₈cycloalkenyl rings such as cyclopentenyl, cyclohexenyl or cycloheptenyl.
"Cycloalkyl" means a saturated monocyclic or bicyclic ring system of 3 to 10 carbon atoms optionally substituted by oxo. Exemplary monocyclic cycloalkyl rings include C₃₋₈cycloalkyl such as cyclopropyl, cyclopentyl, cyclohexyl and cycloheptyl.
"Cycloalkylalkyl" means a cycloalkyl-alkyl- group in which the cycloalkyl and alkyl moieties are as previously described. Exemplary monocyclic cycloalkylalkyl groups include C₃₋₈cycloalkylC₁₋₄alkyl groups such as cyclopropylmethyl, cyclopentylmethyl, cyclohexylmethyl and cycloheptylmethyl.
"Cycloalkylene" means a divalent radical derived from a cycloalkyl group as defined above. Exemplary cycloalkylene radicals include C₃₋₈cycloalkylene radicals such as cyclopentylene and cyclohexylene.
"Halo" or "halogen" means fluoro, chloro, bromo, or iodo. Preferred are fluoro or chloro.
"Heteroaroyl" means a heteroaryl-CO- group in which the heteroaryl group is as described herein. Exemplary groups include pyridylcarbonyl.
"Heteroaryl" as a group or part of a group denotes: (i) an optionally substituted aromatic monocyclic or multicyclic organic moiety of 5 to 10 ring members in which one or more of the ring members is/are element(s) other than carbon, for example nitrogen, oxygen or sulphur (examples of such groups include benzimidazolyl, benzthiazolyl, furyl, imidazolyl, indolyl, indolizinyl, isoxazolyl, isoquinolinyl, isothiazolyl, oxadiazolyl, pyrazinyl, pyridazinyl, pyrazolyl, pyridyl, pyrimidinyl, pyrrolyl, quinazolinyl, quinolinyl, 1,3,4-thiadiazolyl, thiazolyl, thienyl and triazolyl groups. optionally substituted by one or more aryl group substituents as defined above); (ii) an optionally substituted partially saturated multicyclic heterocarbocyclic moiety in which a heteroaryl and a cycloalkyl or cycloalkenyl group are fused together to form a cyclic structure (examples of such groups include pyrindanyl groups). Optional substituents are one or more "aryl group substituents" as defined above. When L¹ or R⁴ contains an optionally substituted heteroaryl group this may particularly represent an optionally substituted "azaheteroaryl" group.
"Heteroarylalkyl" means a heteroaryl-alkyl- group in which the heteroaryl and alkyl moieties are as previously described. Preferred heteroarylalkyl groups contain a C₁₋₄alkyl moiety such as optionally substituted pyridylC₁₋₄alkyl (e.g. optionally substituted pyridylmethyl).
"Heteroarylalkyloxy" means an heteroarylalkyl-O- group in which the heteroarylalkyl group is as previously described. Preferred heteroarylalkytoxy groups include heteroarylC₁₋₄alkyloxy such as optionally substituted pyridylC₁₋₄alkyloxy (e.g. optionally substituted pyridytmethoxy).
"Heteroarylamino" means a heteroaryl-NH- group in which the heteroaryl moiety are as previously described.
"Heteroaryldiyl" means a bivalent radical derived from a heteroaryl group as defined above.
"Heteroaryloxy" means an heteroaryl-O- group in which the heteroaryl group is as previousty described. Exemplary heteroaryloxy groups include optionally substituted pyridyioxy.
"Heteroarylsulphonylcarbamoyl" means a heteroaryl-SO₂-NH-C(=O)- group in which the heteroaryl group is as previously described.
"Heterocycloalkyl" means: (i) a cycloalkyl group of 3 to 7 ring members in which one or more of the ring carbon atoms is replaced by O, S or NY⁵ (where Y⁵ is hydrogen. alkyl, arylalkyl, and aryl); (ii) a partially saturated bicyclic system in which an aryl or heteroaryl ring is fused to a heterocycloalkyl ring as defined in (i) above. Examples of (ii) include 1,4-benzodioxanyl, 1,3-benzodioxolyl, chromanyl, dihydrobenzofuranyl, indolinyl and dihydropyrrolopyridinyl groups).
"Heterocycloalkylalkyl" means a heterocycloalkyl-alkyl- group in which the heterocycloalkyl and alkyl moieties are as previously described.
"Heterocycloalkylene" means a bivalent radical derived from a heterocycloalkyl group as defined above.
"Hydroxyalkyl" means a HO-alkyl- group in which alkyl is as previously defined. Preferred hydroxyalkyl groups contain C₁₋₄alkyl for example hydroxymethyl and 2-hydroxyethyl.
"Y³Y⁴N-" means a substituted or unsubstituted amino group, wherein Y³ and Y⁴ are as previously described. Exemplary groups include amino (H₂N-), methylamino, ethylmethylamino, dimethylamino and diethylamino.
"Y³Y⁴NCO-" means a substituted or unsubstituted carbamoyl group, wherein Y³ and Y⁴ are as previously described. Exemplary groups are carbamoyl (H₂NCO-) and dimethylcarbamoyl (Me₂NCO-).
"Y³Y⁴NSO₂-" means a substituted or unsubstituted sulphamoyl group, wherein Y³ and Y⁴ are as previously described. Exemplary groups are sulphamoyl (H₂NSO₂-) and dimethylsulphamoyl (Me₂NSO₂-).
"Ester Prodrung" means an ester of a compound of formula (I) containing a hydroxy group which may be convertible by hydrolysis in vivo to the parent molecule. Alternatively an ester of a compound of formula (I) containing a carboxy group may be convertible by hydrolysis in vivo to the parent molecule.

Suitable esters of compounds of formula (I) containing a hydroxy group, are for example acetates, citrates, lactates, tartrates, malonates, oxalates, salicylates, propionates, succinates, fumarates, maleates, methylene-bis-β-hydroxynaphthoates, gentisates, isethionates, di-p-toluoyltartrates, methanesulphonates, ethanesulphonates, benzenesulphonates, p-toluenesulphonates, cyclohexylsulphamates and quinates.

An especially useful class of esters of compounds of formula (I) containing a hydroxy group, may be formed from acid moieties selected from those described by Bundgaard et. al., J. Med. Chem., 1989, 32 , page 2503-2507, and include substituted (aminomethyl)-benzoates, for example dialkylamino-methylbenzoates in which the two alkyl groups may be joined together and/or interrupted by an oxygen atom or by an optionally substituted nitrogen atom, e.g. an alkylated nitrogen atom, more especially (morpholino-methyl)benzoates, e.g. 3- or 4-(morpholinomethyl)-benzoates, and (4-alkylpiperazin-1-yl)benzoates, e.g. 3- or 4-(4-alkylpiperazin-1-yl)benzoates.

The compound of the invention contains a carboxy group and base addition salts may be formed and are simply a more convenient form for use; and in practice, use of the salt form inherently amounts to use of the free acid form. The bases which can be used to prepare the base addition salts include preferably those which produce, when combined with the free acid, pharmaceutically acceptable salts, that is, salts whose cations are non-toxic to the patient in pharmaceutical doses of the salts, so that the beneficial inhibitory effects inherent in the free base are not vitiated by side effects ascribable to the cations. Pharmaceutically acceptable salts, including those derived from alkali and alkaline earth metal salts, within the scope of the invention include those derived from the following bases: sodium hydride, sodium hydroxide, potassium hydroxide, calcium hydroxide, aluminium hydroxide, lithium hydroxide, magnesium hydroxide, zinc hydroxide, ammonia, ethylenediamine, N-methylglucamine, lysine, arginine, ornithine, choline, N,N'-dibenzylethylenediamine, chloroprocaine. diethanolamine, procaine, N-benzylphenethylamine, diethylamine, piperazine, tris(hydroxymethyl)aminomethane or tetramethylammonium hydroxide.

Some of the compounds of the present invention are basic, and such compounds are useful in the form of the free base or in the form of a pharmaceutically acceptable acid addition salt thereof.

Acid addition salts are a more convenient form for use; and in practice, use of the salt form inherently amounts to use of the free base form. The acids which can be used to prepare the acid addition salts include preferably those which produce, when combined with the free base, pharmaceutically acceptable salts, that is, salts whose anions are non-toxic to the patient in pharmaceutical doses of the salts, so that the beneficial inhibitory effects inherent in the free base are not vitiated by side effects ascribable to the anions. Although pharmaceutically acceptable salts of said basic compounds are preferred. all acid addition salts are useful as sources of the tree base form even if the particular salt, per se, is desired only as an intermediate product as, for example, when the salt is formed only for purposes of purification, and identification, or when it is used as intermediate in preparing a pharmaceutically acceptable salt by ion exchange procedures. Pharmaceutically acceptable salts within thescope of the invention include those derived from mineral acids and organic acids, and include hydrohalides, e.g. hydrochlorides and hydrobromides, sulphates, phosphates, nitrates, sulphamates, acetates, citrates, lactates, tartrates, malonates, oxalates, salicylates, propionates, succinates, fumarates, maleates, methylene-bis-b-hydroxynaphthoates, gentisates, isethionates, di-p-toluoyltartrates, methane-sulphonates, ethanesulphonates, benzenesulphonates, p-toluenesulphonates, cyclohexylsulphamates and quinates.

As well as being useful in themselves as active compounds, salts of compounds of the invention are useful for the purposes of purification of the compounds, for example by exploitation of the solubility differences between the salts and the parent compounds, side products and/or starting materials by techniques well known to those skilled in the art.

With reference to formula (I) above, the following are particular and preferred groupings:
X¹ may particularly represent CR², where R² is C₁₋₄alkyl (e.g. methyl) or C₁₋₄alkoxy (e.g. methoxy).
X² may particularly represent CR², where R² is C₁₋₄alkyl (e.g. methyl) or C₁₋₄alkoxy (e.g. methoxy).
R⁹ may particularly represent a straight or branched C₁₋₆alkylene chain, especially a straight C₁₋₄alkylene chain such as methylene or ethylene, and R" represents especially:-
   (i) hydrogen;
   (ii) C₁₋₆alkyl (e.g. C₁₋₄alkyl groups such as ethyl, propyl or especially methyl);
   (iii) C₁₋₆alkyl (especially C₁₋₃alkyl) substituted by R⁵, where R⁵ is aryl (e.g. phenyl);
   (iv) C₁₋₆alkyl (especially C₁₋₃alkyl) substituted by R⁵, where R⁵ is heteroaryl (exemplary heteroaryl groups include indolyl, imidazolyl, pyridyl and furyl);
   (v) C₁₋₆alkyl (especially C₁₋₃alkyl) substituted by R⁵, where R⁵ is cycloalkyl (e.g. C₃₋₈cycloalkyl such as cyclopentyl and cyclohexyl);
   (vi) C₁₋₆alkyl (especially C₁₋₃alkyl) substituted by R⁵, where R⁵ is carboxy ; or
   (vii) C₁₋₆alkyl (e.g. C₁₋₄alkyl such as ethyl or propyl) substituted by R⁵, where R⁵ is -NY¹Y² (exemplary -NY¹Y² groups include acylamino, aryl(alkyl)amino, N-Pyrrolidinyl and 2-oxo-N-pyrrolidinyl).

The moiety R⁴ may particularly represent straight or branched C₁₋₁₀alkyl (e.g. methyl, ethyl, n-propyl, i-propyl, n-butyl, i-butyl, n-pentyl, i-pentyl, n-hexyl, n-heptyl, n-octyl, 1,5-dimethylhexyl, n-nonyl or n-decyl).

The moiety R⁴ may also particularly represent straight or branched C₁₋₆alkyl (e.g. methyl, ethyl, n-propyl, i-propyl or n-butyl) substituted by R⁵, where R⁵ is aryl. Exemplary aryl groups include phenyl optionally substituted by one or more "aryl group substituents", for example alkoxyphenyl, dialkoxyphenyl, arylalkyloxy(alkoxy)phenyl, halophenyl, dialkylaminophenyl, trifluoromethylphenyl and methanesulphonylphenyl. R⁴ is preferably straight or branched C₁₋₃alkyl substituted by diC₁₋₃alkoxyphenyl and is particularly 3,4-diC₁₋₃alkoxybenzyl.

The moiety R⁴ may also particularly represent straight or branched C₁₋₆alkyl (e.g. methyl, ethyl, n-propyl, i-propyl or n-butyl) substituted by R⁵, where R⁵ is heteroaryl. Exemplary heteroaryl groups include indolyl, imidazolyl, pyridyl and furyl. R⁴ is preferably straight or branched C₁₋₃alkyl substituted by azaheteroaryl and is particularly 3-(imidazol-1-yl)-C₁₋₃alkyl).

The moiety R⁴ may also particularly represent straight or branched C₁₋₆alkyl (e.g. methyl, ethyl, n-propyl, i-propyl or n-butyl) substituted by R⁵, where R⁵ is C₃₋₈cycloalkyl. R⁴ is preferably straight or branched C₁₋₃alkyl substituted by C₅₋₆cycloalkyl.

The moiety R⁴ may also particularly represent straight or branched C₁₋₆alkyl (e.g. methyl, ethyl, n-propyl, i-propyl or n-butyl)substituted by R⁵, where R⁵ is C₁₋₆alkoxy, especially C₁₋₄alkoxy such as methoxy.

The moiety R⁴ may also particularly represent straight or branched C₁₋₆alkyl (e.g. methyl, ethyl, n-propyl, i-propyl or n-butyl) substituted by R⁵, where R⁵ is halo.

The moiety R⁴ may also particularly represent straight or branched C₁₋₆alkyl (e.g. methyl, ethyl, n-propyl, i-propyl or n-butyl) substituted by R⁵, where R⁵ is an acidic functional group. R⁴ is preferably straight or branched C₁₋₃alkyl substituted by carboxy.

The moiety R⁴ may also particularly represent straight or branched C₁₋₆alkyl (e.g. methyl, ethyl, n-propyl, i-propyl or n-butyl) substituted by R⁵, where R⁵ is heterocycloalkyl. Exemplary heterocycloalkyl groups include benzodioxolyl and benzodioxanyl. R⁴ is preferably straight or branched C₁₋₃alkyl substituted by benzodioxolyl and benzodioxanyl.

The moiety R⁴ may also particularly represent straight or branched C₁₋₆alkyl (e.g. methyl, ethyl, n-propyl, i-propyl or n-butyl) substituted by -NY¹Y². Exemplary -NY¹Y² groups, include acylamino, aryl(alkylamino) and -NY¹Y² groups derived from 5-7 membered cyclic amines such as morpholine, piperidine, pyrrolidine and 2-oxo-pyrrolidine. R⁴ is preferably straight or branched C₂₋₃alkyl substituted by an N-linked 5-7 membered cyclic amine, especially 3-(2-oxo-pyrrolidin-1-yl)-C₂₋₃alkyl.

The moiety R⁴ may also particularly represent C₁₋₄alkenyl (e.g. allyl).

Compounds of formula (I) in which R⁴ represents straight or branched C₁₋₁₀alkyl (e.g. methyl, ethyl, n-propyl, i-propyl, n-butyl, i-butyl, n-pentyl, i-pentyl, n-hexyl, n-heptyl, n-octyl, 1,5-dimethylhexyl, n-nonyl, or n-decyl) are preferred.

Compounds of formula (I) in which R⁴ represents straight or branched C₁₋₆alkyl (e.g. methyl, ethyl, n-propyl, i-propyl or n-butyl) substituted by R⁵, where R⁵ is aryl are also preferred. Exemplary aryl groups include phenyl optionally substituted by one or more "aryl group substituents", for example alkoxyphenyl, dialkoxyphenyl, arylalkyloxy(akoxy)phenyl, halophenyl, dialkylaminophenyl, trifluoromethyl and methanesulphonylphenyl. Compounds of formula (I) in which R⁴ represents straight or branched C₁₋₃alkyl substituted by diC₁₋₃alkoxyphenyl, particularly 3,4-diC₁₋₃alkoxybenzyl (e.g. 3,4-dimethoxybenzyl 3,4-diethoxybenzyl and 3-ethoxy-4-methoxybenzyl), are especially preferred.

Compounds of formula (I) in which R⁴ represents straight or branched C₁₋₆alkyl (e.g. methyl, ethyl, n-propyl, i-propyl or n-butyl) substituted by R⁵, where R⁵ is heteroaryl are also preferred. Exemplary heteroaryl groups include indolyl, imidazolyl, pyridyl and furyl. Compounds of formula (I) in which R⁴ represents straight or branched C₁₋₃alkyl substituted by azaheteroaryl, particularly 3-(imidazol-1-yl)-C₁₋₃alkyl (e.g. 3-(imidazol-1-yl)-propyl), are especially preferred.

Compounds of formula (I) in which R⁴ represents straight or branched C₁₋₆alkyl (e.g. methyl, ethyl, n-propyl, i-propyl or n-butyl) substituted by R⁵, where R⁵ is C₃₋₈cycloalkyl are also preferred. Compounds of formula (I) in which R⁴ represents straight or branched C₁₋₃alkyl substituted by C₅₋₆cycloalkyl groups are especially preferred.

Compounds of formula (I) in which R⁴ represents straight or branched C₁₋₆alkyl (e.g. methyl, ethyl, n-propyl, i-propyl or n-butyl) substituted by R⁵, where R⁵ is C₁₋₆alkoxy, especially C₁₋₄alkoxy (e.g. methoxy), are also preferred.

Compounds of formula (I) in which R⁴ represents straight or branched C₁₋₆alkyl (e.g. methyl, ethyl, n-propyl, i-propyl or n-butyl) substituted by R⁵, where R⁵ is halo are also preferred.

Compounds of formula (I) in which R⁴ represents straight or branched C₁₋₆alkyl (e.g. methyl, ethyl, n-propyl, i-propyl or n-butyl) substituted by R⁵, where R⁵ is an acidic functional group are also preferred. Compounds of formula (I) in which R⁴ is straight or branched C₁₋₃alkyl substituted by carboxy are especially preferred.

Compounds of formula (I) in which R⁴ represents straight or branched C₁₋₆alkyl (e.g. methyl, ethyl, n-propyl, i-propyl or n-butyl) substituted by R⁵, where R⁵ is heterocycloalkyl are also preferred. Compounds of formula (I) in which R⁴ represents straight or branched C₁₋₃alkyl substituted by benzodioxolyl and benzodioxanyl are especially preferred.

Compounds of formula (I) in which R⁴ represents straight or branched C₁₋₆alkyl (e.g. methyl, ethyl, n-propyl, i-propyl or n-butyl) substituted by -NY¹Y² are also preferred. Exemplary -NY¹Y² groups include acylamino, aryl(alkylamino) and -NY¹Y² groups derived from 5-7 membered cyclic amines such as morpholine, piperidine, pyrrolidine and 2-oxo-pyrrolidine. Compounds of formula (I) in which R⁴ represents straight or branched C₁₋₃alkyl substituted by an N-linked 5-7 membered cyclic amine, especially 3-(2-oxo-pyrrolidin-1-yl)-C₁₋₃alkyl (e.g. 3-(2-oxo-pyrrolidin-1-yl)-propyl), are particularly preferred.

Compounds of formula (I) in which R⁴ represents C₁₋₄alkenyl (e.g. allyl) are also preferred.

Compounds of formula (I) in which R⁹ represents a straight or branched C₁₋₆alkylene chain, especially a straight or branched C₁₋₄alkylene chain, more especially methylene, are preferred.

Compounds of formula (I) in which R¹¹ represents hydrogen are preferred.

Compounds of formula (I) in which R¹¹ represents straight or branched C₁₋₄alkyl, particularly methyl, are also preferred.

Compounds of formula (I) in which R¹¹ represents straight or branched C₁₋₃alkyl substituted by R⁵, where R⁵ is aryl (e.g. phenyl), are also preferred. Compounds of formula (Ia) in which R¹¹ represents straight chain C₁₋₃alkyl substituted by phenyl are especially preferred.

Compounds of formula (I) in which R¹¹ represents straight or branched C₁₋₃alkyl substituted by R⁵, where R⁵ is heteroaryl, are also preferred. Exemplary heteroaryl groups include indolyl, imidazolyl, pyridyl and furyl. Compounds of formula (I) in which R¹¹ represents straight chain C₁₋₃alkyl substituted by azaheteroaryl (e.g. imidazolyl or pyridyl) are especially preferred.

Compounds of formula (I) in which R¹¹ represents straight or branched C₁₋₃alkyl substituted by R⁵, where R⁵ is cycloalkyl (e.g. C₃₋₈cycloalkyl), are also preferred. Exemplary C₃₋₈cycloalkyl groups include cyclopentyl and cyclohexyl. Compounds of formula (I) in which R¹¹ represents straight chain C₁₋₃alkyl substituted by cyclohexyl are especially preferred.

Compounds of formula (Ia) in which R¹¹ represents straight or branched C₁₋₃alkyl substituted by carboxy are also preferred.

Compounds of formula (I) in which R¹¹ represents straight or branched C₂₋₃alkyl (e.g. ethyl and n-propyl) substituted by -NY¹Y² are also preferred. Exemplary -NY¹Y² groups include acylamino, aryl(alkyl)amino and -NY¹Y² groups derived from 5-7 membered cyclic amines such as pyrrolidine and 2-oxo-pyrrolidine. Compounds of formula (I) in which R¹¹ represents ethyl or propyl substituted by 3-(2-oxo-pyrrolidin-1-yl), especially 3-(2-oxo-pyrrolidin-1-yl)-propyl, are preferred.

Compounds of formula (I) in which X¹ represents CR² where R² is C₁₋₄alkyl or C₁₋₄alkoxy (e.g. methyl or methoxy), especially methyl, are preferred.

Compounds of formula (I) in which X² represents CR² where R² is hydrogen or C₁₋₄alkoxy, especially methoxy, are also preferred.

The group -R⁹-C(=O)-N(R¹¹)-CH₂-C(=O)-NR⁴-CH₂-CH₂-Y may preferably be attached at the ring 4 position.

A preferred group of compounds of the invention are compounds of formula (I) in which:- R⁴ is C₁₋₁₀alkyl, C₁₋₆alkyl substituted by aryl (especially 3,4-dimethoxyphenylC₁₋₃alkyl), C₁₋₆alkyl substituted by heteroaryl (especially 3-(imidazol-1-yl)-propyl), C₁₋₆alkyl substituted by cycloalkyl (especially cyclopentyl- and cyclohexyl-C₁₋₃alkyl), C₁₋₆alkyl substituted by heterocycloalkyl (especially C₁₋₃alkyl substituted by benzodioxolyl and benzodioxanyl), C₁₋₆alkyl substituted by C₁₋₆alkoxy, C₁₋₆alkyl substituted by halo, C₁₋₆alkyl substituted by -NY¹Y², [especially (2-oxo-pyrrolidin-1-yl)propyl], or C₁₋₄alkenyl (e.g. allyl); R¹¹ represents hydrogen, C₁₋₄alkyl (especially methyl), C₁₋₃alkyl substituted by aryl (especially phenylC₁₋₃alkyl), C₁₋₃alkyl substituted by heteroaryl (especially imidazol-1-ylC₁₋₃alkyl and pyridylC₁₋₃alkyl), C₁₋₃alkyl substituted by C₃₋₈cycloalkyl (especially cyclohexylC₁₋₃alkyl), C₁₋₃alkyl substituted by carboxy (especially -(CH₂)₃CO₂H), or C₂₋₃alkyl substituted by -NY¹Y² [especially (2-oxo-pyrrolidin-1-yl)propyl]; R⁹ represents a straight or branched C₁₋₄alkylene chain, (preferably methylene); X¹ represents CR² where R² is C₁₋₄alkyl (especially methyl); X² represent CR² where R² is C₁₋₄alkoxy (e.g. methoxy); Y represents carboxy; and the group -R⁹-C(=O)-N(R¹¹)-CH₂-C(=O)-NR⁴-CH₂-CH₂-Y is attached at the ring 4 position;
and their ester prodrugs, and pharmaceutically acceptable salts and solvates (e.g. hydrates) of such compounds and their ester prodrugs.

Particular compounds of the invention are selected from the following:
3-{[({[3-methoxy-4-(3-o-tolylureido)phenyl]-acetyl}-N-methylamino)-acetyl)-[3-(2-oxopyrrolidin-1-yl)-prop-1-yl]-amino}-propionic acid, Compound A;
3-{[({[3-methoxy-4-(3-o-tolylureido)phenyl]-acetyl}-N-methylamino)-acetyl]-[3-(3-imidazol-1-yl)-prop-1-yl]-amino}-propionic acid, Compound B;
3-{(3,4-dimethoxy-benzyl)-[({[3-methoxy-4-(3-o-tolylureido)phenyl]-acetyl}-N-methylamino)-acetyl]-amino}-propionic acid, Compound C;
3-{[({[3-methoxy-4-(3-o-tolylureido)phenyl]-acetyl}-amino)-acetyl]-[3-(2-oxo-pyrrolidin-1-yl)-prop-1-yl]-amino}-propionic acid, Compound D;
3-[[2-(ethyl-m-tolyl-amino)-ethyl]-({2-[3-methoxy-4-(3-o-tolylureido)phenyl]-acetylamino}-acetyl)-amino]-propionic acid, Compound E;
3-[(2-acetylamino-ethyl)-({2-[3-methoxy-4-(3-o-tolylureido)phenyl]-acetylamino}-acetyl)-amino]-propionic acid, Compound F;
3-[(2-chloro-benzyl)-({2-[3-methoxy-4-(3-o-tolylureido)phenyl]-acetylamino}-acetyl)-amino]-propionic acid, Compound G;
3-[(3-methoxy-prop-1-yl)-({2-[3-methoxy-4-(3-o-tolylureido)phenyl]-acetylamino}-acetyl)-amino]-propionic acid, Compound H;
3-[cyclohexylmethyl-({2-[3-methoxy-4-(3-o-tolylureido)phenyl]-acetylamino}-acetyl)-amino]-propionic acid, Compound I;
3-[(4-methoxy-benzyl)-({2-[3-methoxy-4-(3-o-tolylureido)phenyl]-acetylamino}-acetyl)-amino]-propionic acid, Compound J;
3-[isobutyl-({2-[3-methoxy-4-(3-o-tolylureido)phenyl]-acetylamino}-acetyl)-amino]-propionic acid, Compound K;
3-[({2-[3-methoxy-4-(3-o-tolylureido)phenyl]-acetylamino}-acetyl)-(1-phenyl-ethyl)-amino]-propionic acid, Compound L;
3-{({2-[3-methoxy-4-(3-o-tolylureido)phenyl]-acetylamino}-acetyl)-[4-(5H-1,2,4-[1,2,3]thiadiazol-4-yl)-benzyl]-amino}-propionic acid. Compound M;
3-[[1-(4-fluoro-phenyl)-ethyl]-({2-[3-methoxy-4-(3-o-tolylureido)phenyl]-acetylamino}-acetyl)-amino]-propionic acid, Compound N;
3-[(2-ethoxy-benzyl)-({2-[3-methoxy-4-(3-o-tolylureido)phenyl]-acetylamino}-acetyl)-amino]-propionic acid, Compound O;
3-[({2-[3-methoxy-4-(3-o-tolylureido)phenyl]-acetylamino}-acetyl)-(2-pyridin-2-yl-ethyl)-amino]-propionic acid, Compound P;
3-[[2-(3-bromo-4-methoxy-phenyl)-ethyl]-({2-[3-methoxy-4-(3-o-tolylurcido)phenyl]-acetylamino}-acetyl)-amino]-propionic acid, Compound Q;
3-[(3-methoxy-benzyl)-({2-[3-methoxy-4-(3-o-tolylureido)phenyl]-acetylamino}-acetyl)-amino]-propionic acid. Compound R;
3-[(2-methoxy-ethyl)-({2-[3-methoxy-4-(3-o-tolylureido)phenyl]-acetylamino}-acetyl)-amino]-propionic acid, Compound S;
3-[({2-[3-methoxy-4-(3-o-tolylureido)phenyl]-acetylamino}-acetyl)-(3-methyl-butyl)-amino]-propionic acid, Compound T;
3-{({2-[3-methoxy-4-(3-o-tolylureido)phenyl]-acetylamino}-acetyl)-[2-(4-phenoxy-phenyl)-ethyl]-amino}-propionic acid. Compound U;
3-[(2-benzo[1,3]dioxol-5-yl-ethyl)-({2-[3-methoxy-4-(3-o-tolylureido)phenyl]-acetylamino}-acetyl)-amino]-propionic acid, Compound V;
3-[butyl-({2-[3-methoxy-4-(3-o-tolylureido)phenyl]-acetylamino}-acetyl)-amino]-propionic acid, Compound W;
3-[[2-(3,5-dimethoxy-phenyl)-ethyl]-({2-[3-methoxy-4-(3-o-tolylureido)phenyl}-acetylamino}-acetyl)-amino]-propionic acid, Compound X;
3-[({2-[3-methoxy-4-(3-o-tolylureido)phenyl]-acetylamino}-acetyl)-(furan-2-y-methyl)-amino]-propionic acid, Compound Y;
3-[allyl-({2-[3-methoxy-4-(3-o-tolylureido)phenyl]-acetylamino}-acetyl)-amino]-propionic acid, Compound Z;
3-[({2-[3-methoxy-4-(3-o-tolylureido)phenyl]-acetylamino}-acetyl)-pyridin-3-ylmethyl-amino]-propionic acid, Compound AA;
3-[(3-chloro-prop-1-yl)-({2-[3-methoxy-4-(3-o-tolylureido)phenyl]-acetylamino)-acetyl)-amino}-propionic acid, Compound AB;
3-[({2-[3-methoxy-4-(3-o-tolylureido)phenyl]-acetylamino}-acetyl)-(3-phenyl-prop-1-yl)-amino]-propionic acid, Compound AC;
3-[(2-methoxy-benzyl)-({2-[3-methoxy-4-(3-o-tolylureido)phenyl]-acetylamino}-acetyl)-amino]-propionic acid, Compound AD;
3-[({2-[3-methoxy-4-(3-o-tolylureido)phenyl]-acetylamino}-acetyl)-(2-morpholin-4-yl-ethyl)-amino]-propionic acid, Compound AE;
3-[(4-methanesulfonyl-benzyl)-({2-[3-methoxy-4-(3-o-tolylureido)phenyl]-acetylamino}-acetyl)-amino]-propionic acid, Compound AF;
3-[({2-[3-methoxy-4-(3-o-tolylureido)phenyl]-acetylamino}-acetyl)-methyl-amino]-propionic acid, Compound AG;
3-{({2-[3-methoxy-4-(3-o-tolylureido)phenyl]-acetylamino}-acetyl)-[2-(naphthalen-2-ylamino)-ethyl]-amino}-propionic acid, Compound AH;
3-[[2-(2,3-dimethoxy-phenyl)-ethyl]-({2-[3-methoxy-4-(3-o-tolylureido)phenyl]-acetylamino}-acetyl)-amino]-propionic acid, Compound AI;
3-[(2-diethylamino-ethyl)-({2-[3-methoxy-4-(3-o-tolylurcido)phenyl]-acetylamino}-acetyl)-amino]-propionic acid, Compound AJ;
3-[(1,5-dimethyl-hexyl)-({2-[3-methoxy-4-(3-o-tolylureido)phenyl]-acetylamino}-acetyl)-amino]-propionic acid, Compound AK;
3-[({2-[3-methoxy-4-(3-o-tolylureido)phenyl]-acetylamino}-acetyl)-pentyl-amino]-propionic acid, Compound AL;
3-[({2-[3-methoxy-4-(3-o-tolylureido)phenyl]-acetylamino}-acetyl)-octyl-amino]-propionic acid, Compound AM;
3-[[2-(2h-indol-3-yl)-ethyl]-({2-[3-methoxy-4-(3-o-tolylureido)phenyl]-acetylamino}-acetyl)-amino]-propionic acid, Compound AN;
3-[(2,3-dimethoxy-benzyl)-({2-[3-methoxy-4-(3-o-tolylureido)phenyl]-acetylamino}-acetyl)-amino]-propionic acid, Compound AO;
3-[({[3-methoxy-4-(3-o-tolylureido)phenyl]-acetylamino}-acetyl)-prop-1-yl-amino]-propionic acid, Compound AP;
3-[(3,3-diphenyl-prop-1-yl)-([2-[3-methoxy-4-(3-o-tolylureido)phenyl]-acetylamino}-acetyl)-amino]-propionic acid, Compound AQ;
3-[(2,2-diphenyl-ethyl)-({2-[3-methoxy-4-(3-o-tolylureido)phenyl]-acetylamino}-acetyl)-amino]-propionic acid, Compound AR;
3-[[2-(5-methoxy-2h-indol-3-yl)-ethyl]-({2-[3-methoxy-4-(3-o-tolylureido)phenyl]-acetylamino}-acetyl)-amino]-propionic acid, Compound AS;
3-[({2-[3-methoxy-4-(3-o-tolylureido)phenyl]-acetylamino}-acetyl)-(4-phenyl-butyl)-amino]-propionic acid, Compound AT;
3-[hexyl-({2-[3-methoxy-4-(3-o-tolylureido)phenyl]-acetylamino}-acetyl)-amino]-propionic acid, Compound AU;
3-[benzo[1,3]dioxol-5-ylmethyl-({2-[3-methoxy-4-(3-o-tolylureido)phenyl]-acetylamino}-acetyl)-amino]-propionic acid, Compound AV;
3-[(2-acetylamino-ethyl)-({2-[3-methoxy-4-(3-o-tolylureido)phenyl]-acetylamino}-acetyl)-amino]-propionic acid, Compound AW;
3-{({2-[3-methoxy-4-(3-o-tolylureido)phenyl]-acetylamino}-acetyl)-[2-(4-nitro-phenyl)-ethyl]-amino}-propionic acid, Compound AX;
3-[({2-[3-methoxy-4-(3-o-tolylureido)phenyl]-acetylamino}-acetyl)-(2-oxo-azepan-3-yl)-amino]-propionic acid, Compound AY;
3-[(3,5-dimethoxy-benzyl)-({2-[3-methoxy-4-(3-o-tolylureido)phenyl]-acetylamino}-acetyl)-amino]-propionic acid, Compound AZ;
3-[(3-dimethylamino-prop-1-yl)-({2-[3-methoxy-4-(3-o-tolylureido)phenyl]-acetylamino}-acetyl)-amino]-propionic acid, Compound BA;
3-[({2-[3-methoxy-4-(3-o-tolylureido)phenyl]-acetylamino}-acetyl)-naphthalen-1-ylmethylamino]-propionic acid. Compound BB;
3-[(1-cyclohexyl-ethyl)-({2-[3-methoxy-4-(3-o-tolylureido)phenyl]-acetylamino}-acetyl)-amino]-propionic acid, Compound BC;
3-[N-(3,4-dimethoxybenzyl)-2-{2-[3-methoxy-4-(3-o-tolylureido)phenyl]acetylamino}acetamido]-propionic acid, alternative name: 3-[(3,4-dimethoxy-benzyl)-({2-[3-methoxy-4-(3-o-tolyl-ureido)-phenyl]-acetylamino}-acetyl)-amino]-propionic acid, Compound BD;
3-[(2-diethylamino-ethyl)-({2-[3-methoxy-4-(3-o-tolylureido)phenyl]-acetylamino}-acetyl)-amino]-propionic acid, Compound BE;
3-[({2-[3-methoxy-4-(3-o-tolylureido)phenyl]-acetylamino}-acetyl)-(4-nitro-benzyl)-amino]-propionic acid, Compound BF;
3-[({2-[3-methoxy-4-(3-o-tolylureido)phenyl]-acetylamino}-acetyl)-(2-piperidin-1-yl-ethyl)-amino]-propionic acid, Compound BG;
3-[benzyl-({2-[3-methoxy-4-(3-o-tolylureido)phenyl]-acetylamino}-acetyl)-amino]-propionic acid, Compound BH;
3-[cyclohexyl-({2-[3-methoxy-4-(3-o-tolylureido)phenyl]-acetylamino}-acetyl)-amino]-propionic acid, Compound BI;
3-[isobutyl-({2-[3-methoxy-4-(3-o-tolylureido)phenyl]-acetylamino}-acetyl)-amino]-propionic acid, Compound BJ;
3{(3-imidazol-1-yl-prop-1-yl)-[({[3-methoxy-4-(3-o-tolylureido)phenyl]-acetyl}-amino)-acetyl]-amino}-propionic acid, Compound BK;
3-[({2-[3-methoxy-4-(3-o-tolylureido)phenyl]-acetylamino}-acetyl)-(4-trifluoromethyl-benzyl)-amino]-propionic acid, Compound BL;
3-{({2-[3-methoxy-4-(3-o-tolylureido)phenyl]-acetylamino}-acetyl)-[2-(7-methyl-2h-indol-3-yl)-ethyl]-amino}-propionic acid, Compound BN;
3-[(4-dimethylamino-benzyl)-({2-[3-methoxy-4-(3-o-tolylureido)phenyl]-acetylamino}-acetyl)-amino]-propionic acid, Compound BO;
3-[isopropyl-({2-[3-methoxy-4-(3-o-tolylureido)phenyl]-acetylamino}-acetyl)-amino]-propionic acid, Compound BP;
3-[(6-chloro-2-phenoxy-phenylmethyl)-({2-[3-methoxy-4-(3-o-tolylureido)phenyl]-acetylamino}-acetyl)-amino]-propionic acid, Compound BQ;
3-[({2-[3-methoxy-4-(3-o-tolylureido)phenyl]-acetylamino}-acetyl)-phenethyl-amino]-propionic acid, Compound BR;
3-[({2-[3-methoxy-4-(3-o-tolylureido)phenyl]-acetylamino}-acetyl)-(1-methyl-2-phenoxy-ethyl)-amino]-propionic acid, Compound BS;
3-[[2-(5-methoxy-2H-indol-3-yl)-ethyl]-({2-[3-methoxy-4-(3-o-tolylureido)phenyl]-acetylamino}-acetyl)-amino]-propionic acid, Compound BT;
3-[({2-[3-methoxy-4-(3-o-tolylureido)phenyl]-acetylamino}-acetyl)-(3-phenyl-prop-1-yl)-amino]-propionic acid, Compound BU;
3-{[({[3-methoxy-4-(3-o-tolylureido)phenyl]-acetyl}-prop-1-ylamino)-acetyl]-[3-(2-oxopyrrolidin-1-yl)-prop-1-yl]-amino}-propionic acid, Compound BV; Compounds BW to KV;
3-{[({[3-methoxy-4-(3-o-tolylureido)phenyl]-acetyl}-N-methylamino)-acetyl]-(3-carboxy-prop-1-yl)-amino}-propionic acid; Compound KW;
3-{[({[3-methoxy-4-(3-o-tolylureido)phenyl]-acetyl}-N-methylamino)-acetyl]-{2-(2-oxopyrrolidin-1-yl)-ethyl]-amino}-propionic acid; Compound KX;
3-{[({[3-methoxy-4-(3-o-tolylureido)phenyl}-acetyl}-amino)-acetyl}-(3-carboxy-prop-1-yl)-amino}-propionic acid; Compound LA;
3-{[({[3-methoxy-4-(3-o-tolylureido)phenyl]-acetyl}-N-methylamino)-acetyl]-[2-(2-oxopyrrolidin-1-yl)-ethyl]-amino}-propionic acid; Compound LB;
3-{[({[3-methoxy-4-(3-o-tolylureido)phenyl]-acetyl}-amino)-acetyl]-(2-carboxy-ethyl)-amino}-propionic acid; Compound LC;
3-{(2,3-dimethoxy-benzyl)-[({[3-methoxy-4-(3-o-tolylureido)phenyl]-acetyl)-amino)-acetyl}-amino}-propionic acid; Compound AO;
3-{[({[3-methoxy-4-(3-o-tolylureido)phenyl]-acetyl}-amino)-acetyl]-phenyl-amino}-propionic acid; Compound LD;
3-{(3-ethoxy-4-methoxy-benzyl)-[({[3-methoxy-4-(3-o-tolylureido)phenyl]-acetyl}-amino)-acetyl]-amino}-propionic acid; Compound LE;
3-{(3,4-diethoxy-benzyl)-[({[3-methoxy-4-(3-o-tolylureido)phenyl]-acetyl}-amino)-acetyl]-amino}-propionic acid; Compound LF;
3-{(4-benzyloxy-3-methoxy -benzyl)-[({[3-methoxy-4-(3-o-tolylureido)phenyl]-acetyl}-amino)-acetyl]-amino}-propionic acid; Compound LG;
3-{[(1,4-benzodioxan-6-yl)-methyl]-[({[3-methoxy-4-(3-o-tolylureido)phenyl]-acetyl}-amino)-acetyl]-amino}-propionic acid; Compound LH;
3-{[({[3-methoxy-4-(3-o-tolylureido)phenyl]-acetyl}-amino)-acetyl]-(3-methanesulphonylaminoprop-1-yl)-amino}-propionic acid; Compound LI;
3-[(4-dimethylamino-benzyl)-[{2-[3-methoxy-4-(3-o-tolylureido)phenyl]-acetyl}-amino)-acetyl]-N-methylamino]-propionic acid;
3-{(3-nitro-benzyl)-[({[3-methoxy-4-(3-o-tolylureido)phenyl]-acetyl}-amino)-acetyl]-amino}-propionic acid, Compound LJ;
3-{(2-thienylmethyl)-({[3-methoxy-4-(3-o-tolylureido)phenyl]-acetyl}-N-methylamino}-acetyl)-amino}-propionic acid, Compound LK;
3-[(2-methoxy-benzyl)-[({[3-methoxy-4-(3-o-tolylureido)phenyl]-acetyl)-N-methylamino)-acetyl]-amino}-propionic acid; Compound LL;
3-{(4-methyl-benzyl)-[({[3-methoxy-4-(3-o-tolylureido)phenyl]-acetyl}-N-methylamino)-acetyl]-amino}-propionic acid, Compound LM;
3-{(3,4-methylenedioxy-benzyl)-[({[3-methoxy-4-(3-o-tolylureido)phenyl]-acetyl}-N-methylamino)-acetyl]-amino}-propionic acid, Compound LN; 3-{(3,5-dimethoxy-benzyl)-[({[3-methoxy-4-(3-o-tolylureido)phenyl]-acetyl}-N-methylamino)-acetyl]-amino}-propionic acid, Compound LO;
3-{(2-pyridylmethyl)-[({[3-methoxy-4-(3-o-tolylureido)phenyl]-acetyl}-amino)-acetyl]-amino}-propionic acid. Compound LP;
3-{(2-furanylmethyl)-[({[3-methoxy-4-(3-o-tolylureido)phenyl]-acetyl}-N-methylamino)-acetyl]-amino}-propionic acid, Compound LQ;
3-{(2-ethoxy-benzyl)-[({[3-methoxy-4-(3-o-tolylureido)phenyl]-acetyl}-N-methylamino)-acetyl]-amino}-propionic acid, Compound LR;
3-{(2-thienylmethyl)-[({[3-methoxy-4-(3-o-tolylureido)phenyl]-acetyl}-amino)-acetyl]-amino}-propionic acid, Compound LS;
3-{(4-pyridylmethyl)-[({[3-methoxy-4-(3-o-tolylureido)phenyl]-acetyl}-amino)-acetyl]-amino}-propionic acid, Compound LT;
3-{(2-pyridylmethyl)-[({[3-methoxy-4-(3-o-tolylureido)phenyl]-acetyl}-N-methylamino)-acetyl]-amino)-propionic acid, Compound LU;
3-{(3-nitro-benzyl)-[({[3-methoxy-4-(3-o-tolylureido)phenyl]-acetyl}-N-methylamino)-acetyl]-amino}-propionic acid, Compound LV;
3-{(3-pyridylmethyl)-[({[3-methoxy-4-(3-o-tolylureido)phenyl]-acetyl}-N-methylamino)-acetyl]-amino}-propionic acid, Compound LW;
3-{(4-[1,2,3-thiadiazol-4-yl]-benzyl)-[({[3-methoxy-4-(3-o-tolylureido)phenyl]-acetyl}-N-methylamino)-acetyl]-amino}-propionic acid, Compound LX; 3-{(4-pyridylmethyl)-[({[3-methoxy-4-(3-o-tolylureido)phenyl]-acetyl}-N-methylamino)-acetyl]-amino}-propionic acid, Compound LY;
3-{(benzyl)-[({[3-methoxy-4-(3-o-tolylureido)phenyl]-acetyl}-N-methylamino)-acetyl]-amino}-propionic acid. Compound LZ;
3-{(2-bromo-benzyl)-[({[3-methoxy-4-(3-o-tolylureido)phenyl]-acetyl}-N-methylamino)-acetyl]-amino}-propionic acid. Compound MA;
3-{(2-bromo-benzyl)-[({[3-methoxy-4-(3-o-tolylureido)phenyl]-acetyl}-amino)-acetyl]-amino}-propionic acid, Compound MB;
3-{(2-chloro-benzyl)-[({[3-methoxy-4-(3-o-tolylureido)phenyl]-acetyl}-N-methylamino)-acetyl]-amino}-propionic acid, Compound MC;
3-{(4-methanesulphonyl-benzyl)-[({[3-methoxy-4-(3-o-tolylureido)phenyl]-acetyl}-N-methylamino)-acetyl]-amino}-propionic acid, Compound MD;
and their ester prodrugs, and pharmaceutically acceptable salts and solvates (e.g. hydrates) of such compounds and their ester prodrugs.

Preferred compounds of the invention include:
3-{[({[3-methoxy-4-(3-o-tolylureido)phenyl]-acetyl}-N-methylamino)-acetyl]-[3-(2-oxopyrrolidin-1-yl)-prop-1-yl]-amino}-propionic acid, Compound A;
3-{[({[3-methoxy-4-(3-o-tolylureido)phenyl]-acetyl}-N-methylamino)-acetyl]-[3-(3-imidazol-1-yl)-prop-1-yl]-amino}-propionic acid, Compound B;
3-{(3,4-dimethoxy-benzyl)-[({[3-methoxy-4-(3-o-tolylureido)phenyl]-acetyl}-N-methylamino)-acetyl]-amino}-propionic acid, Compound C;
3-{[({[3-methoxy-4-(3-o-tolylureido)phenyl]-acetyl}-amino)-acetyl]-[3-(2-oxo-pyrrolidin-1-yl)-prop-1-yl]-amino}-propionic acid, Compound D;
3-[allyl-({2-[3-methoxy-4-(3-o-tolylureido)phenyl]-acetylamino}-acetyl)-amino]-propionic acid, Compound Z;
3-[({2-[3-methoxy-4-(3-o-tolylureido)phenyl]-acetylamino}-acetyl)-(3-phenyl-prop-1-yl)-amino]-propionic acid, Compound AC;
3-[(2,3-dimethoxy-benzyl)-({2-[3-methoxy-4-(3-o-tolylureido)phenyl]-acetylamino}-acetyl)-amino]-propionic acid, Compound AO;
3-[({2-[3-methoxy-4-(3-o-tolylureido)phenyl]-acetylamino}-acetyl)-(4-phenyl-butyl)-amino]-propionic acid, Compound AT;
3-[N-(3,4-dimethoxybenzyl)-2-{2-[3-methoxy-4-(3-o-tolylureido)phenyl]acetylamino}acetamido]-propionic acid, Compound BD;
3{(3-imidazol-1-yl-prop-1-yl)-[({[3-methoxy-4-(3-o-tolylureido)phenyl]-acetyl}-amino)-acetyl]-amino}-propionic acid, Compound BK;
3-[(4-dimethylamino-benzyl)-({2-[3-methoxy-4-(3-o-tolylureido)phenyl]-acetylamino}-acetyl)-amino]-propionic acid, Compound BO;
3-{[({[3-methoxy-4-(3-o-tolylureido)phenyl]-acetyl}-N-methylamino)-acetyl]-(3-carboxyprop-1-yl)-amino}-propionic acid; Compound KW;
3-{[({[3-methoxy-4-(3-o-tolylureido)phenyl]-acetyl}-N-methylamino)-acetyl]-[2-(2-oxopyrrolidin-1-yl)-ethyl]-amino}-propionic acid; Compound KX; 3-{(3,4-dimethoxy-benzyl)-[({[3-methoxy-4-(3-o-tolylureido)phenyl]-acetyl}-amino)-acetyl]-amino}-propionic acid; Compound KY;
3-{[({[3-methoxy-4-(3-o-tolylureido)phenyl]-acetyl}-amino)-acetyl]-(3-carboxy-prop-1-yl)-amino}-propionic acid; Compound LA;
3-{[({[3-methoxy-4-(3-o-tolylureido)phenyl]-acetyl}-amino)-acetyl]-(2-carboxy-ethyl)-amino}-propionic acid; Compound LC;
3-{[({[3-methoxy-4-(3-o-tolylureido)phenyl]-acetyl}-amino)-acetyl]-phenyl-amino}-propionic acid; Compound LD;
3-{(3-ethoxy-4-methoxy-benzyl)-[({[3-methoxy-4-(3-o-tolylureido)phenyl]-acetyl}-amino)-acetyl]-amino}-propionic acid; Compound LE;
3-{(3,4-diethoxy-benzyl)-[({[3-methoxy-4-(3-o-tolylureido)phenyl]-acetyl}-amino)-acetyl]-amino}-propionic acid; Compound LF;
3-{(4-benzyloxy-3-methoxy -benzyl)-[({[3-methoxy-4-(3-o-tolylureido)phenyl]-acetyl}-amino)-acetyl]-amino}-propionic acid; Compound LG;
3-{[(1,4-benzodioxan-6-yl)-methyl]-[({[3-methoxy-4-(3-o-tolylureido)phenyl]-acetyl}-amino)-acetyl]-amino}-propionic acid; Compound LH;
3-{[({[3-methoxy-4-(3-o-tolylureido)phenyl]-acetyl}-amino)-acetyl]-(3-methanesulphonylaminoprop-1-yl)-amino}-propionic acid; Compound LI;
3-{(3-nitro-benzyl)-[({[3-methoxy-4-(3-o-tolylureido)phenyl]-acetyl}-amino)-acetyl]-amino}-propionic acid, Compound LJ;
3-{(2-thienylmethyl)-({[3-methoxy-4-(3-o-tolylureido)phenyl]-acetyl}-N-methylamino}-acetyl)-amino}-propionic acid, Compound LK;
3-{(2-methoxy-benzyl)-[({[3-methoxy-4-(3-o-tolylureido)phenyl]-acetyl}-N-methylamino)-acetyl]-amino}-propionic acid; Compound LL;
3-{(4-methyl-benzyl)-[({[3-methoxy-4-(3-o-tolylureido)phenyl]-acetyl}-N-methylamino)-acetyl}-amino}-propionic acid, Compound LM;
3-{(3,4-methylenedioxy-benzyl)-[({[3-methoxy-4-(3-o-tolylureido)phenyl)-acetyl}-N-methylamino)-acetyl]-amino}-propionic acid, Compound LN;
3-{(3,5-dimethoxy-benzyl)-[({[3-methoxy-4-(3-o-tolylureido)phenyl]-acetyl}-N-methylamino)-acetyl]-amino}-propionic acid, Compound LO;
3-{(2-pyridylmethyl)-[({[3-methoxy-4-(3-o-tolylureido)phenyl]-acetyl}-amino)-acetyl]-amino}-propionic acid. Compound LP;
3-{(2-furanylmethyl)-[({[3-methoxy-4-(3-o-tolylureido)phenyl]-acetyl}-N-methylamino)-acetyl]-amino}-propionic acid, Compound LQ;
3-{(2-ethoxy-benzyl)-[({[3-methoxy-4-(3-o-tolylureido)phenyl]-acetyl}-N-methylamino)-acetyl]-amino}-propionic acid, Compound LR;
3-{(2-thienylmethyl)-[({[3-methoxy-4-(3-o-tolylureido)phenyl]-acetyl}-amino)-acetyl]-amino}-propionic acid, Compound LS;
3-{(4-pyridylmethyl)-[({[3-methoxy-4-(3-o-tolylureido)phenyl]-acetyl}-amino)-acetyl]-amino}-propionic acid, Compound LT;
and their ester prodrugs, and pharmaceutically acceptable salts and solvates (e.g. hydrates) of such compounds and their ester prodrugs.

Especially preferred compounds of the invention include:
3-{[({[3-methoxy-4-(3-o-tolylureido)phenyl]-acetyl}-N-methylamino)-acetyl]-[3-(2-oxopyrrolidin-1-yl)-prop-1-yl]-amino}-propionic acid, Compound A;
3-{(3,4-dimethoxy-benzyl)-[({[3-methoxy-4-(3-o-tolylureido)phenyl]-acetyl}-N-methylamino)-acetyl]-amino}-propionic acid, Compound C;
3-{[({[3-methoxy-4-(3-o-tolylureido)phenyl]-acetyl}-amino)-acetyl]-[3-(2-oxo-pyrrolidin-1-yl)-prop-1-yl]-amino}-propionic acid, Compound D;
3-[(2,3-dimethoxy-benzyl)-({2-[3-methoxy-4-(3-o-tolylureido)phenyl]-acetylamino}-acetyl)-amino]-propionic acid, Compound AO;
3-[N-(3,4-dimethoxybenzyl)-2-{2-[3-methoxy-4-(3-o-tolylureido)phenyl]acetylamino}acetamido]-propionic acid, Compound BD;
3-{[({[3-methoxy-4-(3-o-tolylureido)phenyl]-acetyl}-N-methylamino)-acetyl]-(3-carboxyprop-1-yl)-amino}-propionic acid; Compound KW;
3-{(3-ethoxy-4-methoxy-benzyl)-[({[3-methoxy-4-(3-o-tolylureido)phenyl]-acetyl}-amino)-acetyl]-amino}-propionic acid; Compound LE;
3-{(3,4-diethoxy-benzyl)-[({[3-methoxy-4-(3-o-tolylureido)phenyl]-acetyl}-amino)-acetyl]-amino}-propionic acid; Compound LF;
and their ester prodrugs, and pharmaceutically acceptable salts and solvates (e.g. hydrates) of such compounds and their ester prodrugs.

The compounds of the invention exhibit useful pharmacological activity and accordingly are incorporated into pharmaceutical compositions and used in the treatment of patients suffering from certain medical disorders. The present invention thus provides, according to a further aspect, compounds of the invention and compositions containing compounds of the invention for use in therapy.

Compounds within the scope of the present invention block the interaction of the ligand VCAM-1 to its integrin receptor VLA-4 (α4β1) according to tests described in the literature and described in vitro and in vivo procedures hereinafter, and which tests results are believed to correlate to pharmacological activity in humans and other mammals. Thus, in a further embodiment, the present invention provides compounds of the invention and compositions containing compounds of the invention for use in the treatment of a patient suffering from, or subject to, conditions which can be ameliorated by the administration of an inhibitor of α4β1 mediated cell adhesion. For example, compounds of the present invention are useful in the treatment of inflammatory diseases, for example joint inflammation, including arthritis, rheumatoid arthritis and other arthritic conditions such as rheumatoid spondylitis, gouty arthritis, traumatic arthritis, rubella arthritis, psoriatic arthritis and osteoarthritis. Additionally, the compounds are useful in the treatment of acute synovitis, autoimmune diabetes, autoimmune encephalomyelitis, collitis, atherosclerosis, peripheral vascular disease, cardiovascular disease, multiple sclerosis, asthma, psoriasis restenosis, myocarditis, inflammatory bowel disease and melanoma cell division in metastasis.

A special embodiment of the therapeutic methods of the present invention is the treating of asthma.

Another special embodiment of the therapeutic methods of the present invention is the treating of joint inflammation.

Another special embodiment of the therapeutic methods of the present invention is the treating of inflammatory bowel disease.

According to a further feature of the invention there is provided the use of compound of the formula (I) for the manufactur of a medicament for the treatment of a human or animal patient suffering from, or subject to, conditions which can be ameliorated by the administration of an inhibitor of the interaction of the ligand VCAM-1 to its integrin receptor VLA-4 (α4β1), for example conditions as hereinbefore described.

References herein to treatment should be understood to include prophylactic therapy as well as treatment of established conditions.

The present invention also includes within its scope pharmaceutical compositions comprising at least one of the compounds of the invention in association with a pharmaceutically acceptable carrier or excipient.

Compounds of the invention may be administered by any suitable means. In practice compounds of the present invention may generally be administered parenterally, topically, rectally, orally or by inhalation, especially by the oral route.

Compositions according to the invention may be prepared according to the customary methods, using one or more pharmaceutically acceptable adjuvants or excipients. The adjuvants comprise, inter alia, diluents, sterile aqueous media and the various non-toxic organic solvents. The compositions may be presented in the form of tablets, pills, granules, powders, aqueous solutions or suspensions, injectable solutions, elixirs or syrups, and can contain one or more agents chosen from the group comprising sweeteners, flavourings, colourings, or stabilisers in order to obtain pharmaceutically acceptable preparations. The choice of vehicle and the content of active substance in the vehicle are generally determined in accordance with the solubility and chemical properties of the active compound, the particular mode of administration and the provisions to be observed in pharmaceutical practice. For example, excipients such as lactose, sodium citrate, calcium carbonate, dicalcium phosphate and disintegrating agents such as starch, alginic acids and certain complex silicates combined with lubricants such as magnesium stearate, sodium lauryl sulphate and talc may be used for preparing tablets. To prepare a capsule, it is advantageous to use lactose and high molecular weight polyethylene glycols. When aqueous suspensions are used they can contain emulsifying agents or agents which facilitate suspension. Diluents such as sucrose, ethanol, polyethylene glycol, propylene glycol, glycerol and chloroform or mixtures thereof may also be used.

For parenteral administration, emulsions, suspensions or solutions of the products according to the invention in vegetable oil, for example sesame oil, groundnut oil or olive oil, or aqueous-organic solutions such as water and propylene glycol, injectable organic esters such as ethyl oleate, as well as sterile aqueous solutions of the pharmaceutically acceptable salts, are used. The solutions of the salts of the products according to the invention are especially useful for administration by intramuscular or subcutaneous injection. The aqueous solutions, also comprising solutions of the salts in pure distilled water, may be used for intravenous administration with the proviso that their pH is suitably adjusted, that they are judiciously buffered and rendered isotonic with a sufficient quantity of glucose or sodium chloride and that they are sterilised by heating, irradiation or microfiltration.

For topical administration, gels (water or alcohol based), creams or ointments containing compounds of the invention may be used. Compounds of the invention may also be incorporated in a gel or matrix base for application in a patch, which would allow a controlled release of compound through the transdermal barrier.

For administration by inhalation compounds of the invention may be dissolved or suspended in a suitable carrier for use in a nebuliser or a suspension or solution aerosol, or may be absorbed or adsorbed onto a suitable solid carrier for use in a dry powder inhaler.

Solid compositions for rectal administration include suppositories formulated in accordance with known methods and containing at least one compound of the invention.

The percentage of active ingredient in the compositions of the invention may be varied, it being necessary that it should constitute a proportion such that a suitable dosage shall be obtained. Obviously, several unit dosage forms may be administered at about the same time. The dose employed will be determined by the physician, and depends upon the desired therapeutic effect, the route of administration and the duration of the treatment, and the condition of the patient. In the adult, the doses are generally from about 0.001 to about 50, preferably about 0.001 to about 5, mg/kg body weight per day by inhalation, from about 0.01 to about 100, preferably 0.1 to 70, more especially 0.5 to 10, mg/kg body weight per day by oral administration, and from about 0.001 to about 10, preferably 0.01 to 1, mg/kg body weight per day by intravenous administration. In each particular case, the doses will be determined in accordance with the factors distinctive to the subject to be treated, such as age, weight, general state of health and other characteristics which can influence the efficacy of the medicinal product.

The compounds according to the invention may be administered as frequently as necessary in order to obtain the desired therapeutic effect. Some patients may respond rapidly to a higher or lower dose and may find much weaker maintenance doses adequate. For other patients, it may be necessary to have long-term treatments at the rate of 1 to 4 doses per day, in accordance with the physiological requirements of each particular patient. Generally, the active product may be administered orally I to 4 times per day. Of course, for some patients, it will be necessary to prescribe not more than one or two doses per day.

Compounds of the invention may be prepared by the application or adaptation of known methods, by which is meant methods used heretofore or described in the literature, for example those described by R.C.Larock in Comprehensive Organic Transformations, VCH publishers, 1989.

In the reactions described hereinafter it may be necessary to protect reactive functional groups, for example hydroxy, amino, imino, thio or carboxy groups, where these are desired in the final product, to avoid their unwanted participation in the reactions. Conventional protecting groups may be used in accordance with standard practice, for examples see T.W. Greene and P.G.M.Wuts in "Protective Groups in Organic Chemistry" John Wiley and Sons, 1991.

Thus, for example, compounds of formula (I), wherein the Y moiety is carboxy, may be prepared by hydrolysis of esters of formula (I), wherein the Y moiety is -CO₂R¹² group (in which R¹² is alkyl, alkenyl or arylalkyl). The hydrolysis may conveniently be carried out by alkaline hydrolysis using a base, such as an alkali metal hydroxide, e.g. lithium hydroxide, or an alkali metal carbonate, e.g. potassium carbonate, in the presence of an aqueous/organic solvent mixture, using organic solvents such as dioxan, tetrahydrofuran or methanol, at a temperature from about ambient to about reflux. The hydrolysis of the esters may also be carried out by acid hydrolysis using an inorganic acid, such as hydrochloric acid, in the presence of an aqueous/inert organic solvent mixture, using organic solvents such as dioxan or tetrahydrofuran, at a temperature from 50°C to 80°C.

As another example compounds of formula (I), wherein the Y moiety is carboxy, may be prepared by acid catalysed removal of the tert-butyl group of tert-butyl esters of formula (I), wherein the Y moiety is -CO₂R¹² (in which R¹² is -^{t}Bu), using standard reaction conditions.

In a process A compounds of formula (I), wherein the Y moiety is carboxy, may be prepared by coupling of an acid (or an acid halide) with an amine to give an amide bond within the group -R⁹-CON(=R¹¹)-CH₂-CON(R⁴)-CH₂-CH₂-Y using standard peptide coupling procedures as described hereinafter.

As an example of process A, compounds of formula (I), wherein the Y moiety is carboxy, may be prepared by:-
(i) treating Wang resin (4-hydroxymethylphenoxylated styrene divinylbenzene copolymer) with acryloyl chloride, in the presence of a tertiary amine, such as diisopropylethylamine, in an inert solvent, such as dichloromethane, at a temperature at about room temperature, to give Resin A: 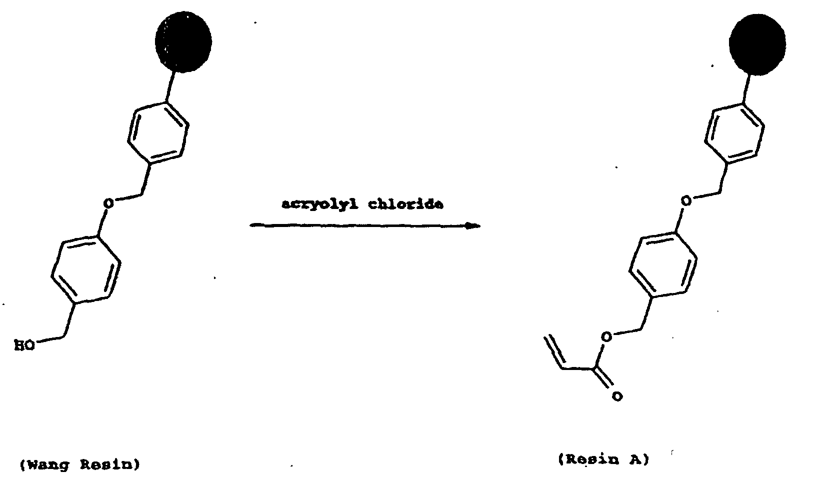 where 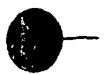 represents the polymeric core comprising polystyrene crosslinked with 1% to 2% divinylbenzene.
(ii) reaction of Resin A with amines of formula (II), wherein R⁴ is as defined hereinbefore, in the presence of a base, such as a tertiary organic base, for example diisopropylethylamine, in dimethylformamide and at a temperature at about room temperature, to give Resin 1, in which R⁴ and 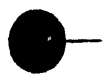 are as defined hereinbefore: 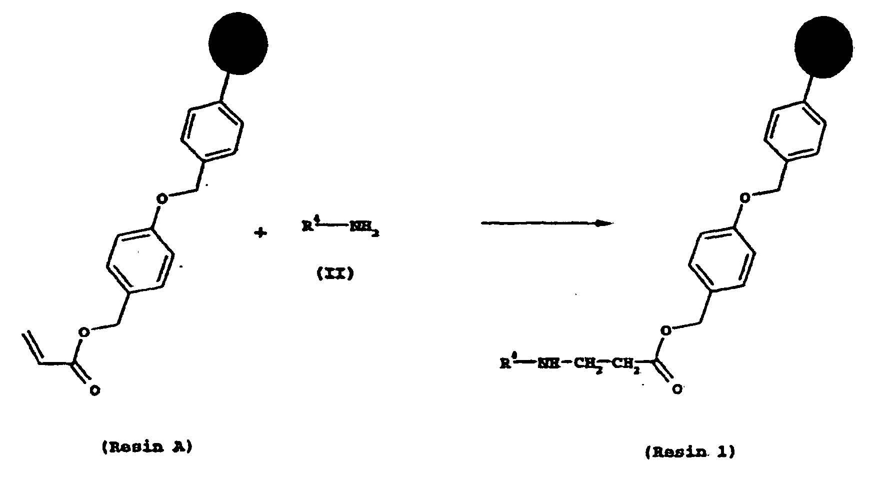
(iii) reaction of Resin 1 with compounds of formula (III) 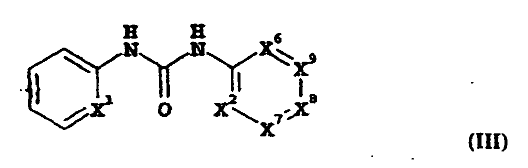 wherein X¹ and X² are as hereinbefore defined, one of X⁸ and X⁹ represents CR¹⁴ in which R¹⁴ is L¹-(CH₂)-COOH whereby L¹ is R⁹-R¹⁰ with R⁹ as defined before and R¹⁰ =C(=O)NH hereinbefore defined)], and the other and X⁶ and X⁷ represent CH, in the presence of O-(7-azabenzotriazol-1-yl)-1,1,3,3-tetramethyluronium hexafluorophosphate and diisopropylethylamine in dimethylformamide, at room temperature, to give resin 2 wherein R⁴, R⁹ and 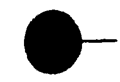 are as hereinbefore defined and R¹⁵ represents a monovalent radical derived from (III) in which X¹, X², X⁶ and X⁷ are as hereinbefore defined, and X⁸ and X⁹ represent CH by removing one of the hydrogen atoms from X⁸ or X⁹: 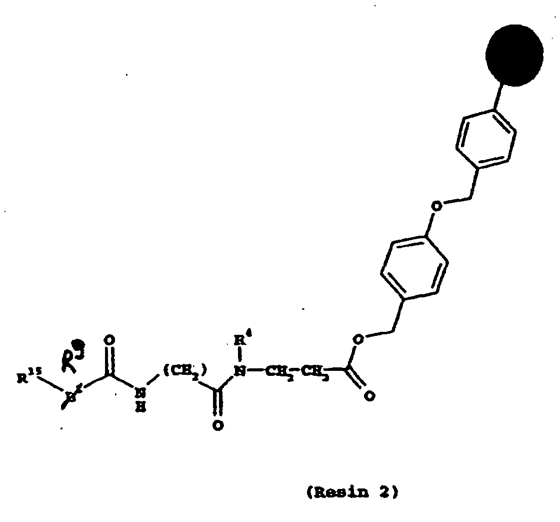
(iv) Resin 2 may then be treated with trifluoroacetic acid in an inert solvent such as dichloromethane and at a temperature at about room temperature.

As another example of process A, compounds of formula (I), wherein X1 and X2 are as hereinbefore defined, and where R11 and Y are H and carboxy respectively, may be prepared by:-
(i) treating Resin 1, wherein R⁴ and 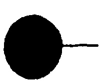 are as hereinbefore defined, with a suitably protected amino-acid of formula (IV), wherein R¹³ is a suitable amino protecting group (such as 9-fluorenylmethoxycarbonyl, FMOC) and n is as hereinbefore defined, in the presence of O-(7-azabenzotriazol-1-yl)-1,1,3,3-tetramethyluronium hexafluorophosphate and diisopropylethylamine in dimethylformamide, at room temperature to give Resin 3, wherein R⁴ and 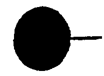 are as hereinbefore defined: 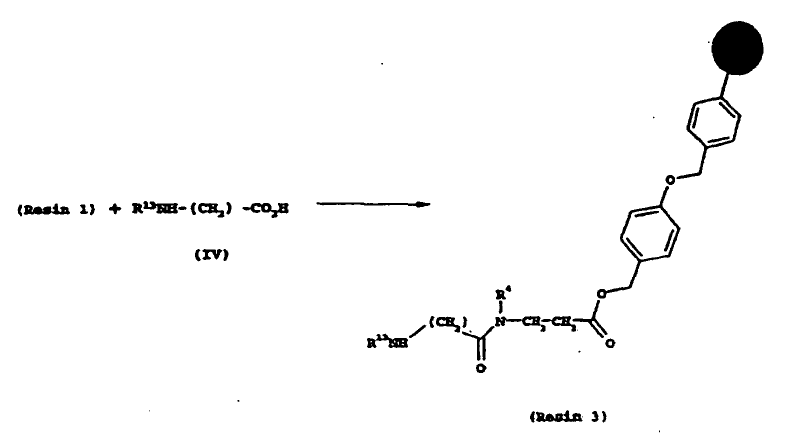
(ii) The resetting Resin 3, may then be deprotected, for example by treating with piperidine in dimethylformamide, at room temperature, to give Resin 4, wherein R⁴, n and 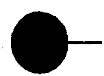 are as hereinbefore defined: 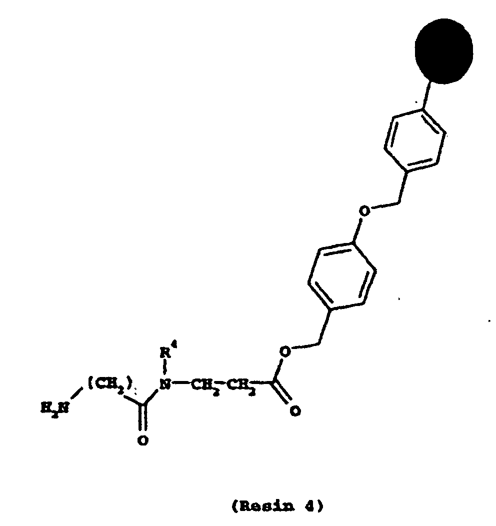
(iii) Resin 4 may then be treated with compounds of general formula (III), wherein X¹, X², X⁶ and X⁷ are as hereinbefore defined, one of X⁸ and X⁹ represents C-R⁹-CO₂H (where R⁹ is as hereinbefore defined)], and the other represents CH, using standard peptide coupling procedures, for example those described hereinabove, to give resin 5, wherein R⁴, R⁹, R¹⁵ and 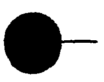 are as hereinbefore defined: 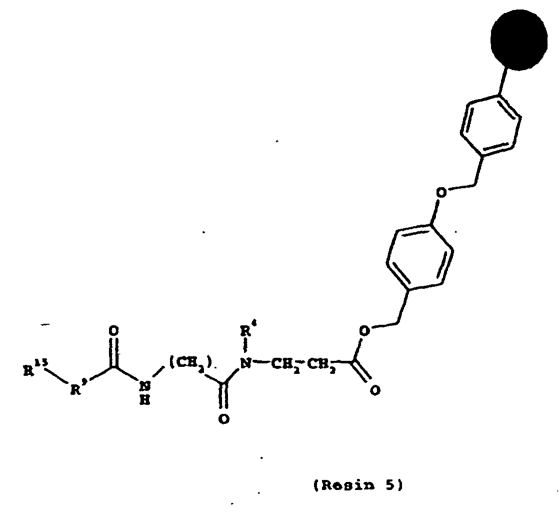
(iv) Resin 5 may then be treated with trifluoroacetic acid in an inert solvent such as dichloromethane and at a temperature at about room temperature.

As another example of process A, compounds of formula (I) may be prepared by:-
(i) treating Resin 1, wherein R⁴ and 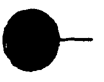 are as hereinbefore defined, with compounds of formula (V), wherein n is as hereinbefore defined and X¹⁰ is a halogen atom, preferably bromine, using standard peptide coupling procedures, for example those described hereinabove, to give Resin 6: 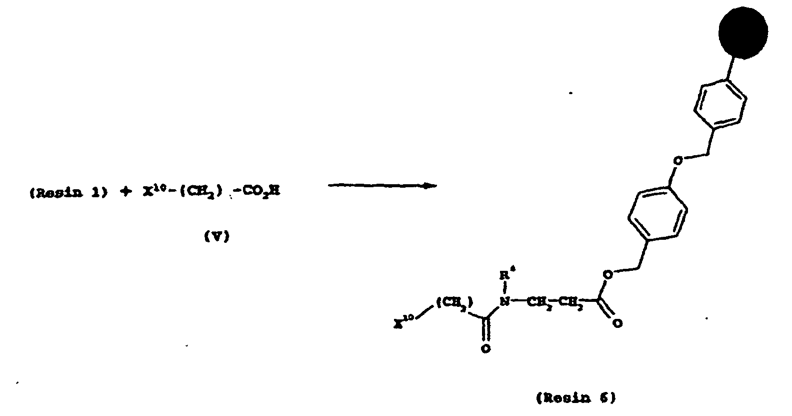
(ii) Reaction of Resin 6 with amines of formula (VI), wherein R¹¹ is as hereinbefore defined, in an inert solvent such as dimethyl sulphoxide, and at a temperature at about 80°C, to give Resin 7, wherein R⁴, R¹¹ and 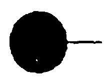 are as hereinbefore defined: 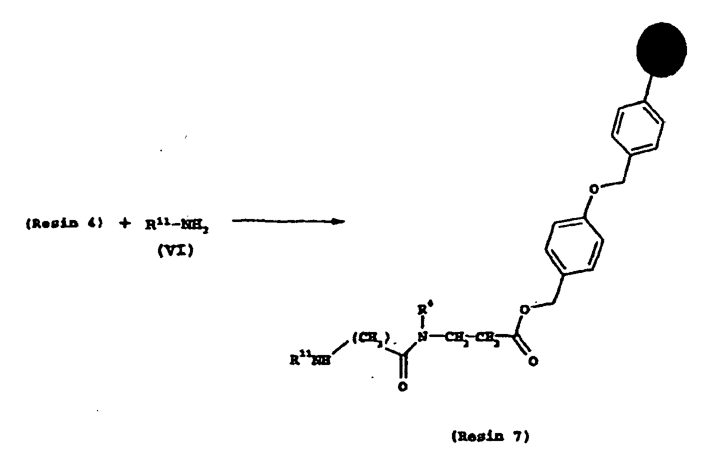
(iii) Resin 7 may then be treated with compounds of general formula (III), wherein X¹, X², X⁶ and X⁷ are as hereinbefore defined, one of X⁸ and X⁹ represents C-R⁹-CO₂H (where R⁹ is as hereinbefore defined)], and the other represents CH, using standard peptide coupling procedures, for example those described hereinabove, to give resin 8 wherein R⁴, R⁹, R¹¹, R¹⁵ and 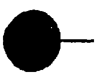 are as hereinbefore defined: 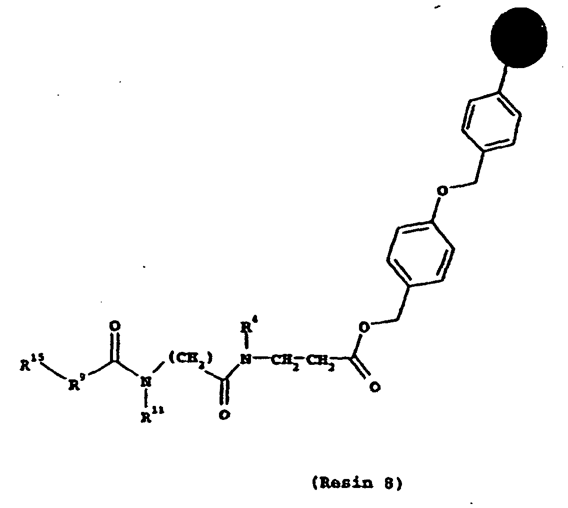
(iv) Resin 8 may then be treated with trifluoroacetic acid in an inert solvent such as dichloromethane and at a temperature at about room temperature.

Esters of formula (I), wherein Y is a -CO2R12 group (in which R12 is as hereinbefore defined), may be prepared by reaction of compounds of formula (III), wherein X¹, X², X⁶ and X⁷ are as hereinbefore defined, one of X⁸ and X⁹ represents C-R⁹-C(=O)X¹¹ (where X¹¹ is a hydroxy group, or a halogen, preferably chlorine, atom)] and the other represents CH, with amines of formula (VII):-

R⁴-HN-CH₂-CH₂-CO₂R¹² (VII)

wherein R⁴ and R¹² are as hereiabefore defined. When X¹¹ is a hydroxy group the reaction may be carried out using standard peptide coupling procedures as described hereinbefore. When X¹¹ is a halogen atom the reaction may be carried out with the aid of a base, such pyridine, preferably in a solvent such as tetrahydrofuran and at a temperature at about room temperature.

According to a further process B compounds of the invention may be prepared by interconversion of other compounds of the invention.

As another example of the interconversion process, compounds of formula (I) containing sulphoxide linkages may be prepared by the oxidation of corresponding compounds containing -S- linkages. For example, the oxidation may conveniently be carried out by means of reaction with a peroxyacid, e.g. 3-chloroperbenzoic acid, preferably in an inert solvent, e.g. dichloromethane, preferably at or near room temperature, or alternatively by means of potassium hydrogen peroxomonosulphate in a medium such as aqueous methanol, buffered to about pH5, at temperatures between about 0°C and room temperature. This latter method is preferred for compounds containing an acid-labile group.

As another example of the Interconversion process, compounds of formula (I) containing sulphone linkages may be prepared by the oxidation of corresponding compounds containing -S- or sulphoxide linkages. For example, the oxidation may conveniently be carried out by means of reaction with a peroxyacid, e.g. 3-chloroperbenzoic add, preferably in an inert solvent, e.g. dichloromethane, preferably at or near room temperature.

It will be appreciated that compounds of the present invention may contain asymmetric centres. These asymmetric centres may independently be in either the R or S configuration. It will be apparent to those skilled in the art that certain compounds of the invention may also exhibit geometrical isomerism. It is to be understood that the present invention includes individual geometrical isomers and stereoisomers and mixtures thereof, including racemic mixtures, of compounds of formula (I) hereinabove. Such isomers can be separated from their mixtures, by the application or adaptation of known methods, for example chromatographic techniques and recrystallisation techniques, or they are separately prepared from the appropriate isomers of their intermediates.

According to a further feature of the invention, acid addition salts of the compounds of this invention may be prepared by reaction of the free base with the appropriate acid, by the application or adaptation of known methods. For example, the acid addition salts of the compounds of this invention may be prepared either by dissolving the free base in water or aqueous alcohol solution or other suitable solvents containing the appropriate acid and isolating the salt by evaporating the solution, or by reacting the free base and acid in an organic solvent, in which case the silt separates directly or can be obtained by concentration of the solution.

The acid addition salts of the compounds of this invention can be regenerated from the salts by the application or adaptation of known methods. For example, parent compounds of the invention can be regenerated from their acid addition salts by treatment with an alkali, e.g. aqueous sodium bicarbonate solution or aqueous ammonia solution.

Compounds of this invention can be regenerated from their base addition salts by the application or adaptation of known methods. For example, parent compounds of the invention can be regenerated from their base addition salts by treatment with an acid, e.g. hydrochloric add.

Compounds of the present invention may be conveniently prepared, or formed during the process of the invention, as solvates (e.g. hydrates). Hydrates of compounds of the present invention may be conveniently prepared by recrystallisation from an aqueous/organic solvent mixture, using organic solvents such as dioxan, tetrahydrofuran or methanol.

According to a further feature of the invention, base addition salts of the compounds of this invention may be prepared by reaction of the free add with the appropriate base, by the application or adaptation of known methods. For example, the base addition salts of the compounds of this invention may be prepared either by dissolving the free add in water or aqueous alcohol solution or other suitable solvents containing the appropriate base and isolating the salt by evaporating the solution, or by reacting the free acid and base in an organic solvent, in which case the salt separates directly or can be obtained by concentration of the solution.

The starting materials and intermediates may be prepared by the application or adaptation of known methods, for example methods as described in the Reference Examples or their obvious chemical equivalents.

Compounds of formula (III), wherein X¹, X², X⁶ and X⁷ are as hereinbefore defined, one of X⁸ and X⁹ represents C-R⁹-CO₂H (in which R⁹ is as described above, or a suitably protected derivative thereof) and the other represents CH, may be prepared by the application or adaptation of methods described in the specification of International Patent Application Publication No. WO 96/22966.

Intermediates of formulae (Resin 1), (Resin 2), (Resin 3), (Resin 4), (Resin 5), (Resin 6), (Resin 7) and (Resin 8) are compounds and, as such, they constitute further features of the present invention.

The present invention is further Exemplified but not limited by the following illustrative Examples and Reference Examples.

In the nuclear magnetic resonance spectra (NMR) the chemical shifts are expressed in ppm relative to tetramethylsilane. Abbreviations have the following significances: s = singlet; d = doublet; t = triplet; m = multiptet; dd = doublet of doublets; b = broad.

Mass spectra (MS) were recorded on a Micromass Platform II mass spectrometer fitted with an Electrospray source and an HP1100 liquid chromatograph; using a mixture of acetonitrile and water (1:1, v/v) as the mobile phase, a flow rate of 0.3 ml/minute, an injection volume of 20µl, a run time of 2.0 minutes, a scan range of 150 - 850 Daltons Positive/Negative, a scan time of 2.0 seconds, an ESI voltage of 3.5Kv, an ESI pressure of 20n/m2 Nitrogen. Abbreviations have the following significances: w = weak.

### EXAMPLE 1

### Compounds A, B and C

A solution of ({[3-methoxy-4-(3-o-tolylureido)phenyl]-acetyl}-N-methylamino)-acetic acid [0.80g, Reference Example 1] and 3-[3-(2-oxo-pyrrolidin-1-yl)-prop-1-ylamino]-propionic acid ethyl ester [0.51g, Reference Example 2(c)] in dimethylformamide (25ml) was treated with [O-(7-azabenzotriazol-1-yl)-1,1,3,3,-tetramethyluronium hexafluorophosphate] (0.80g) and diisopropylethylamine (0.75ml). After stirring at room temperature for 2 hours the reaction mixture was treated with water (100ml) then extracted three times with ethyl acetate. The combined organic extracts were washed with hydrochloric acid (1M), then with brine. then dried over magnesium sulphate and then evaporated. The residual oil was subjected to flash chromatography on silica eluting with a mixture of dichloromethane and methanol (25: 1. v/v to give 3-{[({[3-methoxy-4-(3-o-tolylureido)phenyl]-acetyl}-N-methylamino)-acetyl]-[3-(2-oxopyrrolidin-1-yl)-prop-1-yl]-amino}-propionic acid ethyl ester as a colouriess oil (0.76g). The ester was dissolved in tetrahydrofuran (50ml) and then treated with lithium hydroxide hydrate (0.065g) in water (10ml). After stirring at room temperature for 2 hours the mixture was concentrated to remove the tetrahydrofuran. The residual aqueous residue was washed with ethyl acetate then acidified by addition of hydrochloric acid (1M) and then extracted three times with dichloromethane. The combined organic extracts were washed with brine, then dried over magnesium sulphate and then evaporated to give 3-{[({[3-methoxy-4-(3-o-tolylureido)phenyl]-acetyl}-N-methylamino)-acetyl]-[3-(2-oxo-pyrrolidin-1-yl)-prop-1-yl]-amino]-propionic acid as a white solid (0.58g, Compound A), m.p.73-76°C. [Elemental analysis:- C,60.69; H,6.85; N,11.79%. Calculated for C₃₀H₃₉N₅O₇ •0.67H₂O:- C,60.69; H,6.69; N,11.58%]. MS: 580 [MH]⁻. HPLC: R_{T}=9.72 minutes (gradient elution using a mixture of acetonitrile and water 1:4 to 4:1).

(b) By proceeding in a manner similar to Example 1(a) but using 3-(3-imidazol-1-yl-prop-1-ylamino)-propionic acid ethyl ester [Reference Example 2(b)] there was prepared 3-{[({[3-methoxy-4-(3-o-tolylureido)phenyl]-acetyl]-N-methylamino)-acetyl]-[3-(3-imidazol-1-yl)-prop-1-yl]-amino}-propionic acid as a white solid (Compound B), m.p. 58-62°C. MS: 563 [MH]⁻. HPLC: R_{T}=9.70 minutes (gradient elution using a mixture of acetonitrile and water 1:4 to 4: 1).

(c) By proceeding in a manner similar to Example 1(a) but using 3-(3,4-dimethoxy-benzylamino)-propionic acid ethyl ester [Reference Example 2(a)] there was prepared 3-{(3,4-dimethoxybenzyl)-[({[3-methoxy-4-(3-o-tolylureido)phenyl]-acetyl}-N-methylamino)-acetyl]-amino}-propionic acid as a white solid (Compound C), m.p. 104-106°C. [Elemental analysis:- C,62.19; H,6.43; N,8.94% Calculated for C₃₂H₃₈N₄O₈ •0.67H₂O:- C,62.13; H,6.40; N,9.05%]. MS: 605 [MH]⁻. HPLC: R_{T}=11.92 minutes (gradient elution using a mixture of acetonitrile and water 1:4 to 4:1).

### EXAMPLE 2

### Compounds D to BU

Step 1. Wang resin (3.0g, 0.92mmol/g) was allowed to swell in dichloromethane (30ml) for 15 minutes, then treated with diisopropylethylamine (1.8ml) followed by acryloyl chloride (0.9ml). The mixture was kept at room temperature for 3 hours with occasional gentle shaking then filtered to give resin A which was washed (i) three times with dichloromethane (15ml), (ii) three times with methanol (15ml), (iii) three times with dimethylformamide (15ml), (iv) three times with methanol (15ml), (v) three times with dichloromethane (15ml) and then dried in a desiccator under high vacuum for 2 hours.
Step 2. Resin A (40mg) was placed in a Jones tube. suspended in dimethylformamide (1ml) and then treated with 1-(3-aminoprop-1-yl)-2-pyrrolidinone (50mg). After standing at room temperature for 90 minutes the mixture was filtered to give resin B which was washed (i) four times with dimethylformamide (5ml), (ii) three times with methanol (5ml), (iii) dimethylformamide (5ml).
Step 3. Resin B from step 2 was treated with a solution of ({[3-methoxy-4-(3-o-tolylureido)phenyl]-acetyl}-amino]-acetic acid (30mg, Reference Example 3) in dimethyformamide (1ml), and then with a solution of [O-(7-azabenzotriazol-1-yl)-1,1,3,3,-tetramethyluronium hexafluorophosphate] (30mg) in dimethylformamide (1ml) and diisopropylethylamine (30µl). After standing at room temperature for 3 hours with occasional agitation the mixture was filtered to give resin C which was washed (i) four times with dimethylformamide (5ml), (ii) three times with methanol(5ml), (iii) four times with dichloromethane (45ml) and then dried in a desiccator under vacuum for 2 hours.
Step 4. Resin C from step 3 was treated with a mixture of dichloromethane and trifluoroacetic acid (2ml, 1:1 v/v). After standing at room temperature for 45 minutes the mixture was fettered, and the resin was washed with dichloromethane. The combined filtrate and washings were evaporated to give 3-{[({[3-methoxy-4-(3-o-tolylureido)phenyl]-acetyl}-amino)-acetyl]-acetyl]-[3-(2-oxopyrrolidin-1-yl)-prop-1-yl]-amino}-propionic acid (Compound D). MS: 568 [MH]⁺. HPLC: R_{T}=3.11 minutes, area of main peak as a percentage of the total sample =96% (gradient elution using a mixture of acetonitrile and water 3:7 to 17:3 v/v).

By proceeding in a similar manner to Example 2, but using the appropriately substituted amines in step 2, there were prepared Compounds E to BU depicted in Table 1.

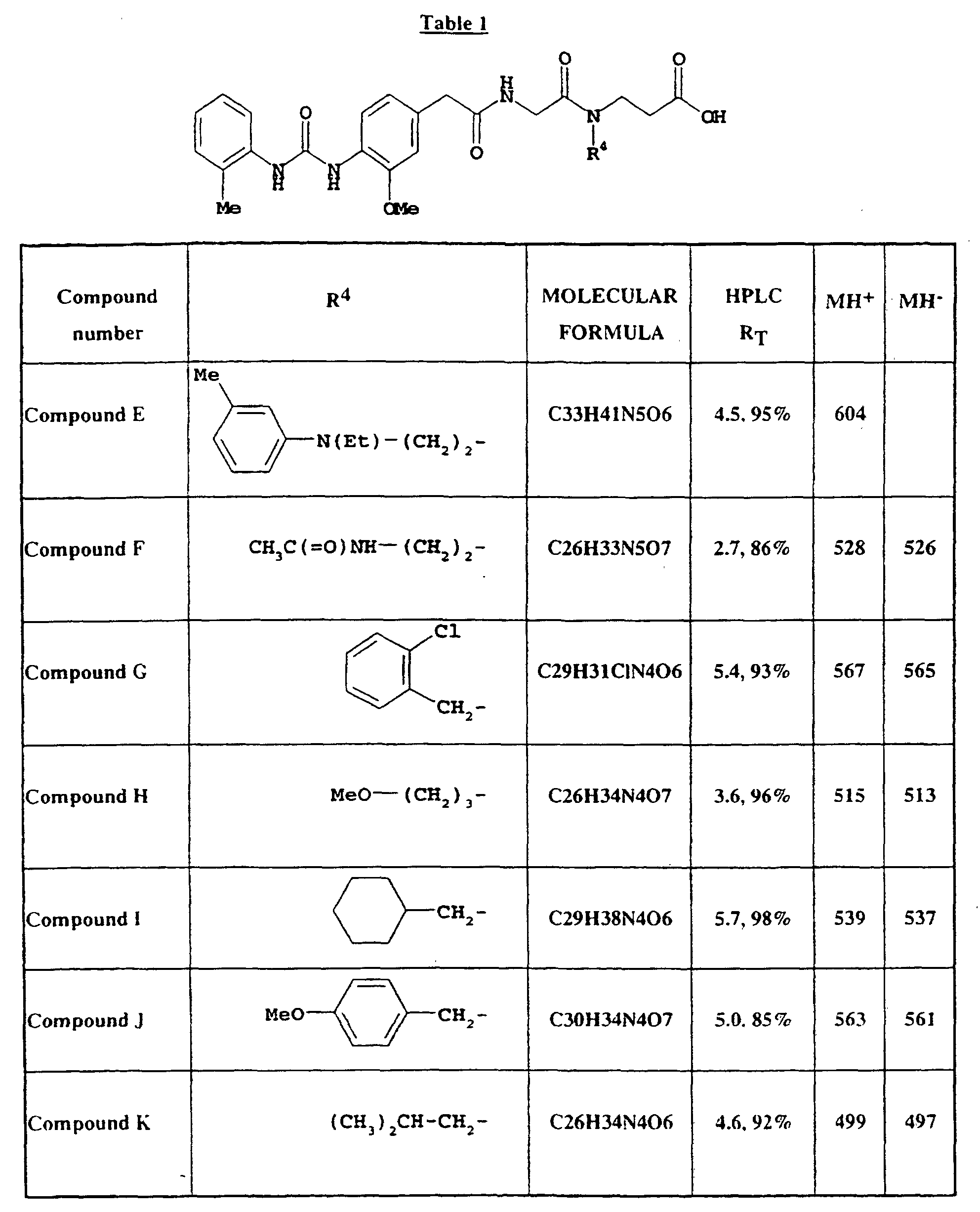

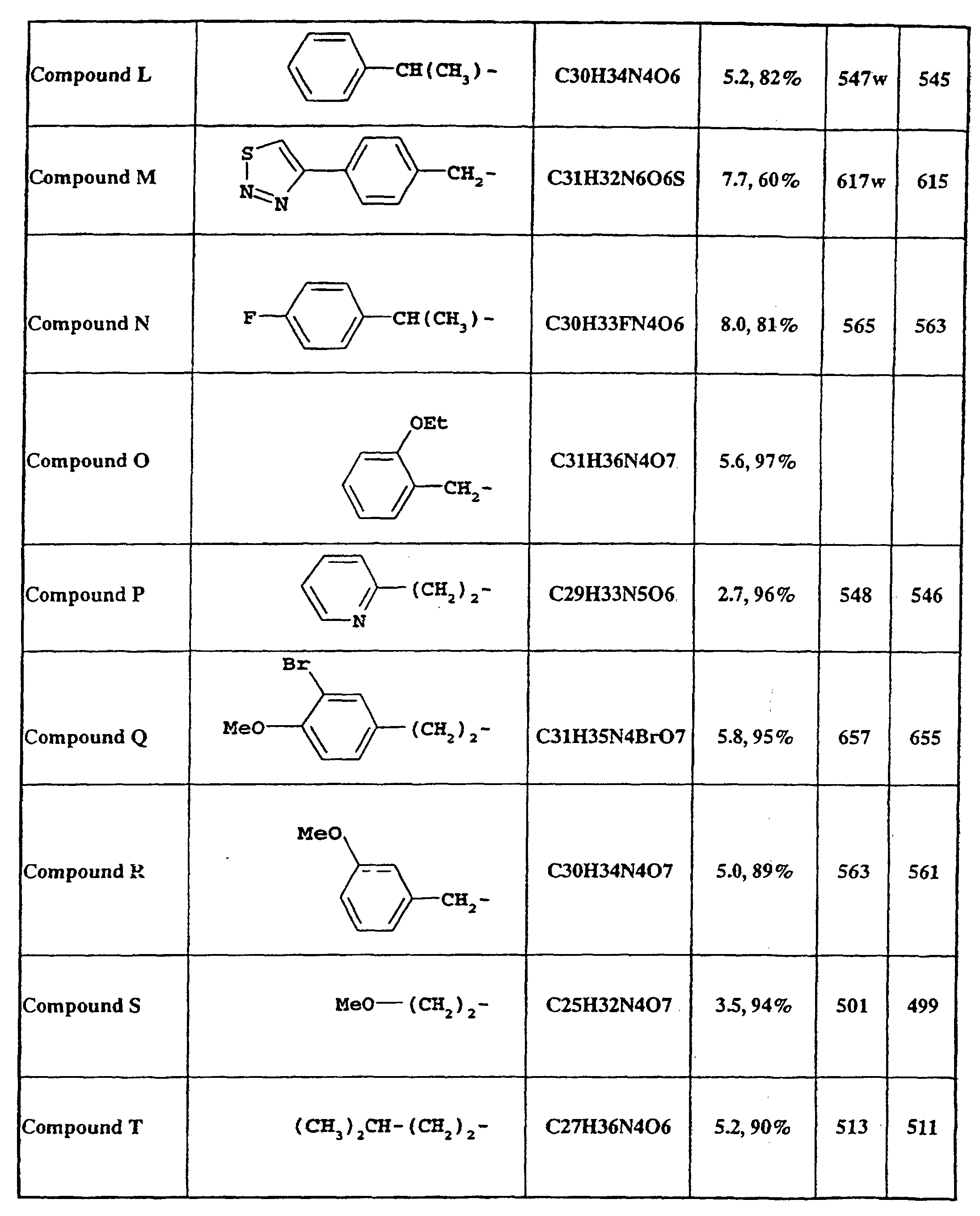

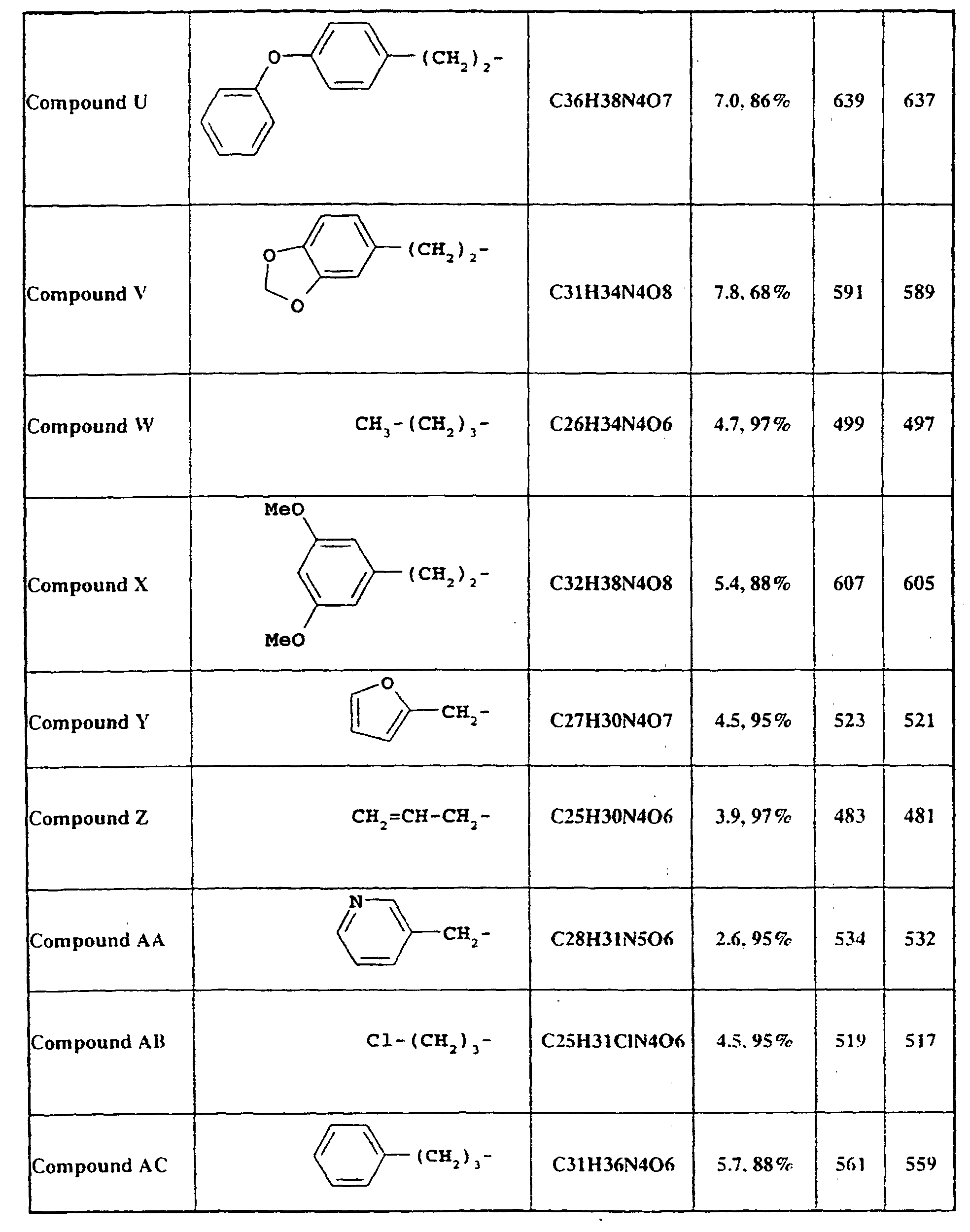

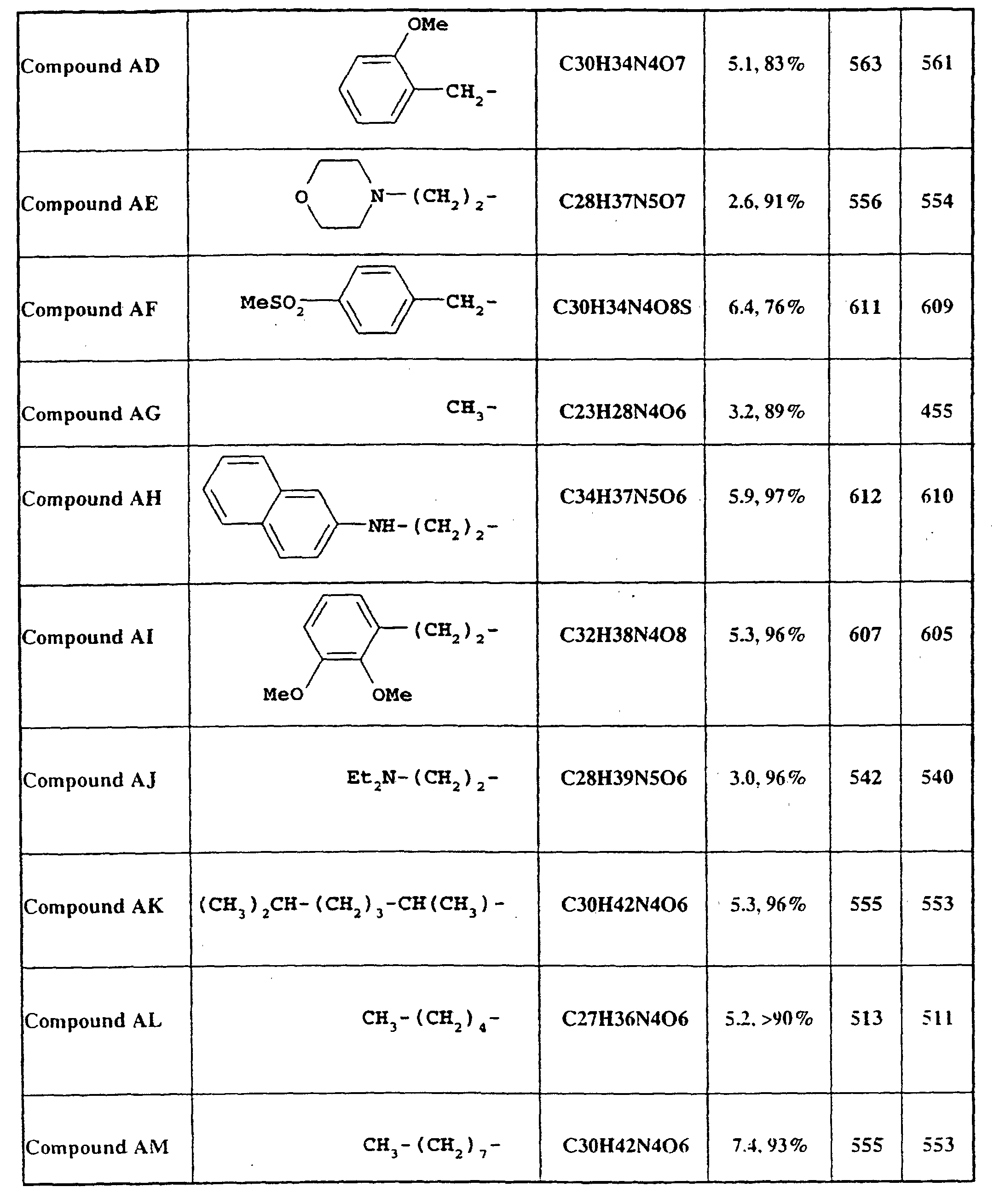

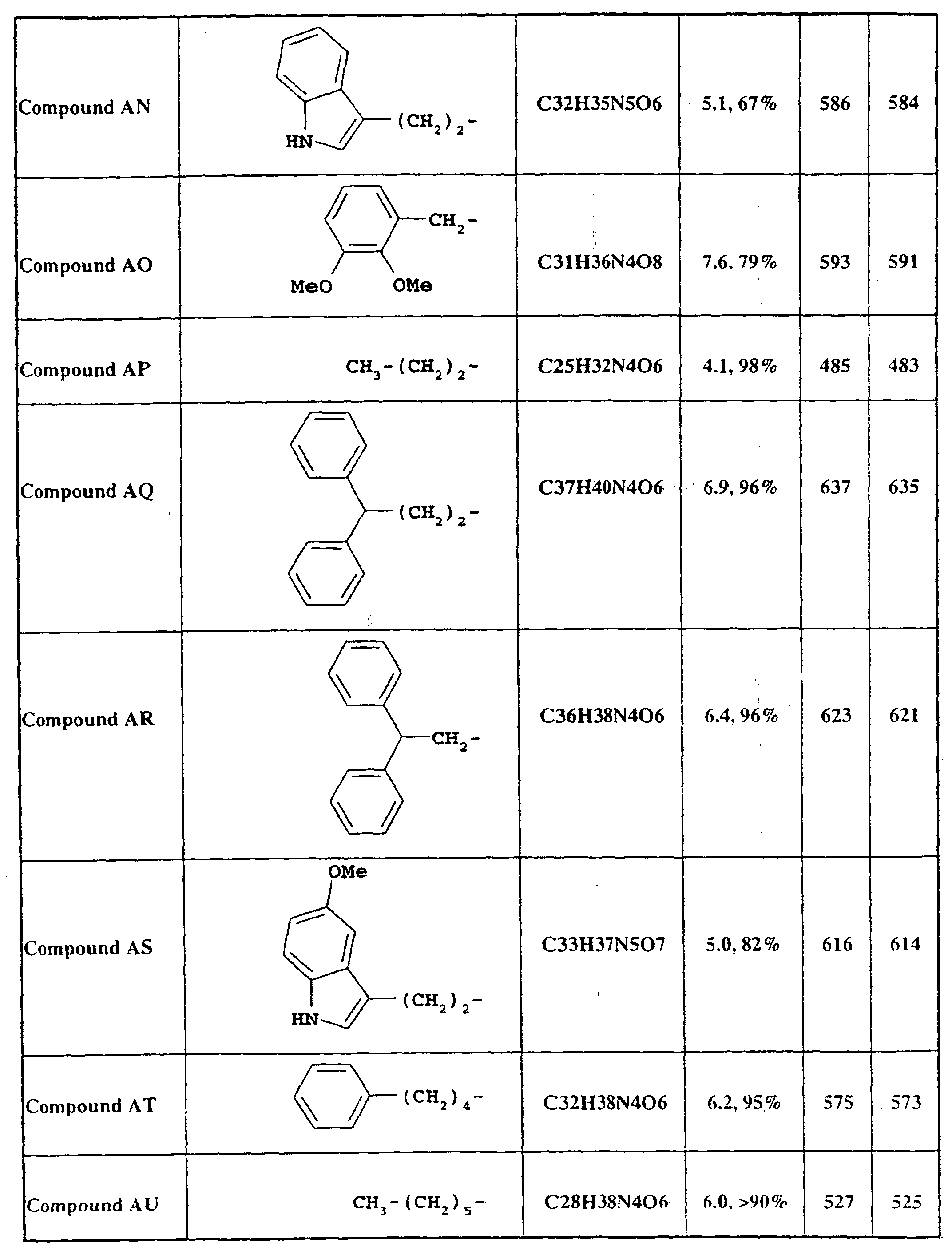

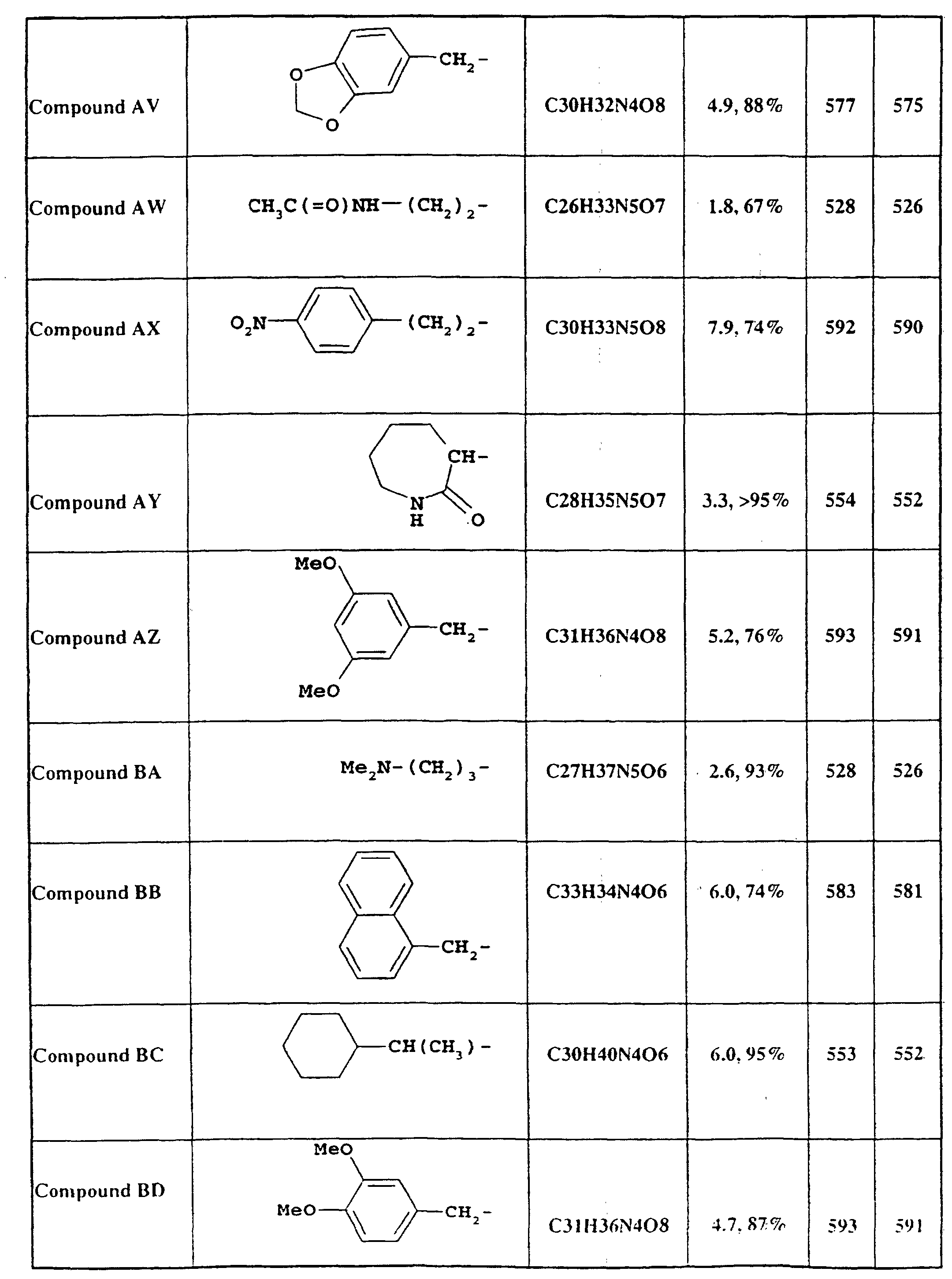

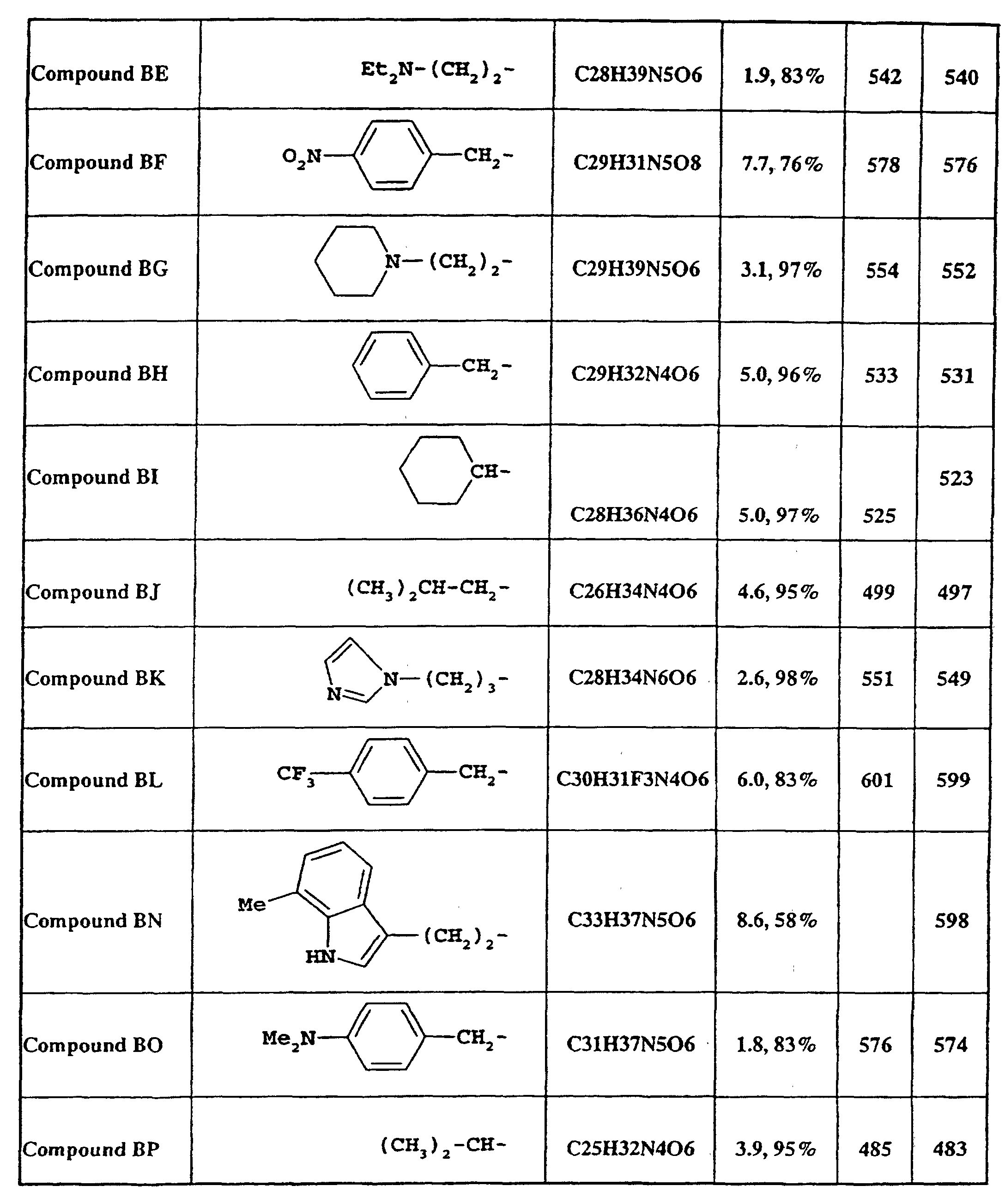

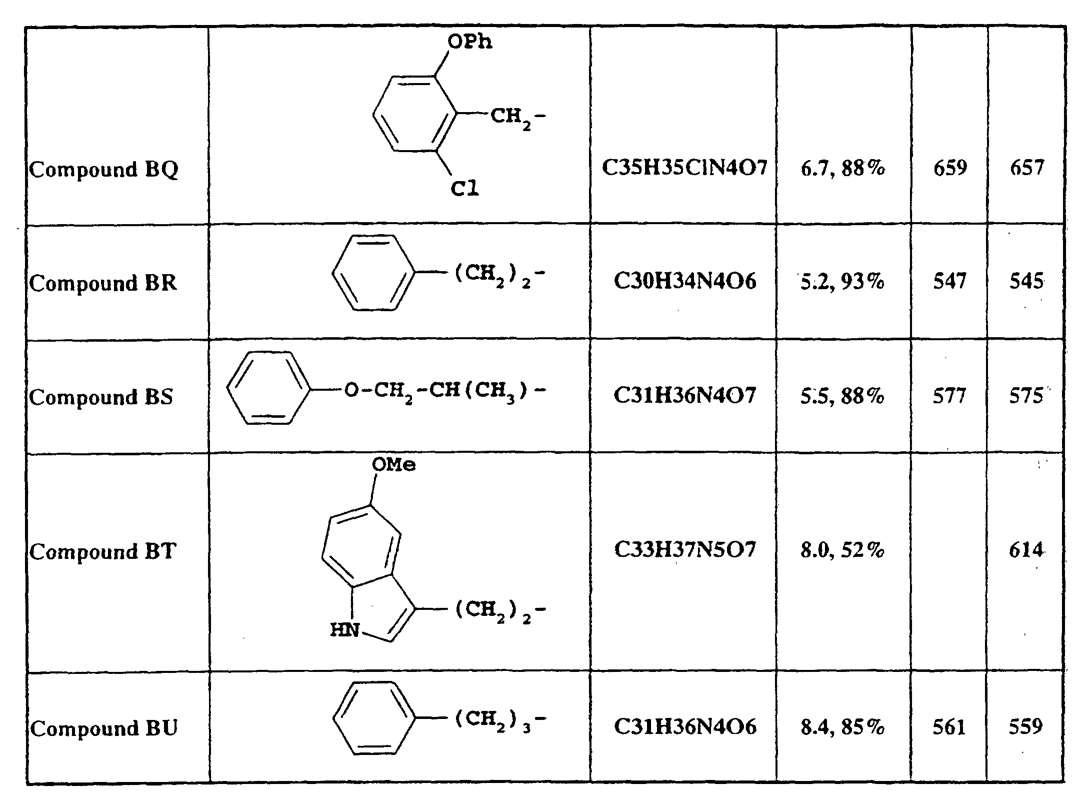

### EXAMPLE 3

### Compounds BV to FJ

Step 1. Resin B from Step 2 Example 2 was treated with bromoacetic acid (0.47g) in dimethylformamide (7ml) and diisopropylcarbodiimide (0.67ml). After standing at room temperature for 1.5 hours the mixture was filtered to give resin D which was washed.
Step 2. Resin D (100mg) was swelled with dimethyl sulphoxide (1.5ml) and then treated with propylamine (10 equivalents). After heating for 2 hours at 80°C the mixture was filtered to give resin E which was washed (i) three times with dimethylformamide, (ii) three times with tetrahydrofuran, (iii) three times with dichloromethane.
Step 3. Resin E was treated with a solution of [3-methoxy-4-(3-o-tolylureido)phenyl]-acetic acid (70 mg, Reference Example 5) in dimethylformamide (1ml), a solution of [O-(7-azahenzotriazol-1-yl)-1,1,3,3,-tetramethyluronium hexafluorophosphate] (75 mg) in dimethylformamide (1ml), and diisopropylethylamine (100µl). After standing at room temperature for 2 hours with occasional agitation the mixture was filtered to give resin F which was washed (i) four times with dimethylformamide, (ii) three times with methanol, (iii) three times with dichloromethane and then dried under vacuum.
Step 4. Resin F was treated with a mixture of dichloromethane and trifluoroacetic acid (2ml, 1:1 v/v) and allowed to stand for 45 minutes. The mixture was filtered and the resin was washed with dichloromethane. The combined filtrate and washings were evaporated to give 3-{[({[3-methoxy-4-(3-o-tolylureido)phenyl]-acetyl)-prop-1-ylaminol-acetyl]-[3-(2-oxopyrrolidin-1-yl)-prop-1-yl]-amino}-propionic acid (Compound BV).

By proceeding in a similar manner to Example 3, but using the appropriately substituted amines in step 1 and the appropriately substituted amines step 3, there were prepared Compounds BW to FJ depicted in Table 2.

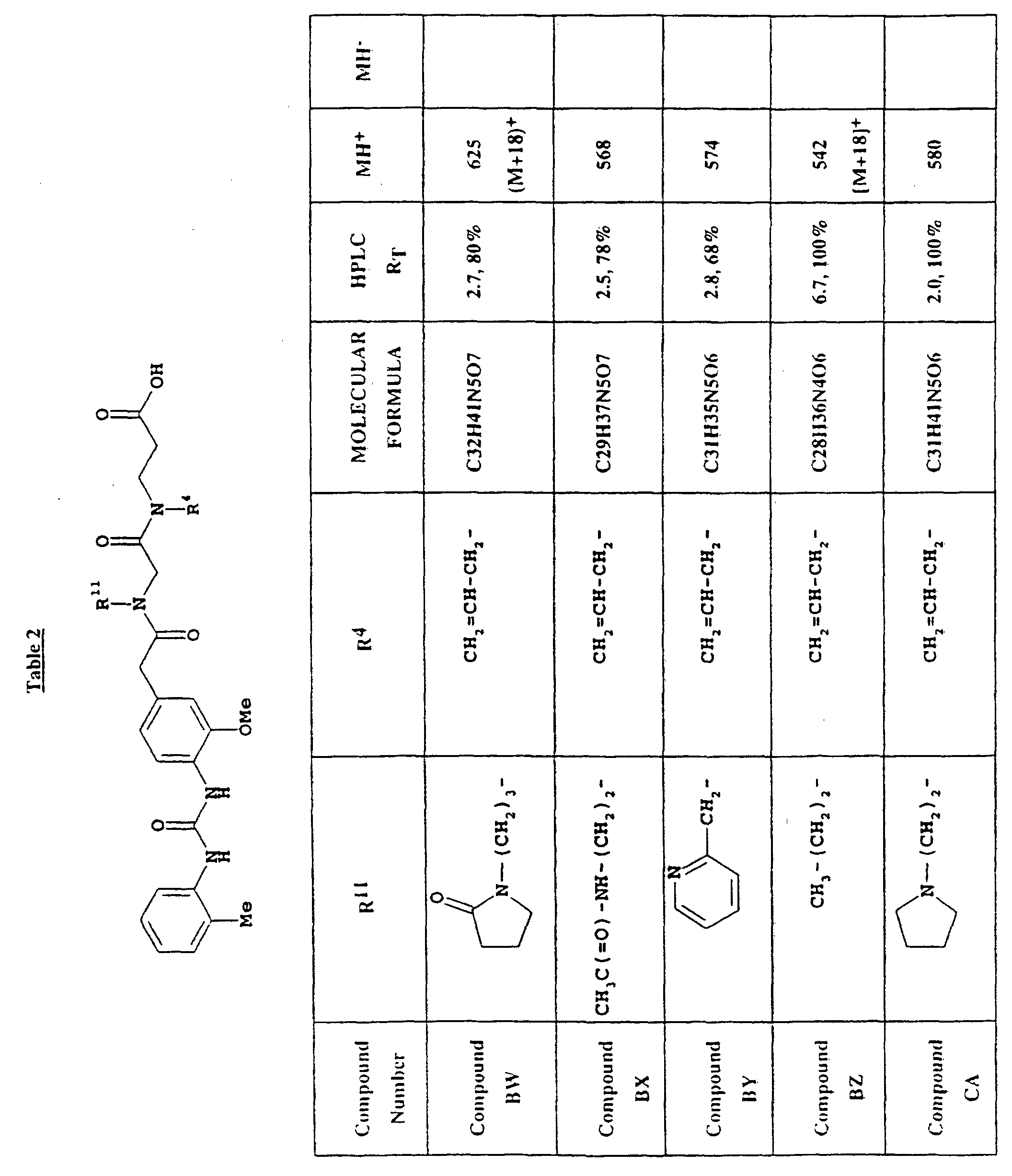

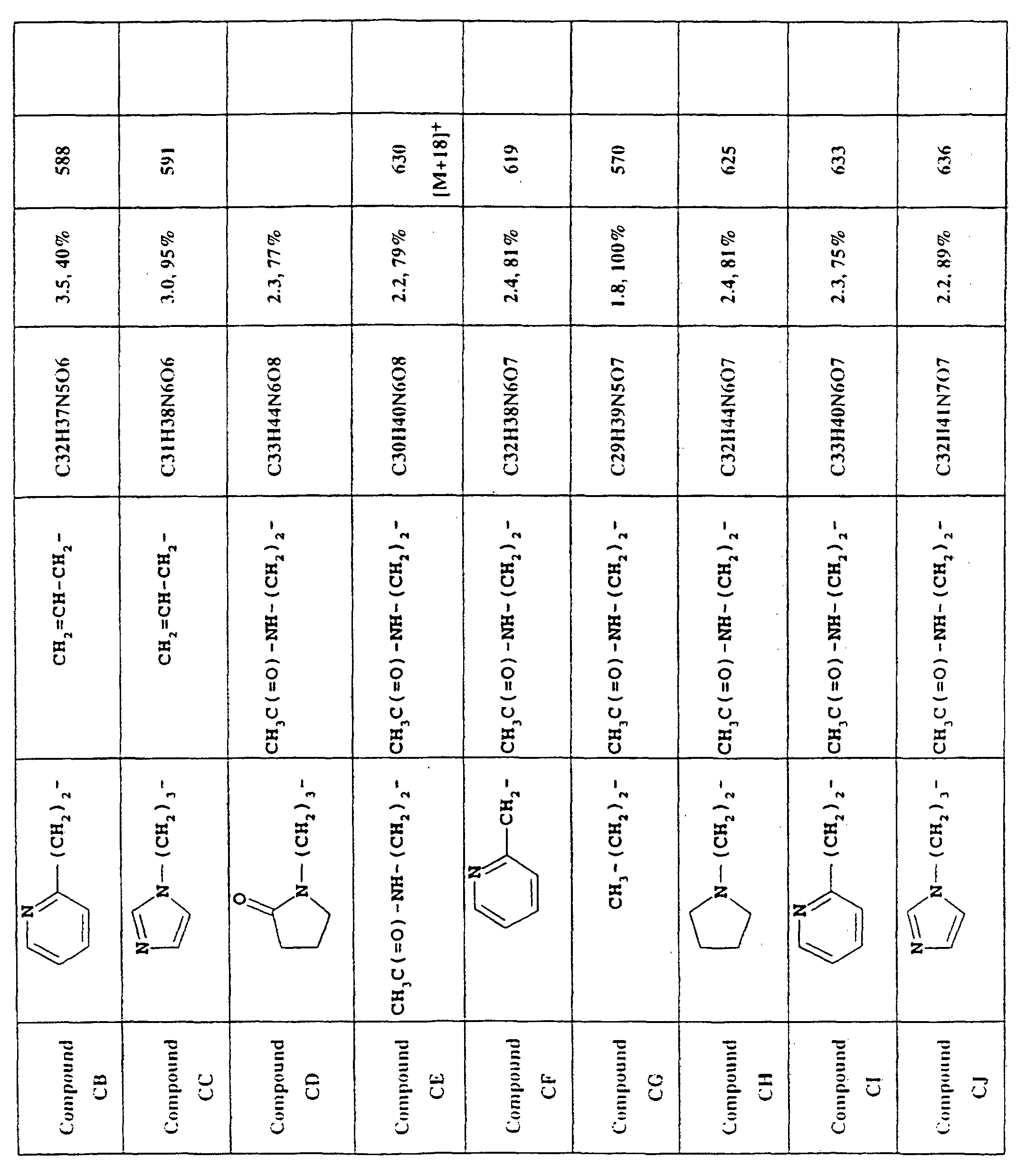

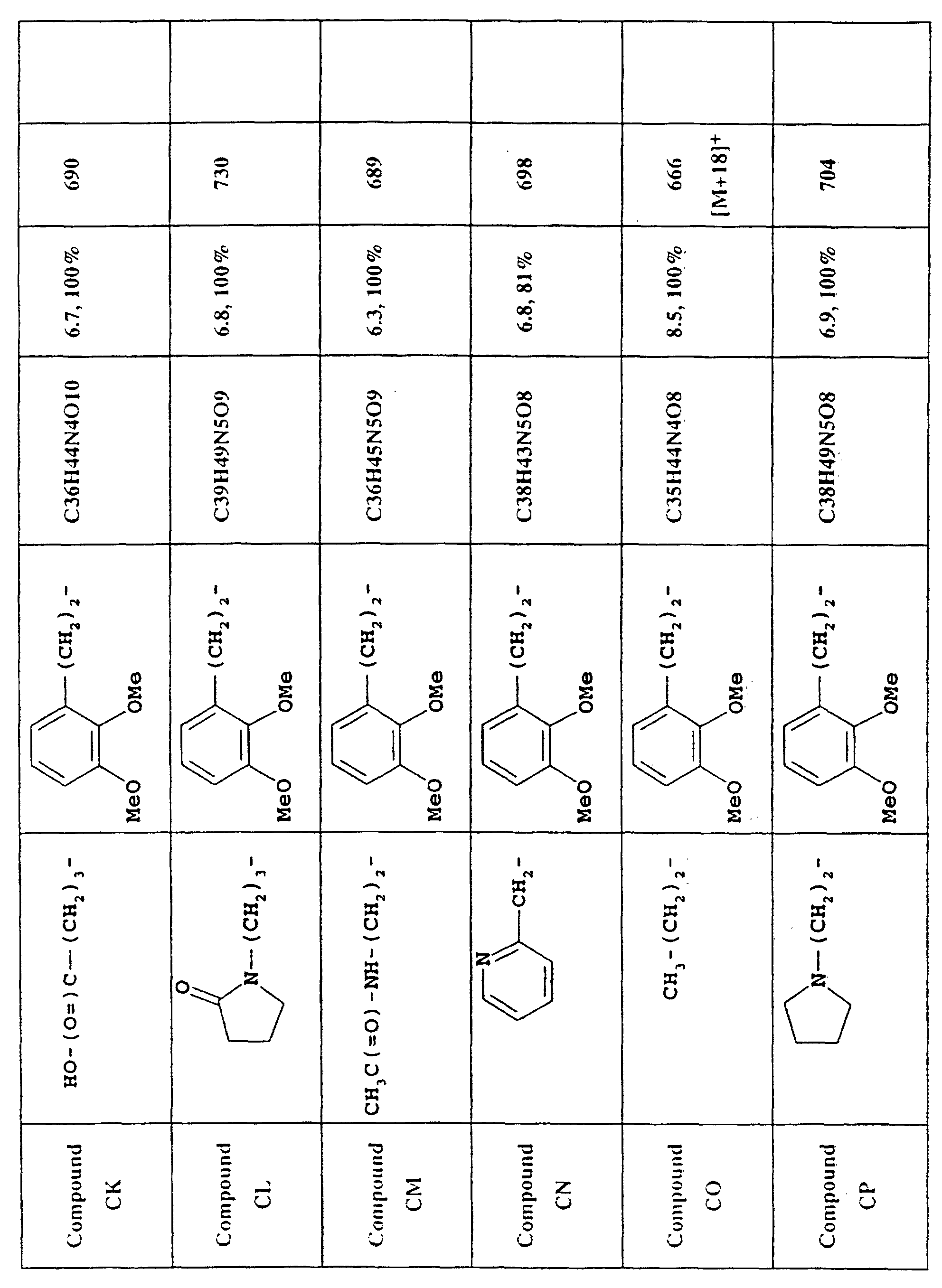

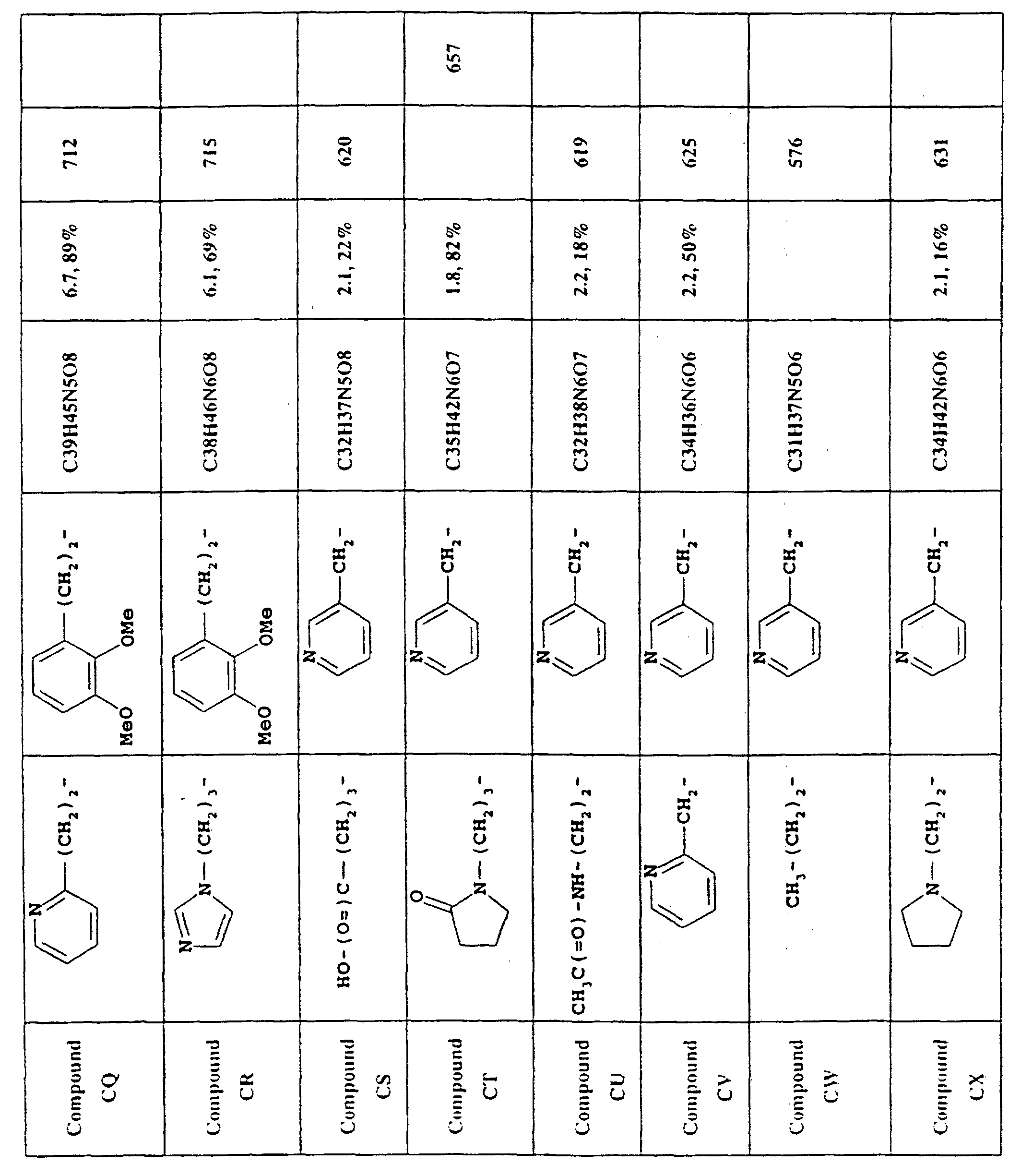

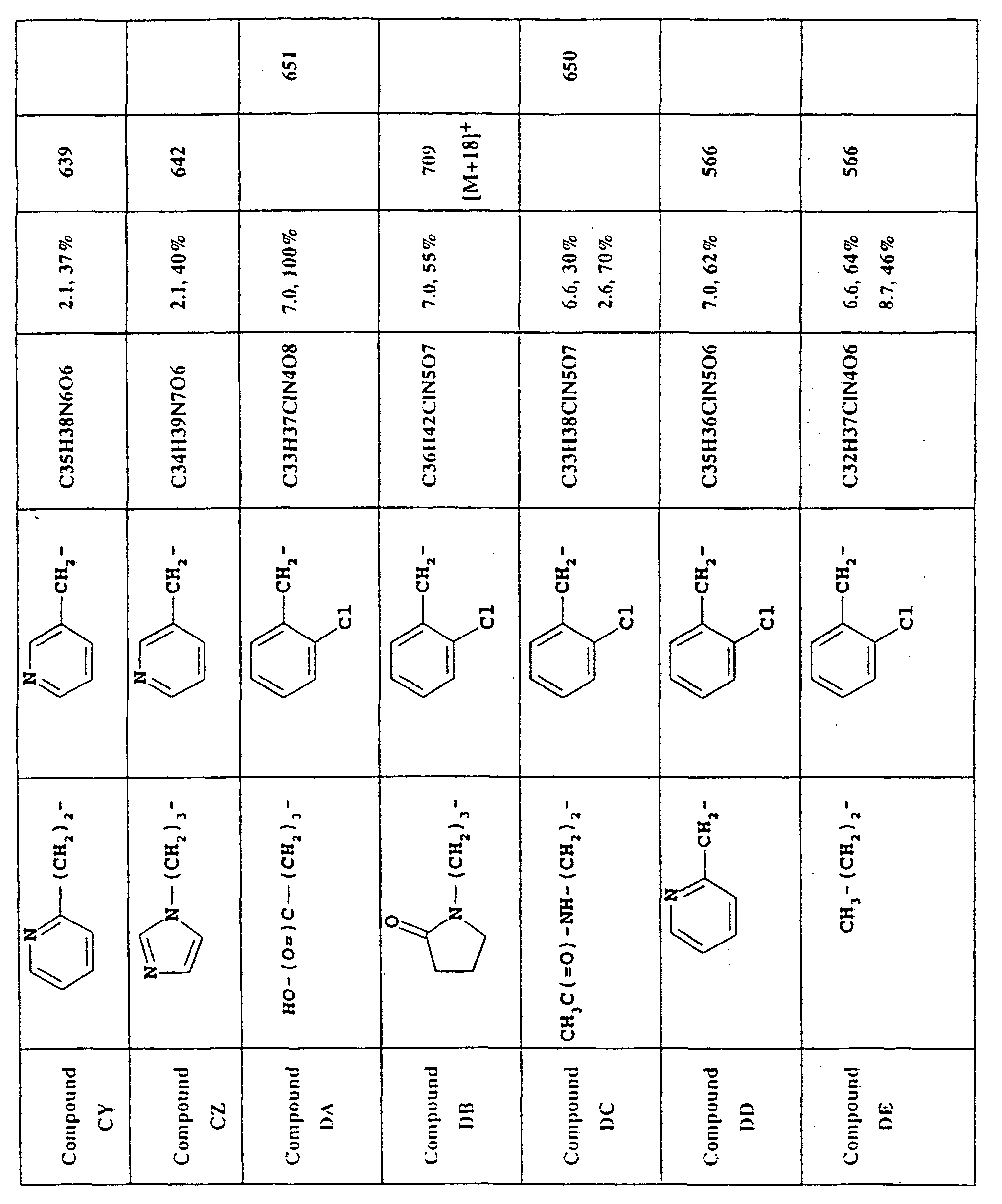

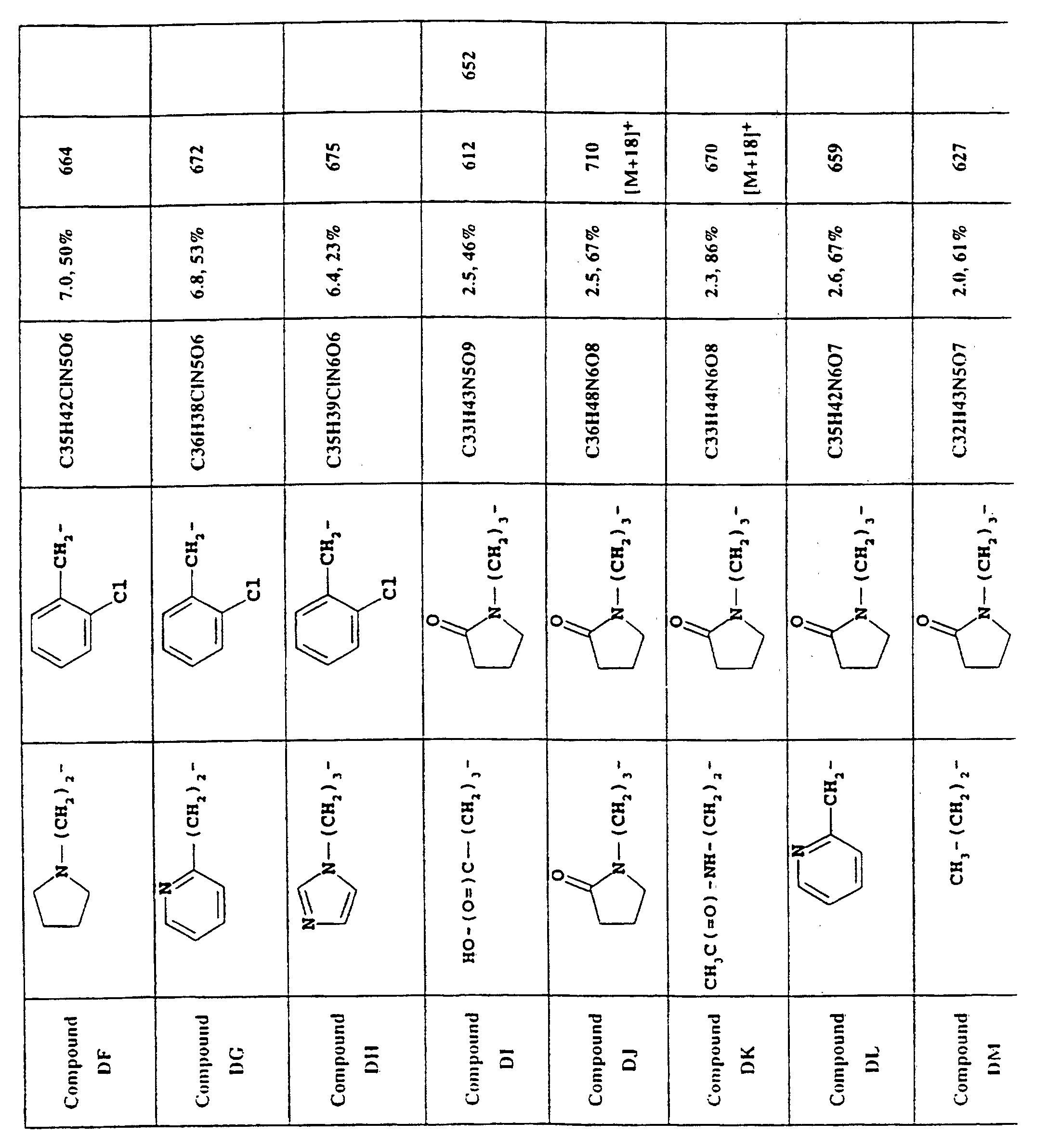

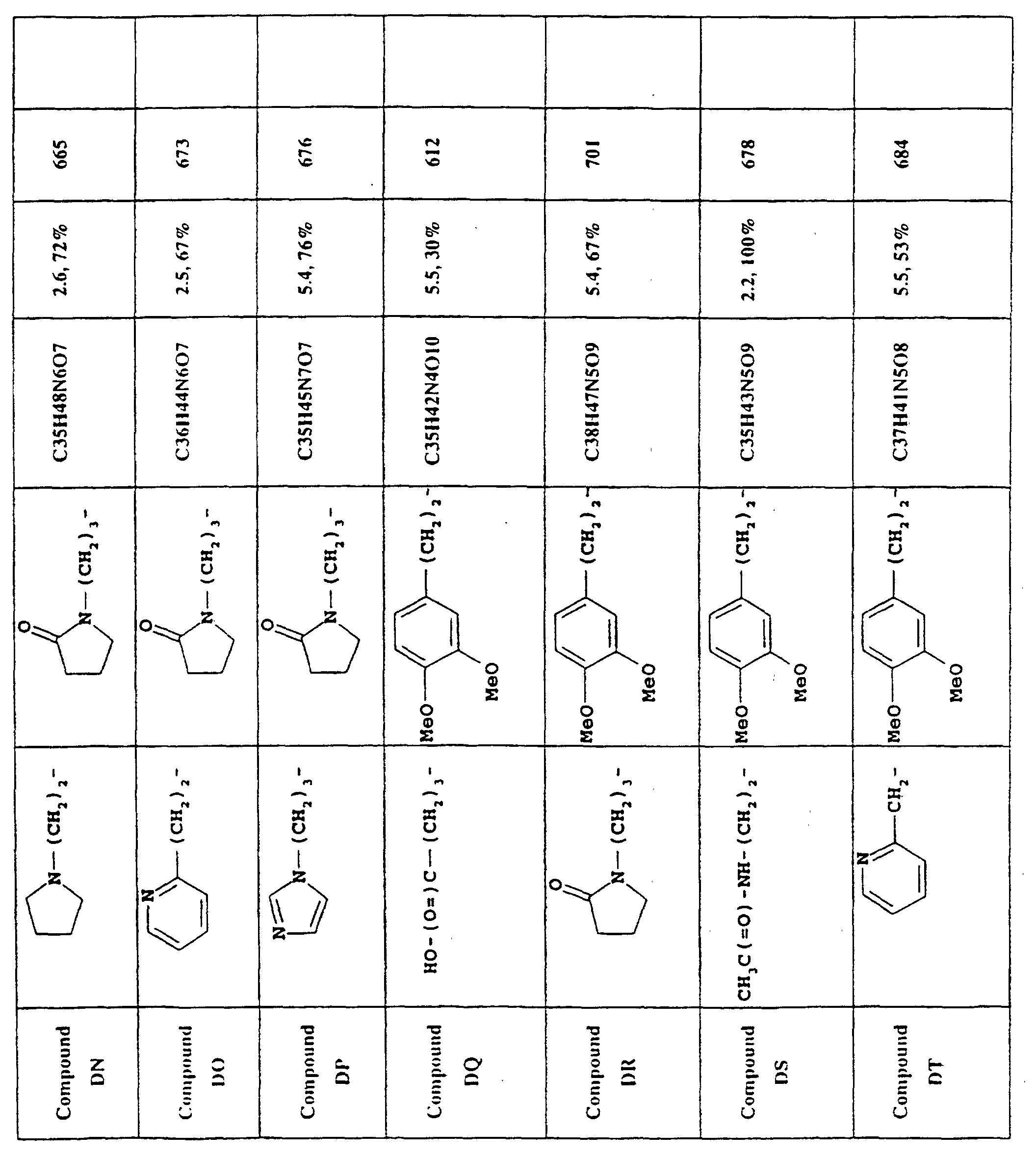

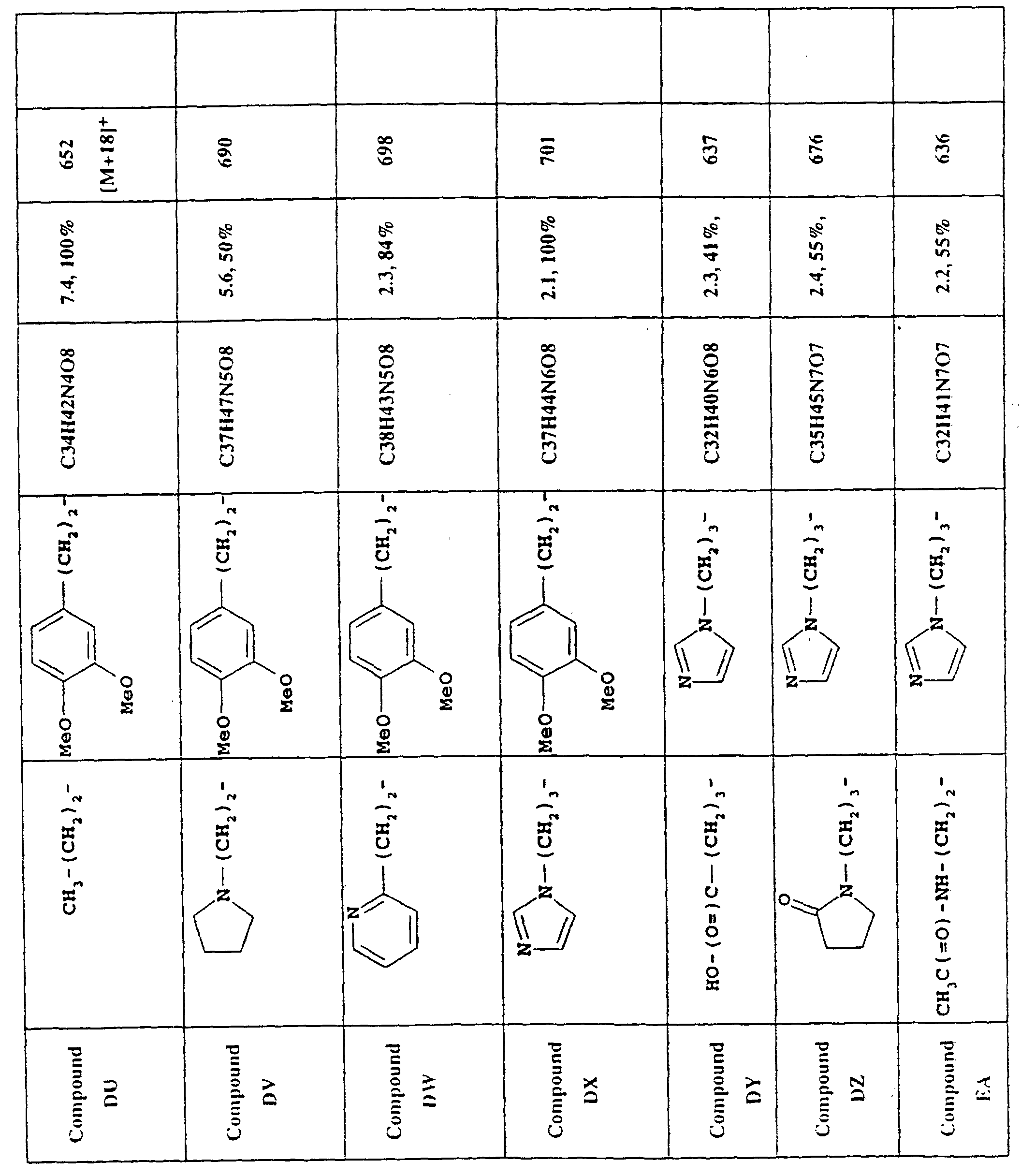

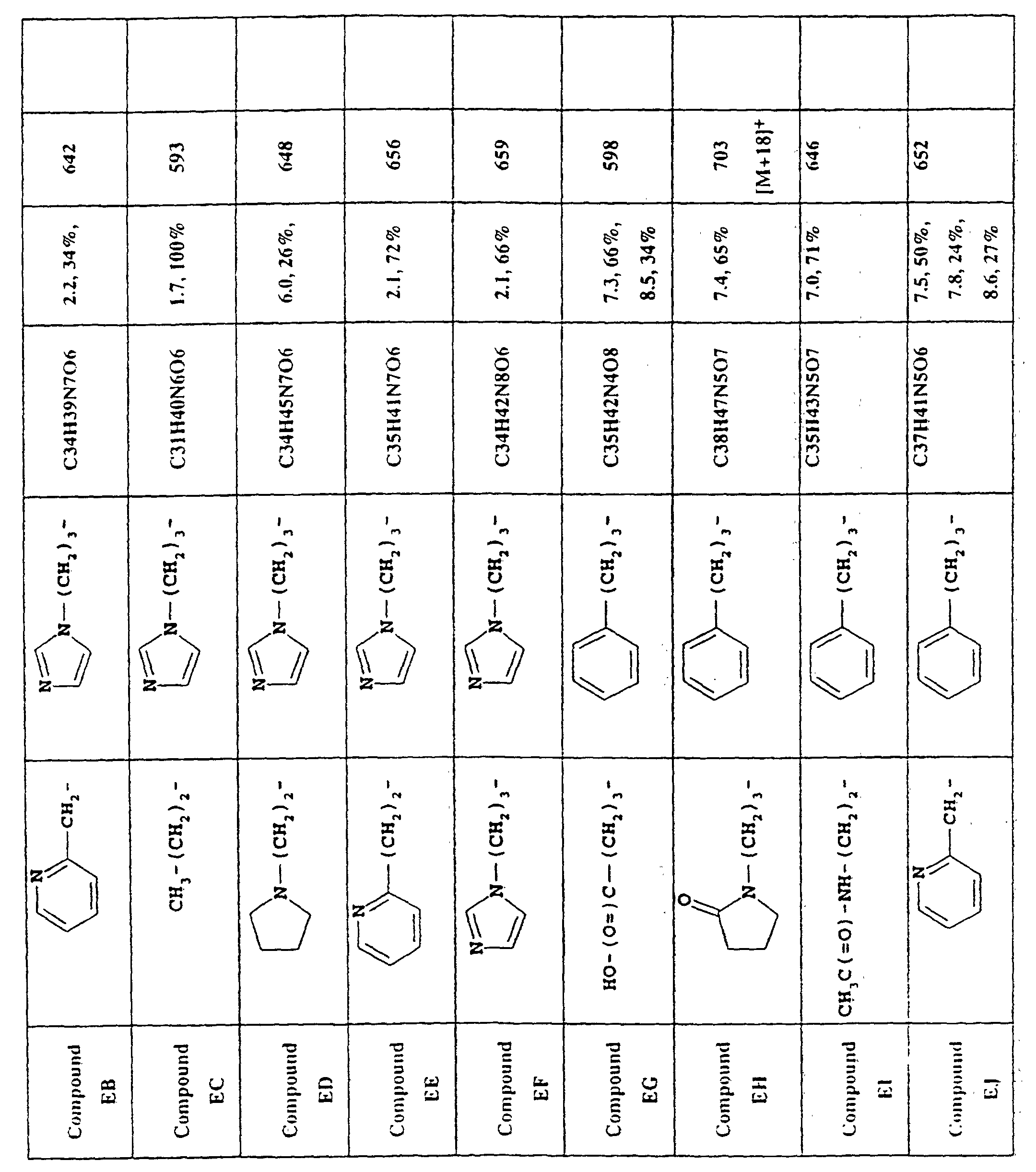

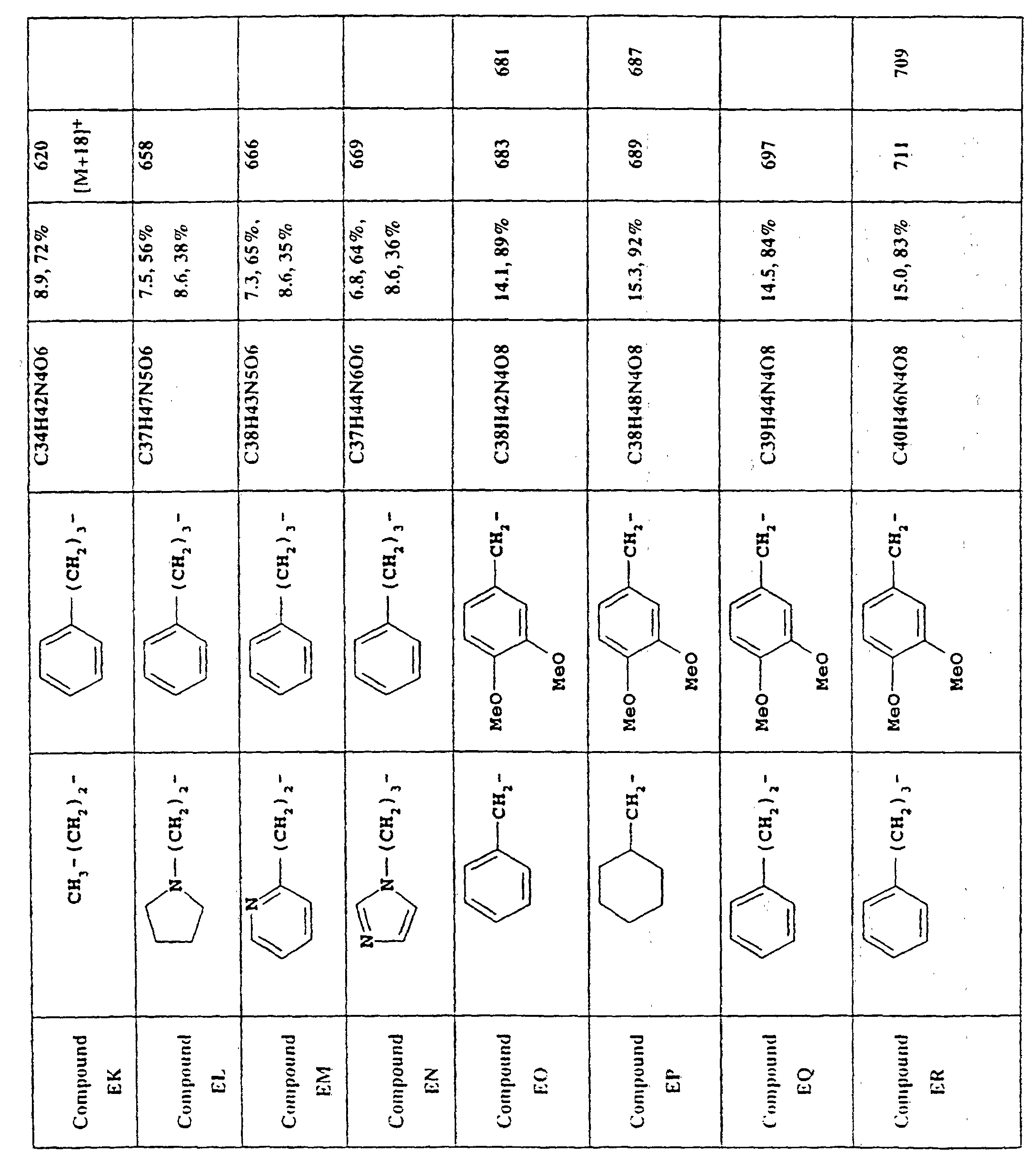

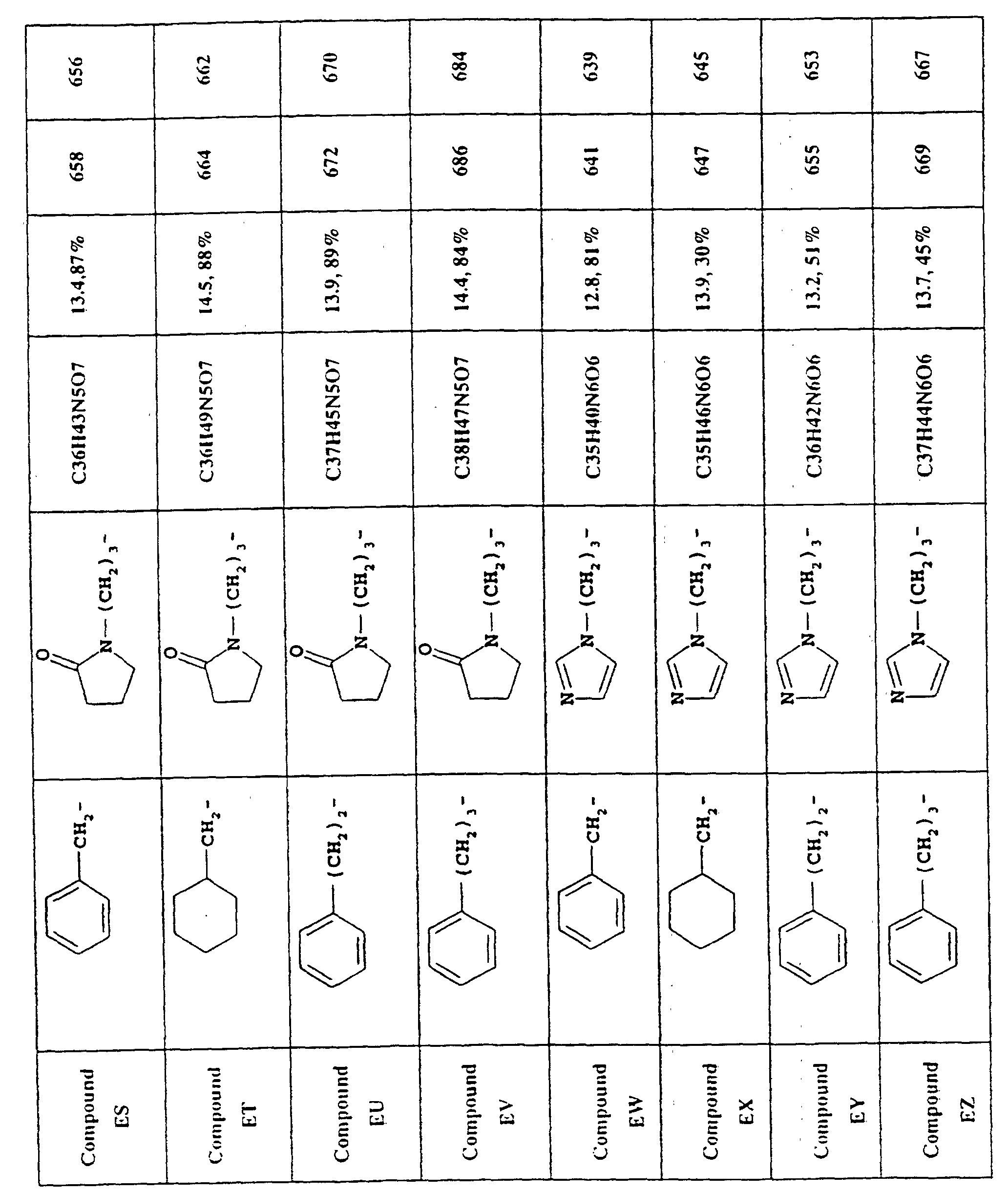

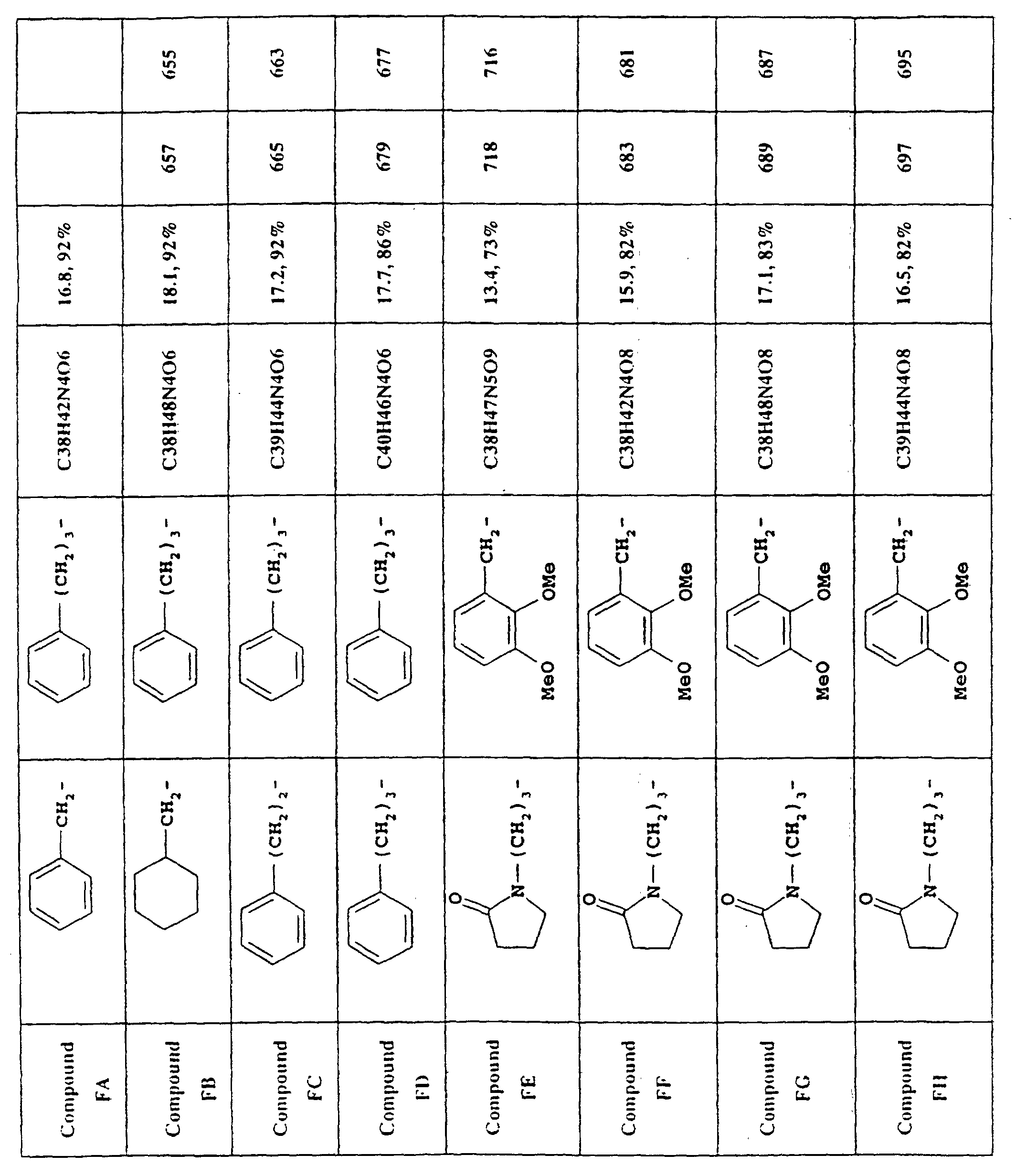

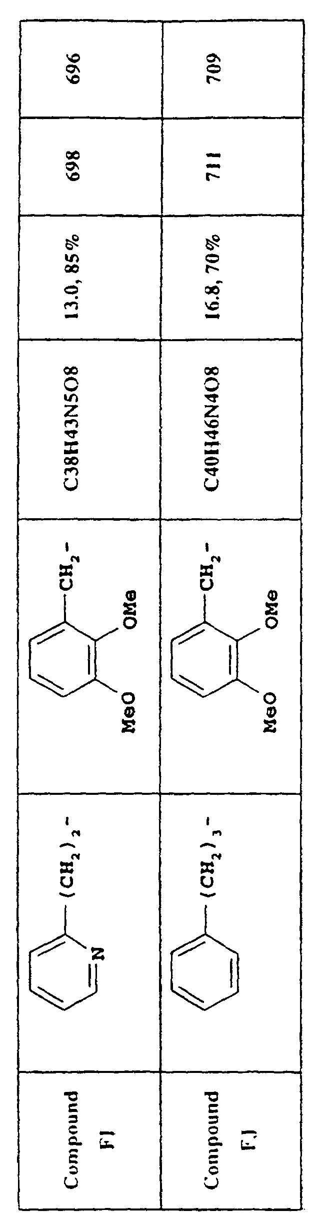

### EXAMPLE 4

### Compounds FK to KV

Step 1. Resin A from Step I Example 2 was suspended in dimethylformamide (30ml) and then treated with phenethylamine (10 eqivalents). After standing at room temperature overnight the mixture was filtered to give resin G which was washed with (i) dimethylformamide, (ii) tetrahydrofuran, (iii) dichloromethane and then dried in a desiccator under high vacuum for 2 hours.

By proceeding in a similar manner but replacing phenethylamine by allylamine, isobutylamine, (cyclohexyl)methylamine, 3-(2-oxo-1-pyrrolvdinyl)prop-1-ytamine, 4-phenyl-1-butylamine, piperonylamine, 3-(1-imidazolyl)prop-1-ylamine, 3-(2-methyl-1-piperdinyl)prop-1-ylamine, 2-(2-pyridinyl)ethylamine, (2-acetamido)ethylamine or 2-methoxybenzylamine there were prepared resins H to R.

A library of 144 compounds were prepared from resins H to R, using an ACT496 robot (96 well plate format), in the following manner.

Step 2. The appropriate resin (40mg, resins H to S prepared as described above) was placed in each well and treated with dimethylformamide (1.2ml) for 5 minutes and then drained. Dimethylformamide (0.35 ml) was added to each well. the system was warmed to 30°C, and each well was treated with (i) a solution of diisopropytethyiamine in dimethylformamide (0.5ml, 0.66M), (ii) a solution of N-(9-fluorenylmethoxycarbonyl)glycine in dimethylformamide (0.375ml, 0.294M) and (iii) a solution of [O-(7-azabenzotriazol-1-yl)-1,1,3,3,-tetramethyluronium hexafluorophosphate] in dimethylformamide (0.375ml, 0.294M). After standing at 30°C for 2 hours with mixing the wells were drained and the resins in each well were then washed five times with dimethylformamide (1.2ml) with mixing for 5 minutes. Further batches of resins H to S were similarly modified by replacing N-(9-fluorenylmethoxycarbonyl)glycine with N-(9-fluorenylmethoxycarbonyl)-3-aminopropionic acid, N-(9-fluorenylmethoxycarbonyl)-4-aminobutyric acid, N-(9-fluorenylmethoxycarbonyl)sarcosine or N-(9-fluorenylmethoxycarbonyl)-4-N-methylaminobutyric acid.

Step 3. The resins from Step 2 in each well were then treated with 20% piperidine in dimethylformamide (1.2ml) with mixing for 5 minutes, the wells were drained and the procedure repeated. The resins in each well were then washed (with mixing for 5 minutes) seven times with dimethylformamide (1.2ml).

Step 4. Dimethylformamide (0.35 ml) was added to the resin in each well followed by (i) a solution of diisopropylethylamine in dimethylformamide (0.5ml, 0.44M), (ii) a solution of 4-(phenytureido)phenylacteic acid in dimethylformamide (0.375ml, 0.196M), (iii) a solution of [O-(7-azabenzotriazol-1-yl)-1,1,3,3,-tetramethyluronium hexafluorophosphate] in dimethylformamide (0.375ml, 0.196M). After mixing for 2 hours the wells drained and each well was washed (with mixing for 5 minutes) (i) three times with dimethylformamide (1.2ml), (ii) five times with tetrahydrofuran (1.2ml), (iii) seven times with dichloromethane (1.2ml). Further batches of resins from Step 3 were similarly modified by replacing [4-(phenylureido)phenyl]-acetic acid with [4-(3-o-tolylureido)phenyl]-acetic acid, [4-(phenylureido)phenyl]-propionic acid, [3-(phenylureido)phenyl]-acetic acid, [3-methoxy-4-(3-o-tolyl-ureido)phenyl]-acetic acid or [3-methoxy-4-(3-o-tolyl-ureido)phenyl]-propionic acid.

Step 5. The system heating was switched off. The resins in each well were treated with a mixture of trifluoroacetic acid and dichloromethane (2ml, 1:1 v/v) for 45 minutes, the filtrate was collected and the procedure repeated once more. The combined filtrates were evaporated on a turbovap evaporator (vortexed N₂ gas) to give Compounds FK to KV depicted in tables 3 to 15. The retention times (R_{T}), and area of main peak as a percentage of the total sample, shown in tables 3 to 15 were determined under HPLC conditions using as elutant (i) mixture of 0.05% trifluoroacetic acid in acetonitrile and 0.05% trifluoroacetic acid in water (1:19, v/v) for 2 minutes (ii) a mixture of 0.05% trifluoroacetic acid in acetonitrile and 0.05% trifluoroacetic acid in water (1:19 to 19:1, v/v) gradient elution over 10 minutes, (iii) a mixture of 0.05% trifluoroacetic acid in acetonitrile and 0.05% trifluoroacetic acid in water (19:1, v/v) for 2 minutes, (iv) a mixture of 0.05% trifluoroacetic acid in acetonitrile and 0.05% trifluoroacetic acid in water (19:1 to 1:19, v/v) gradient elution over 2 minutes.

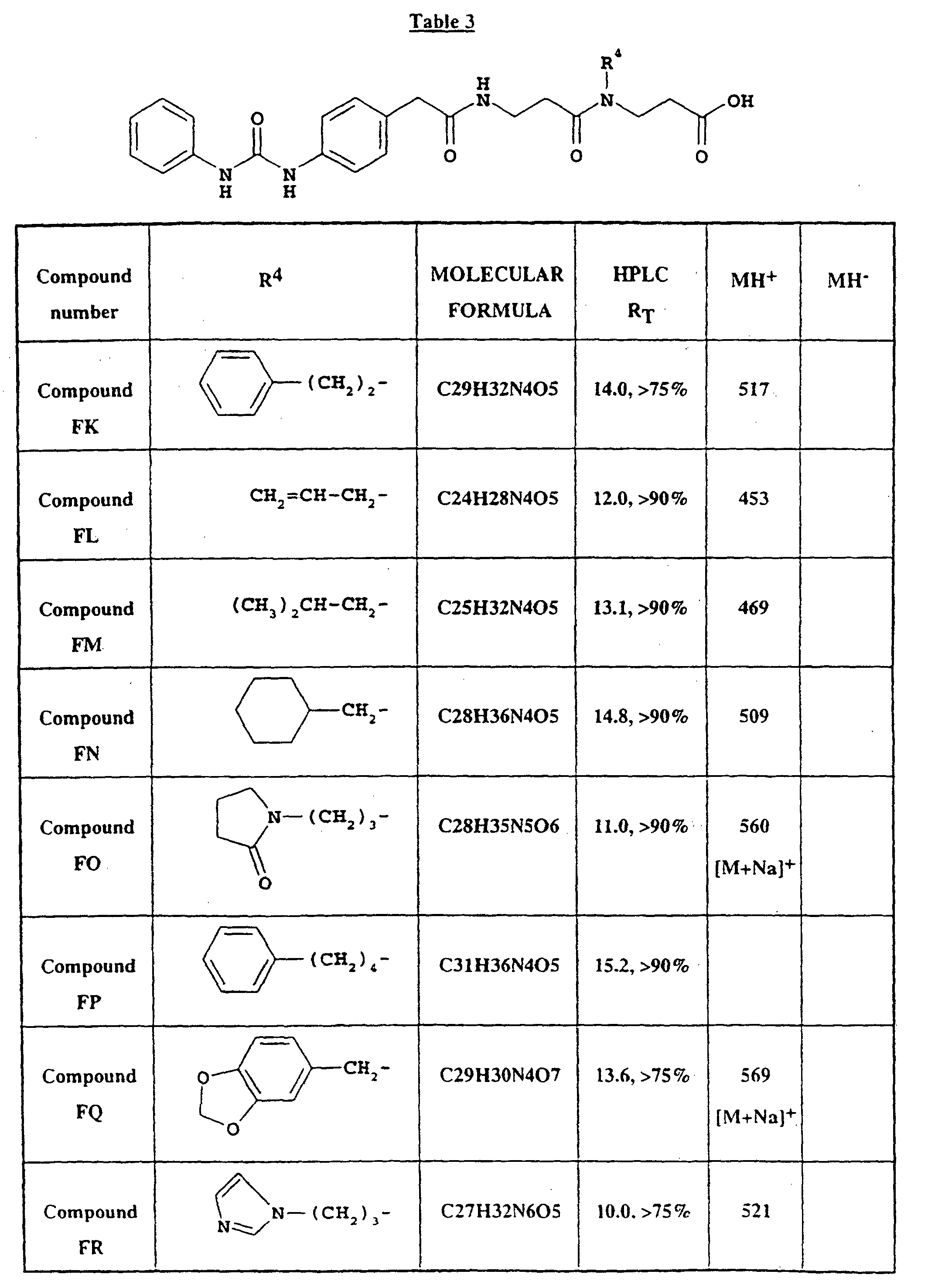

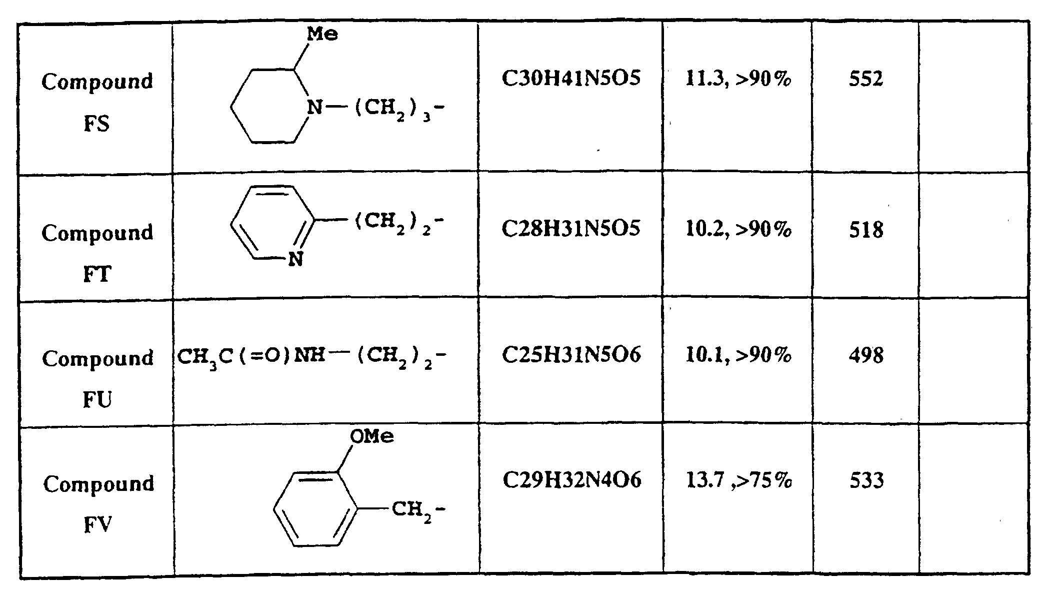

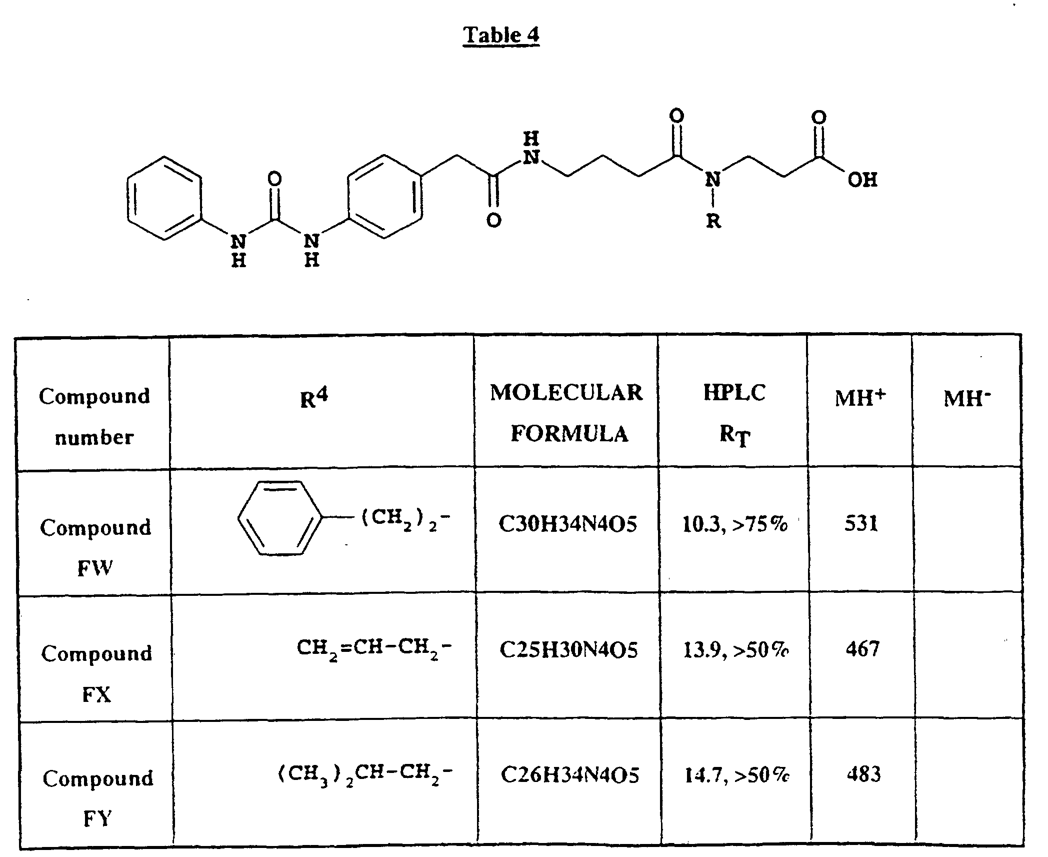

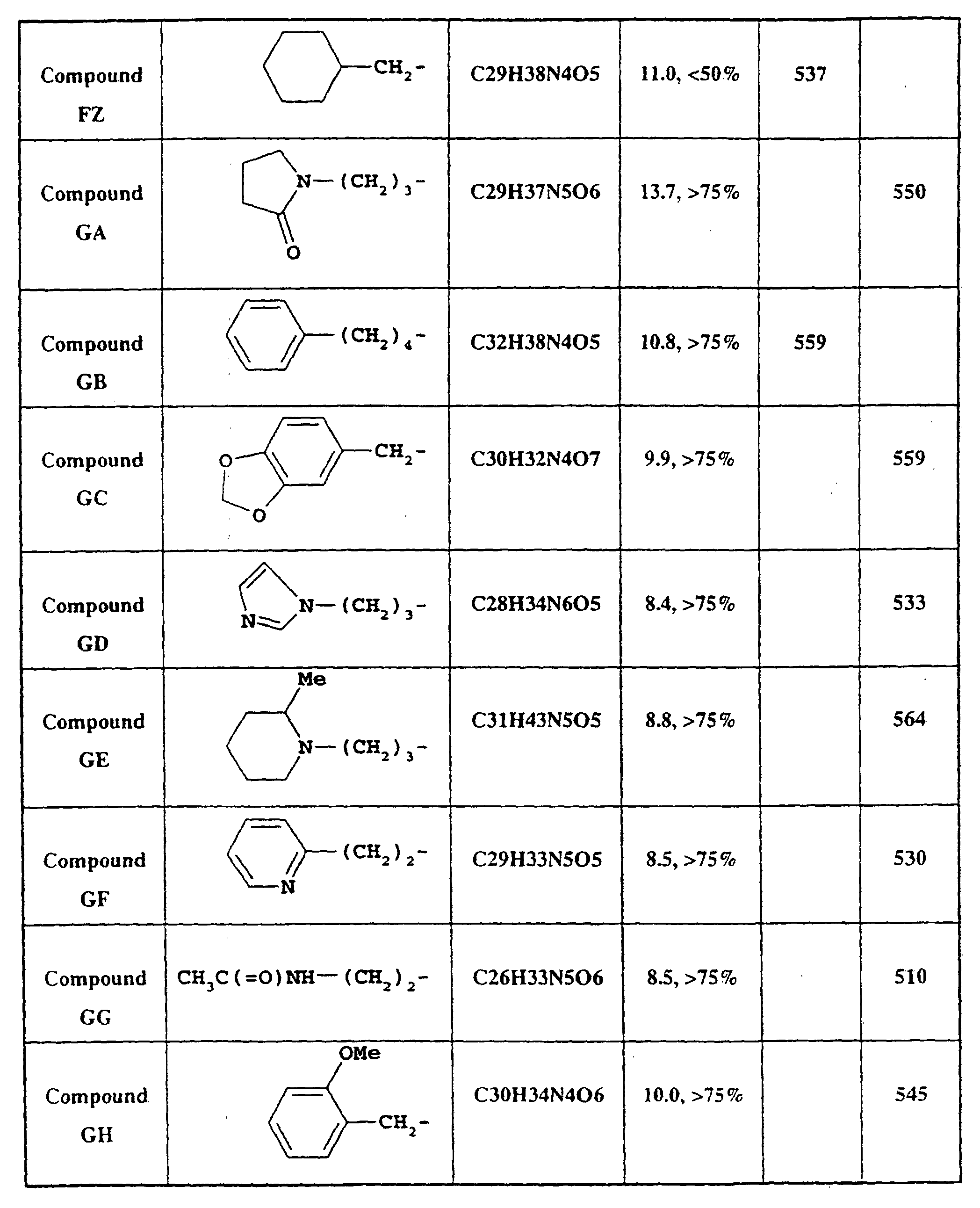

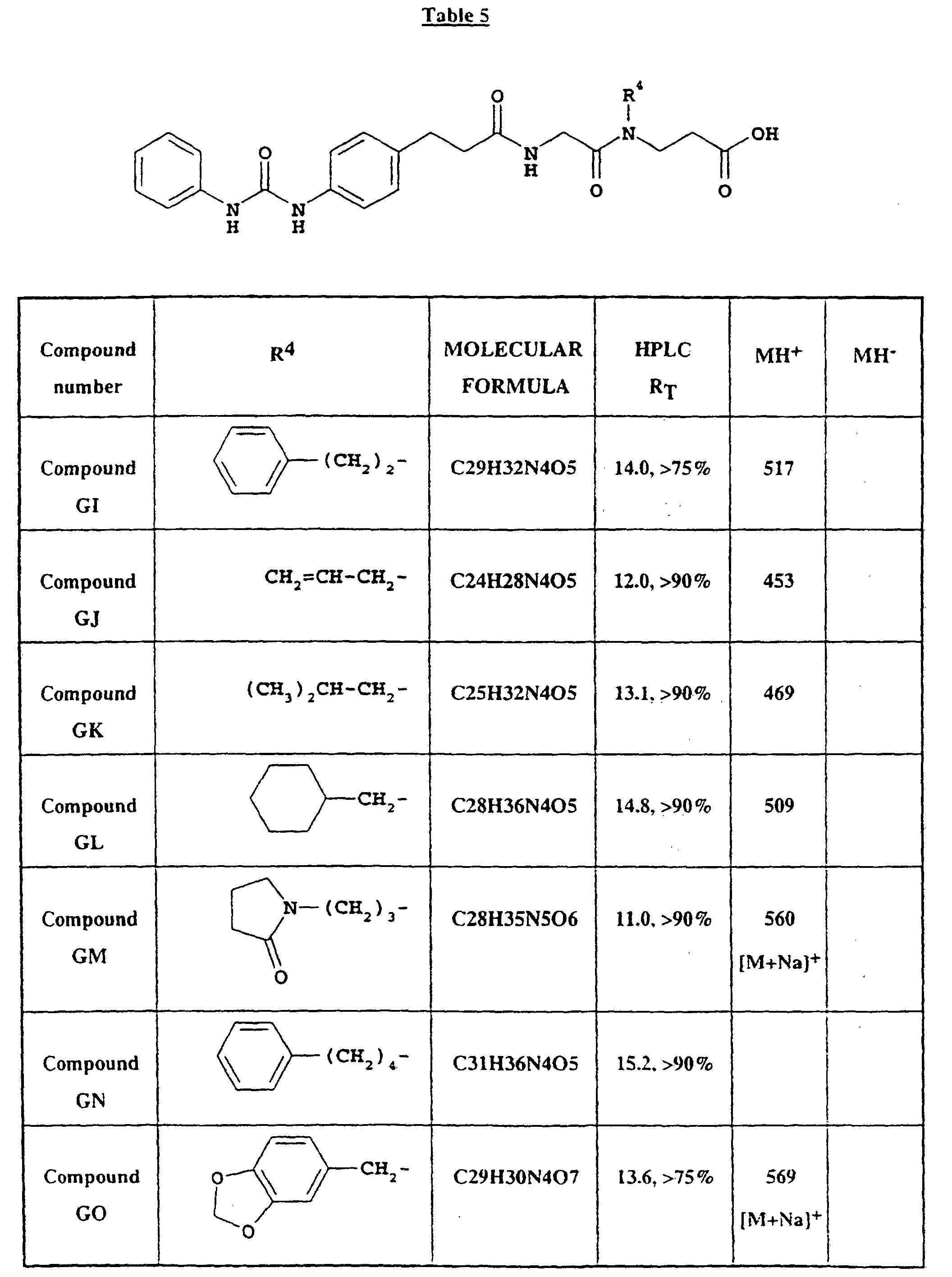

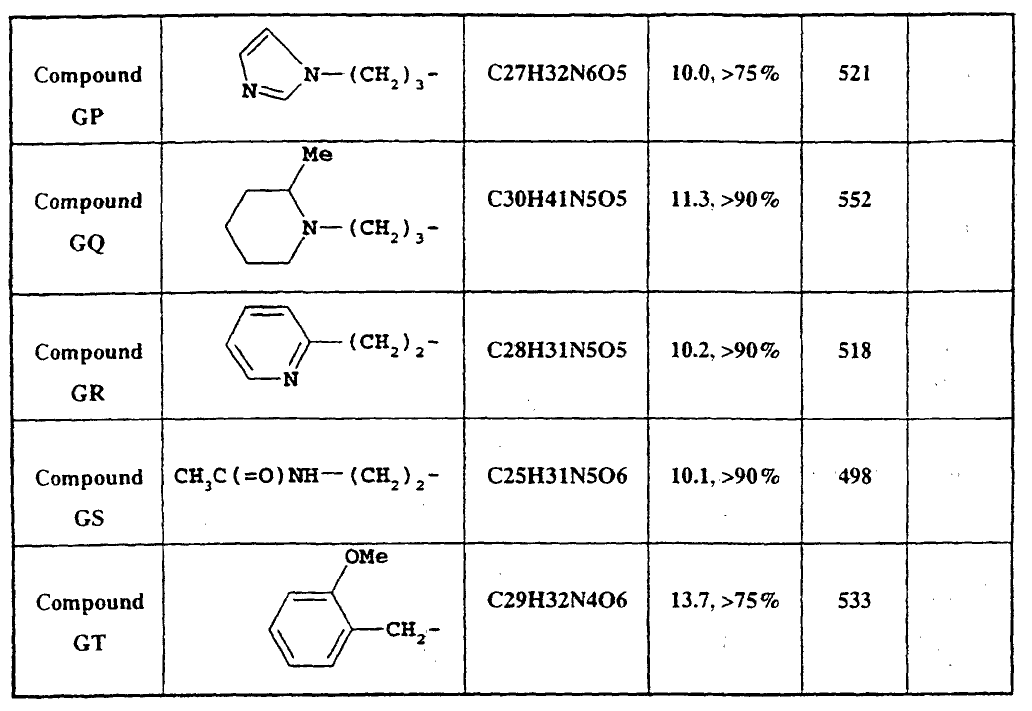

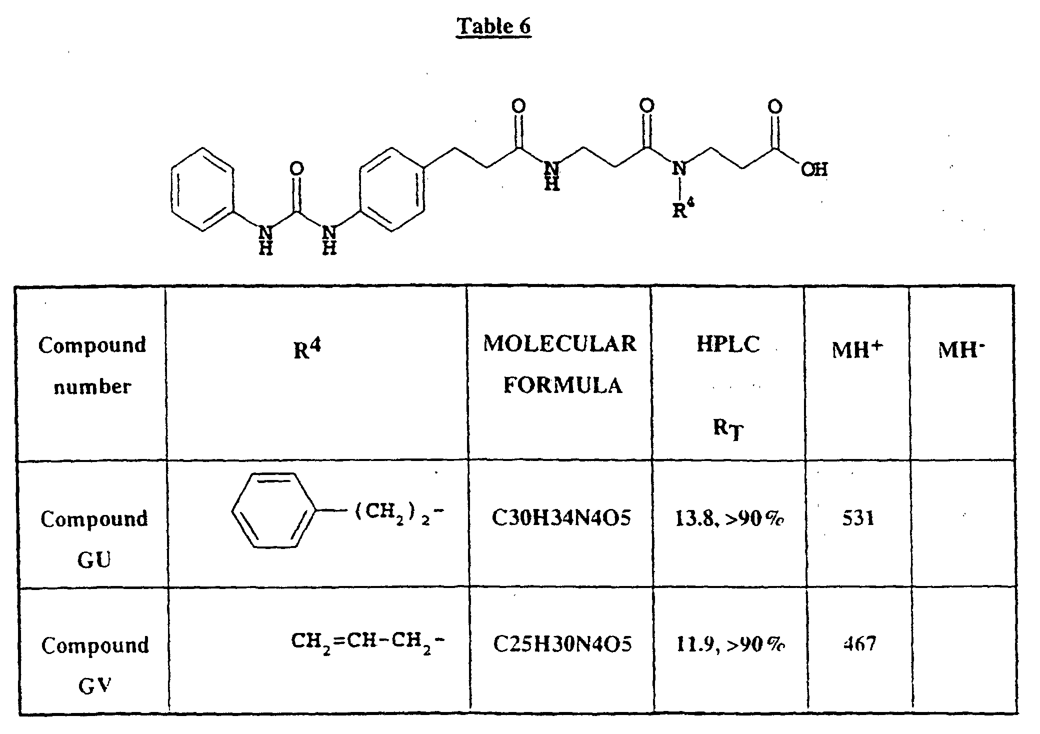

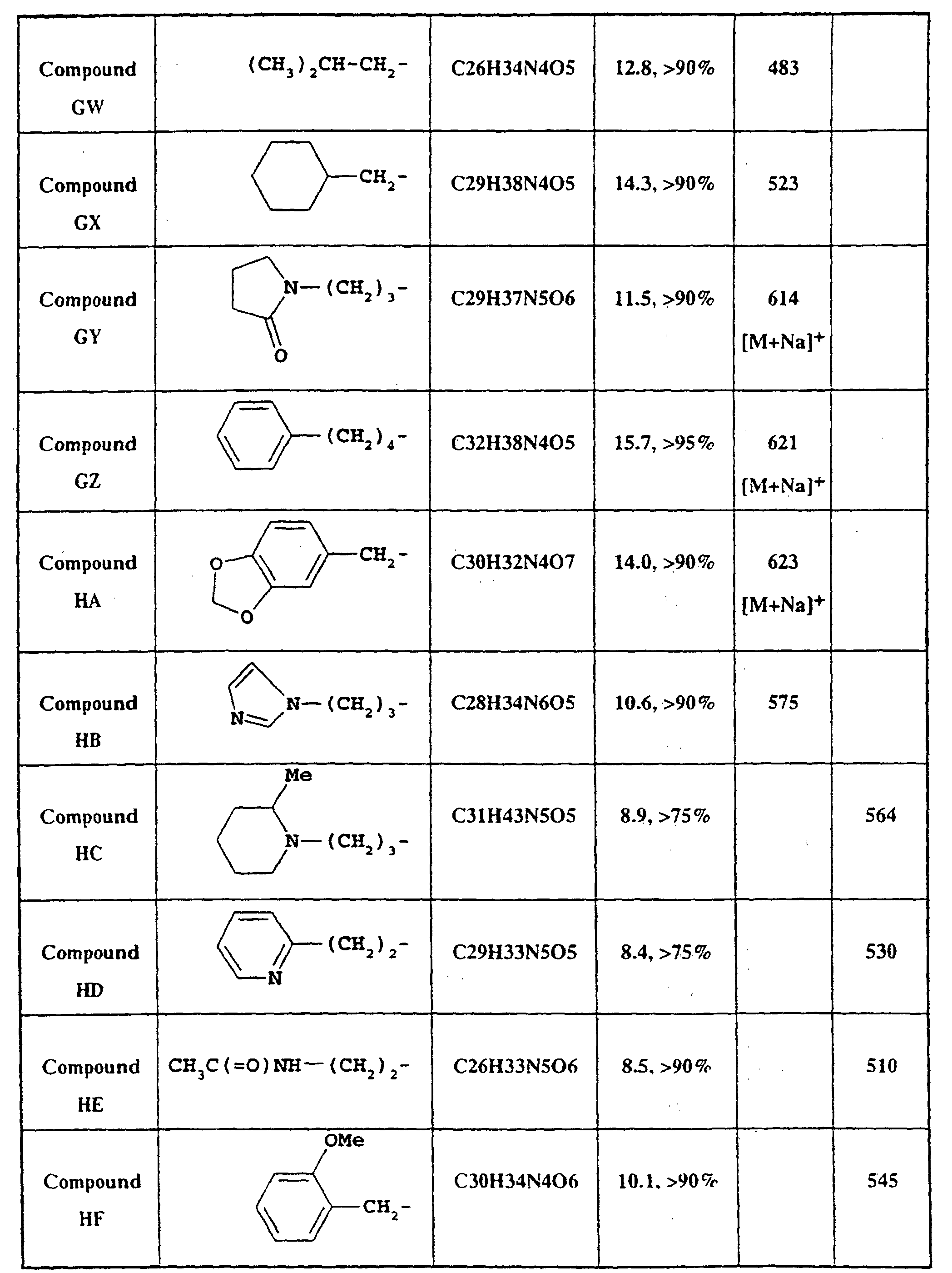

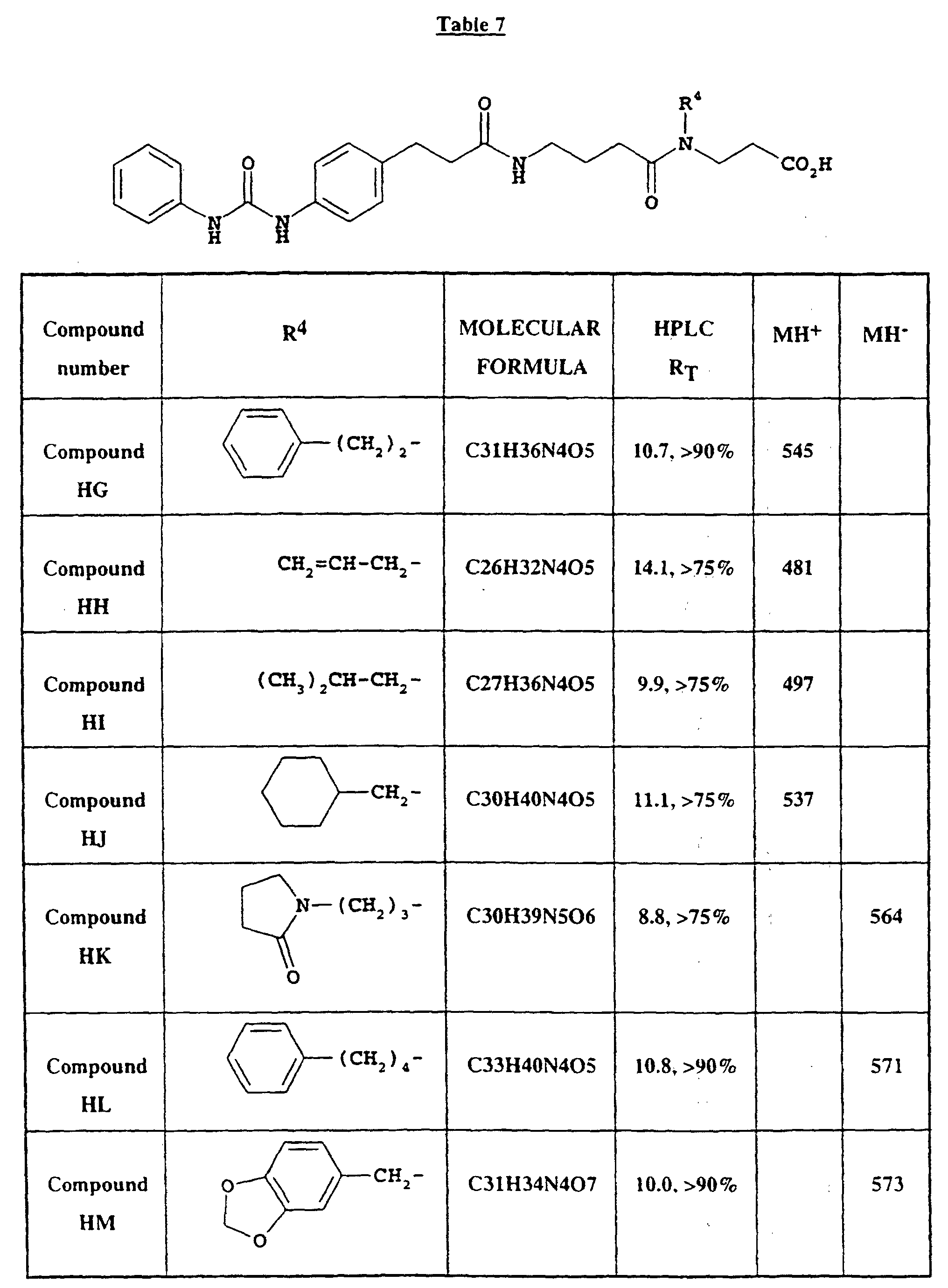

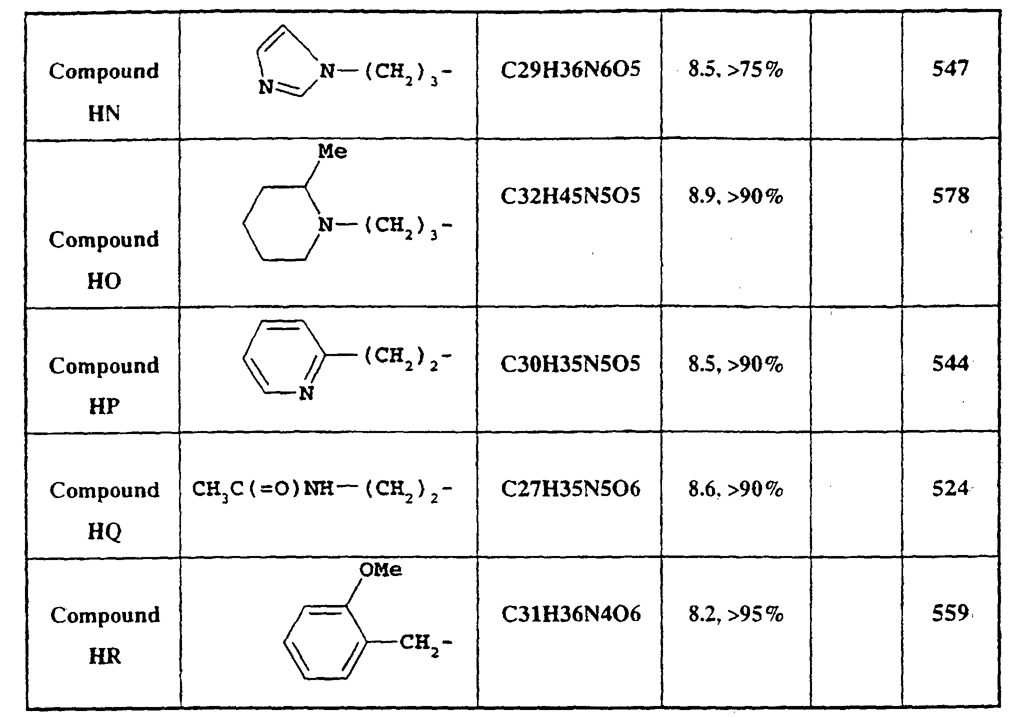

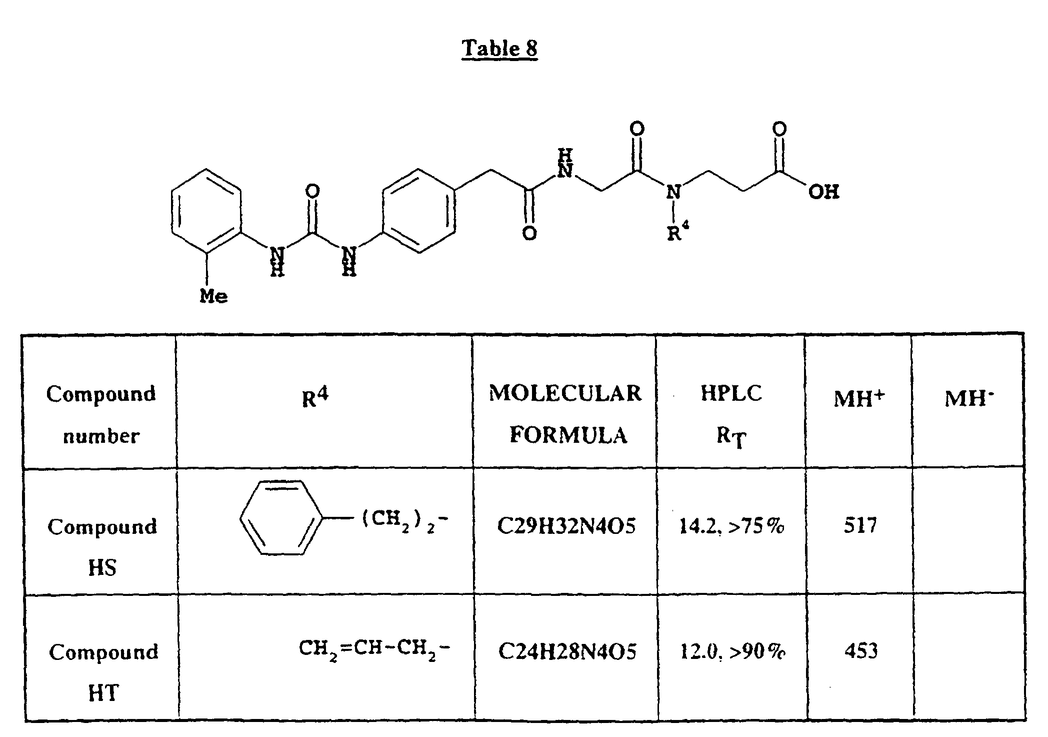

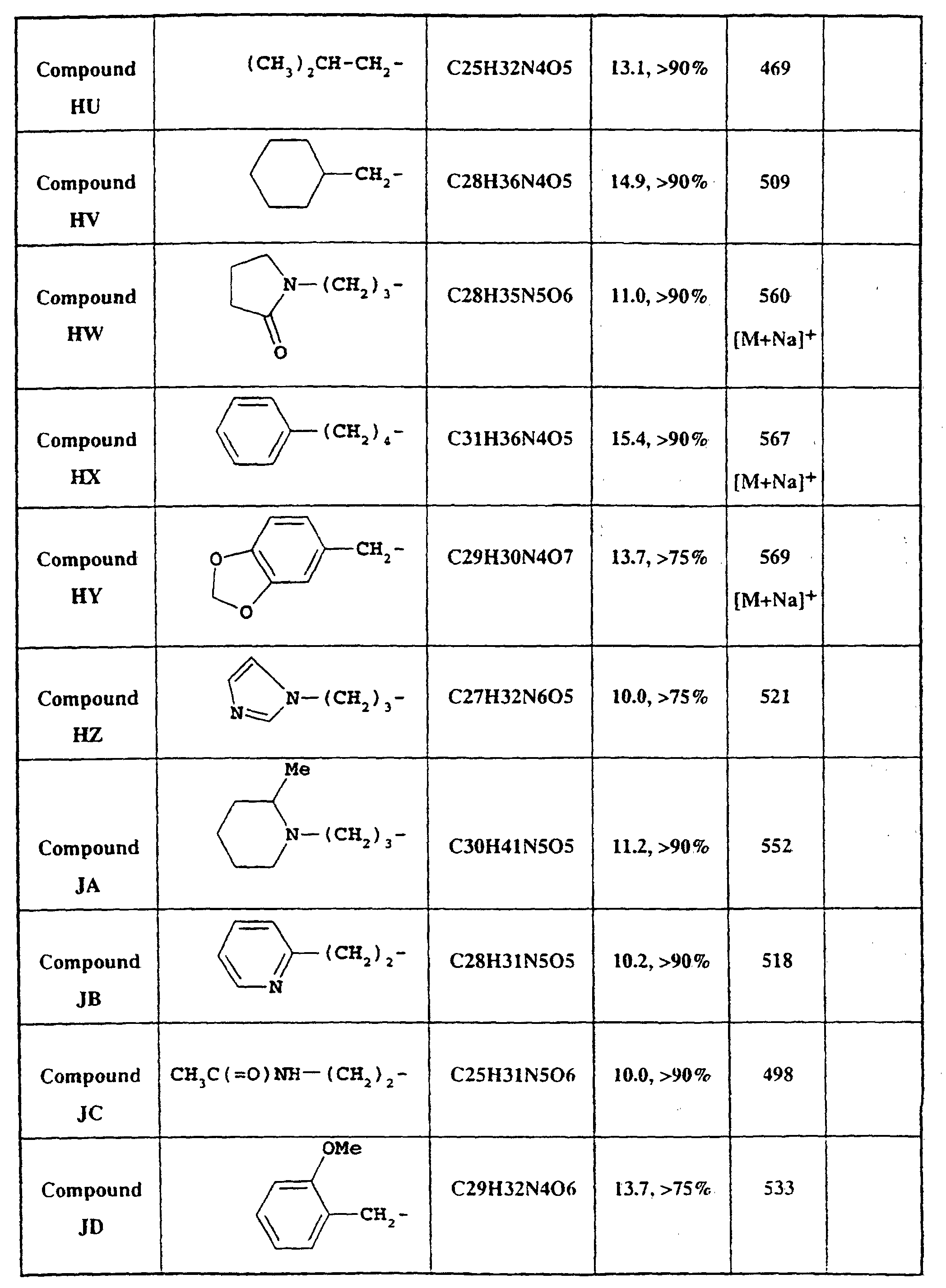

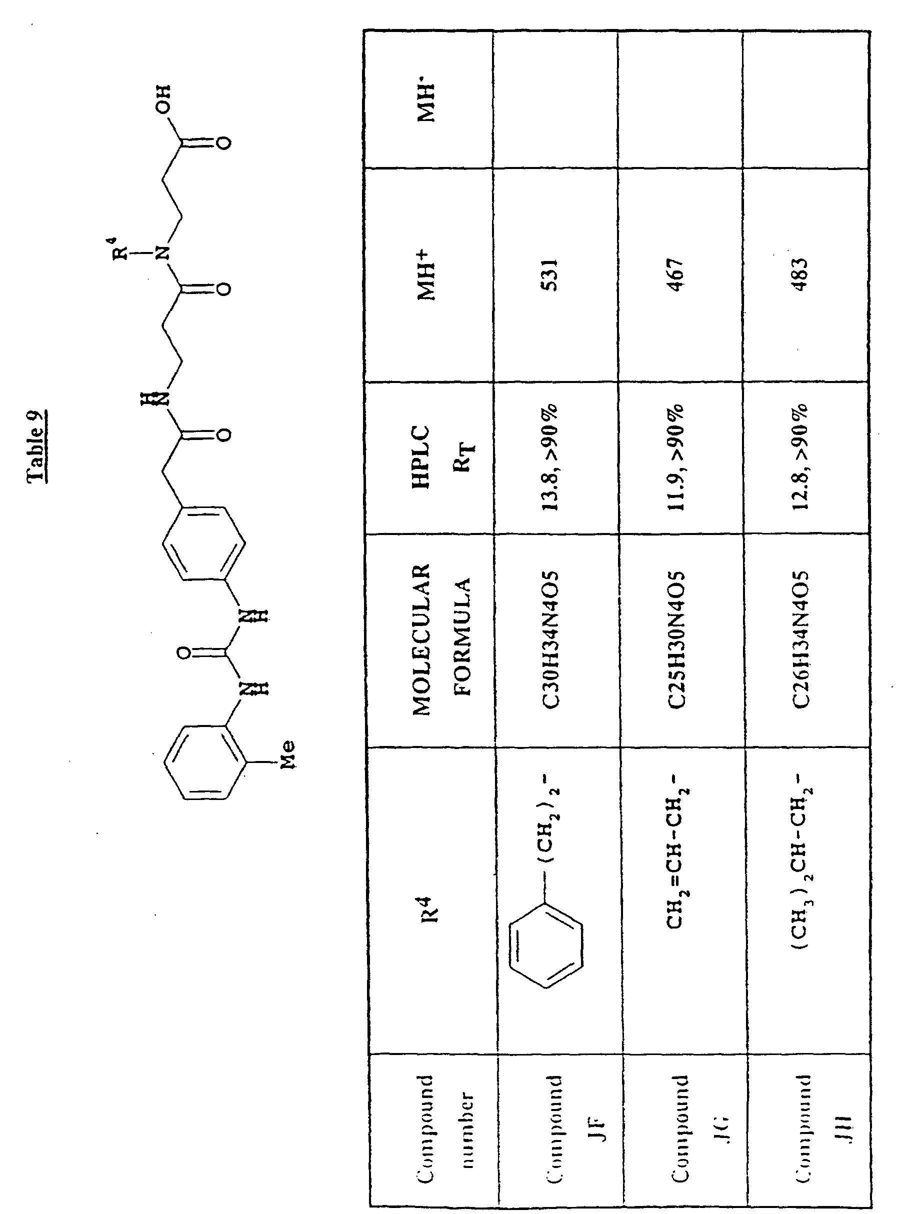

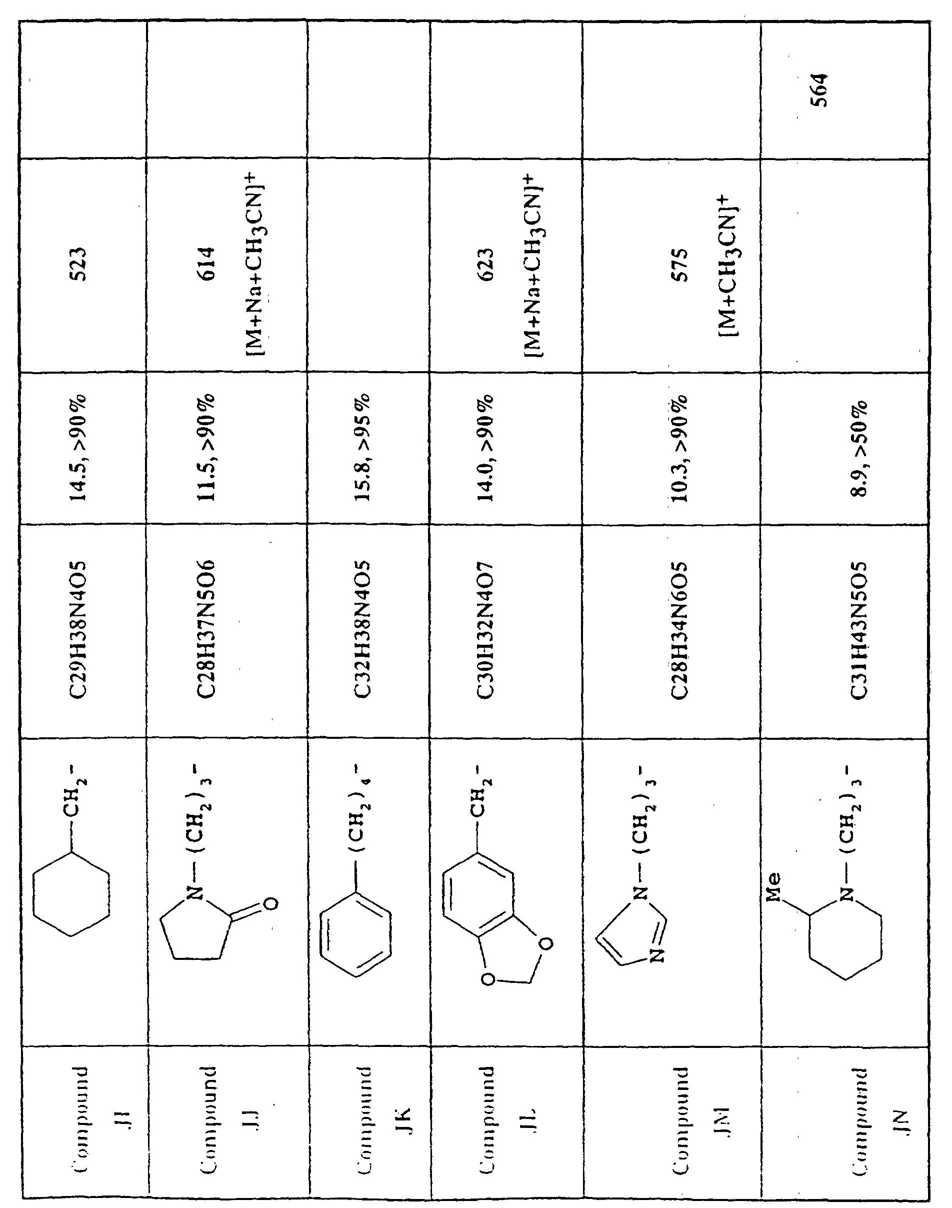

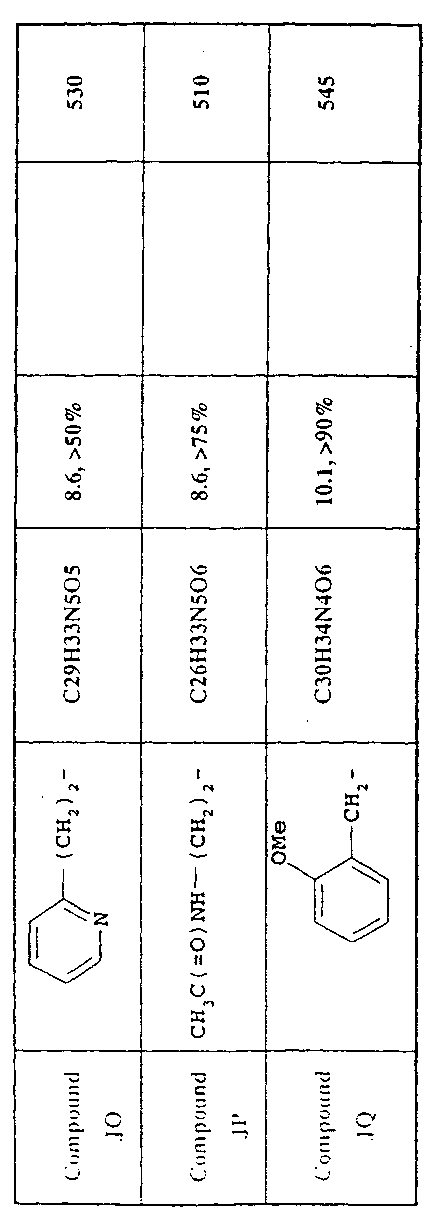

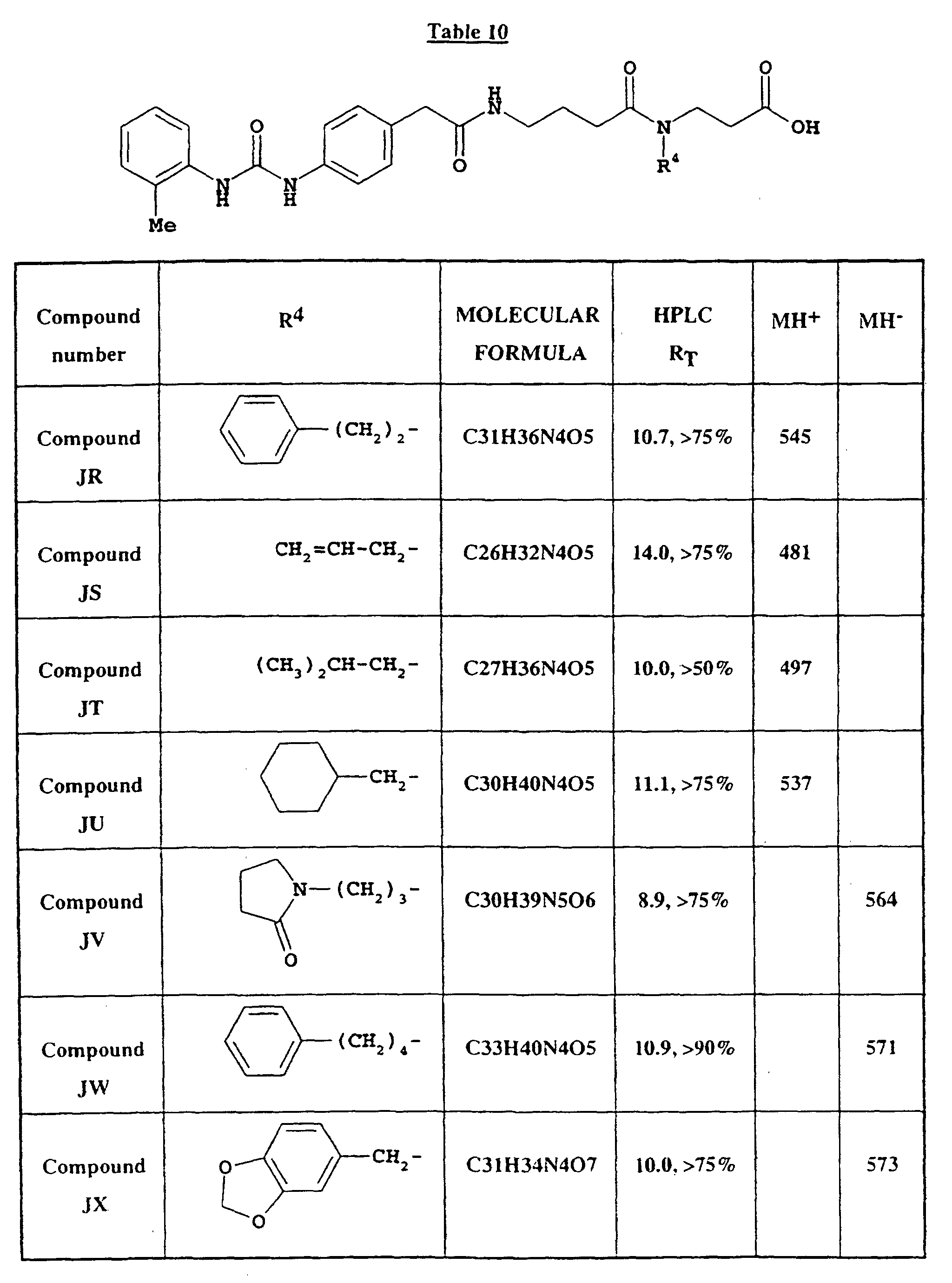

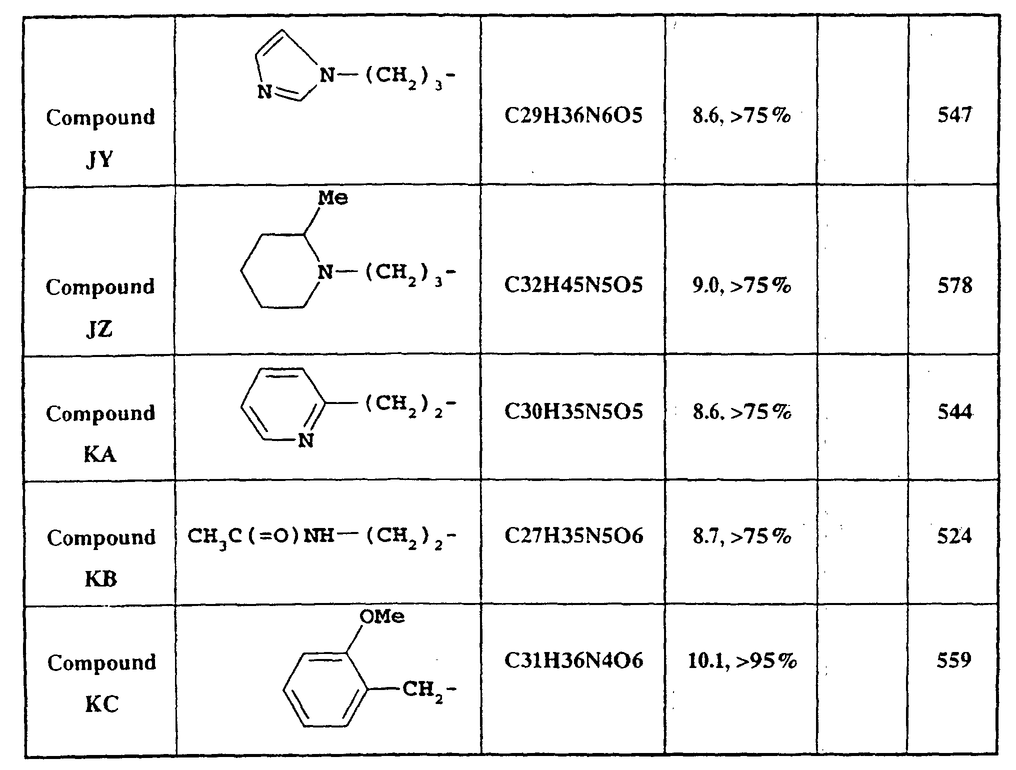

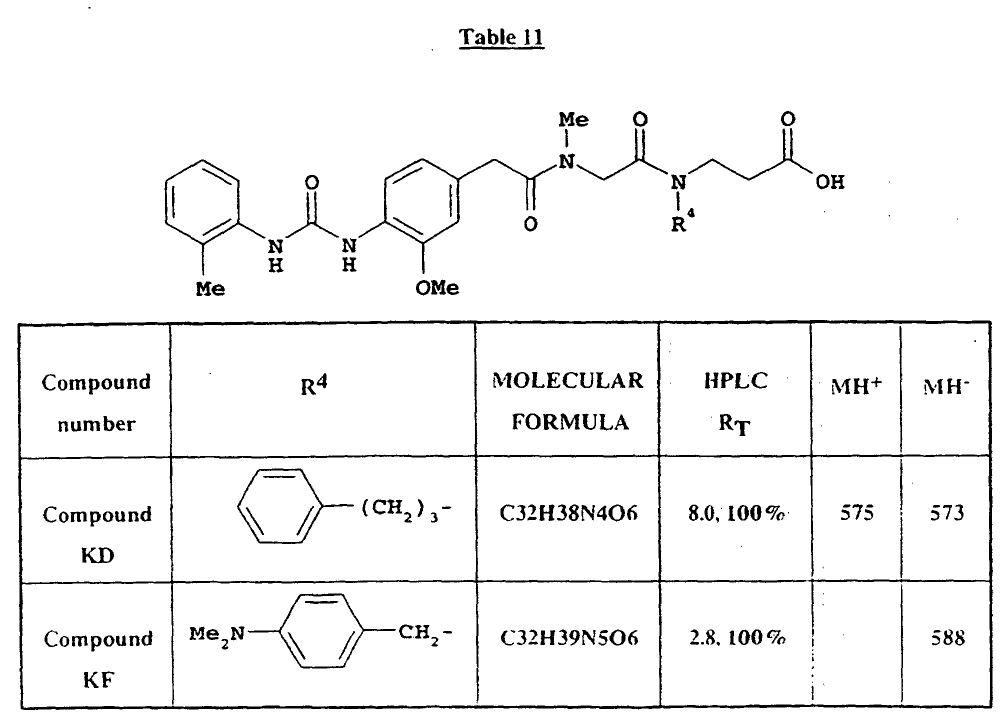

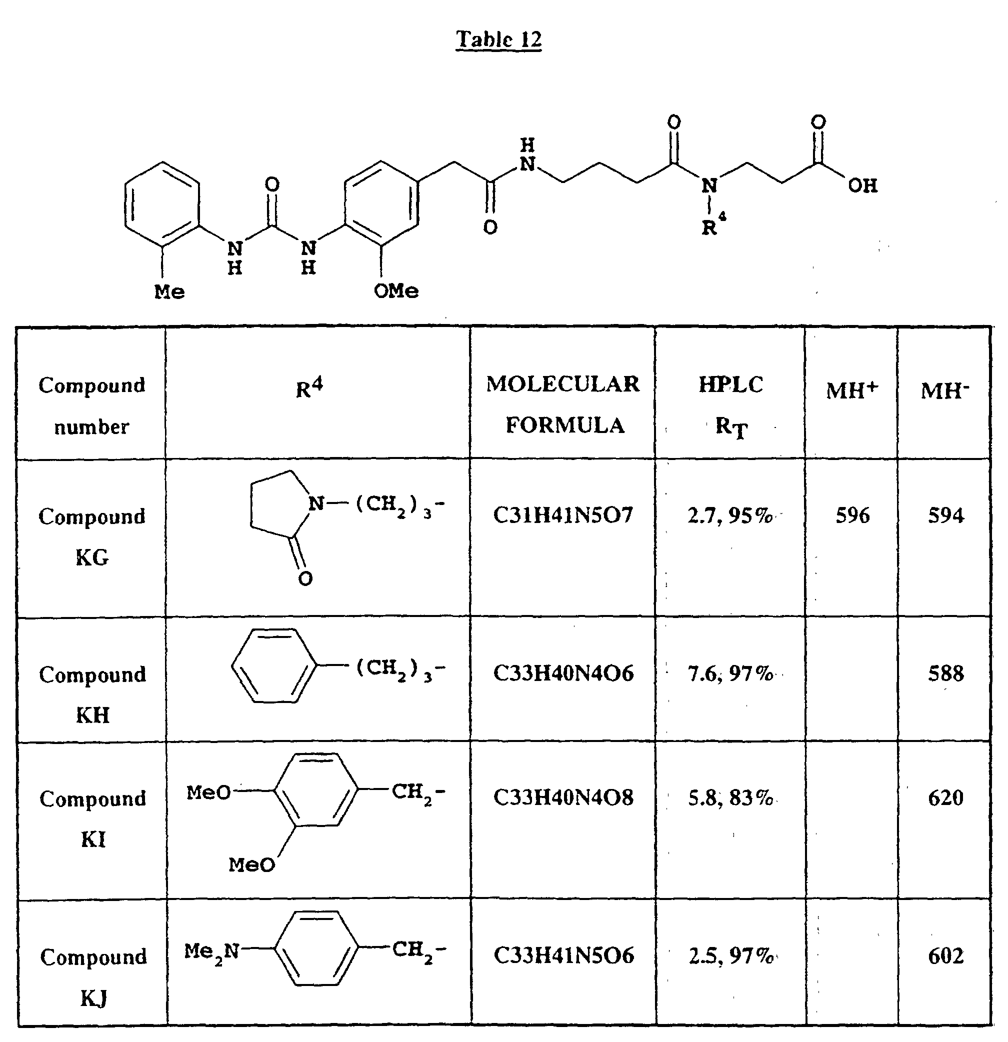

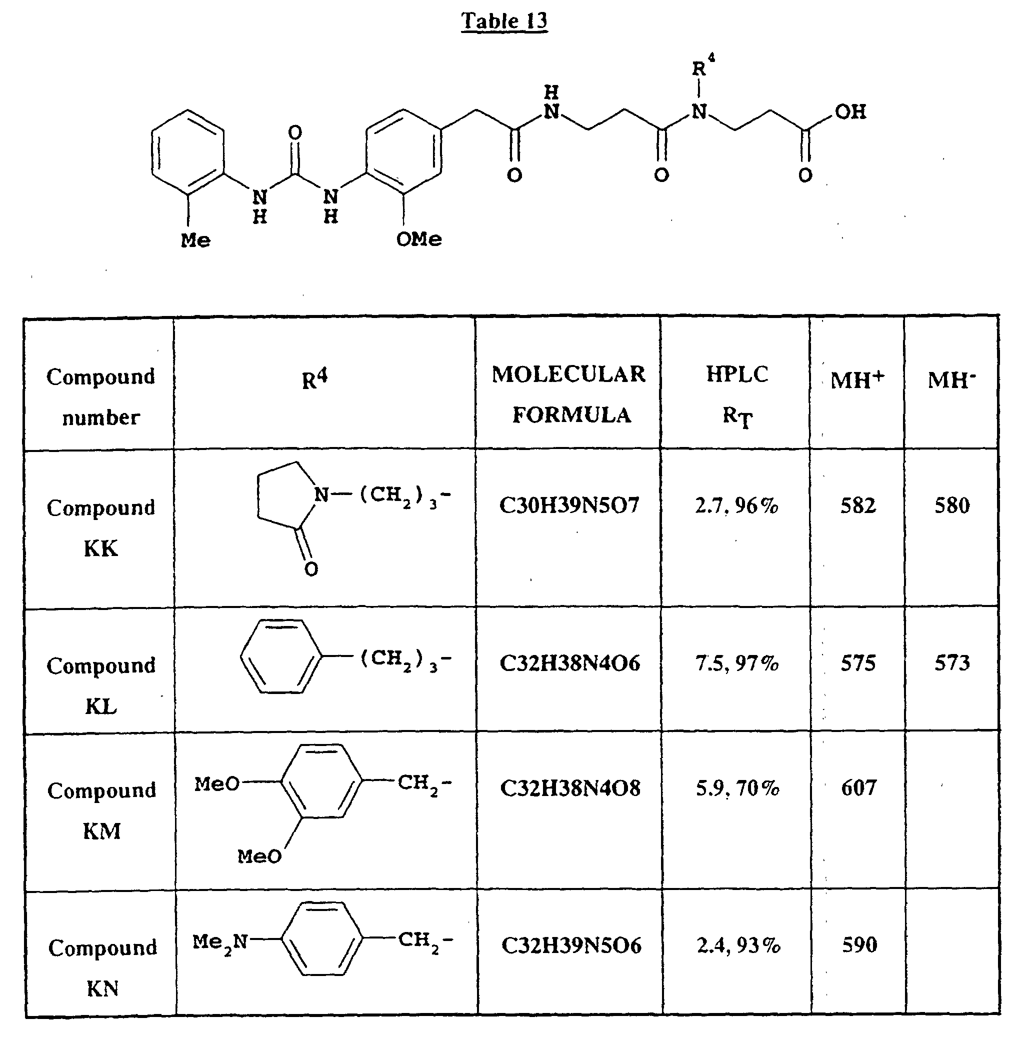

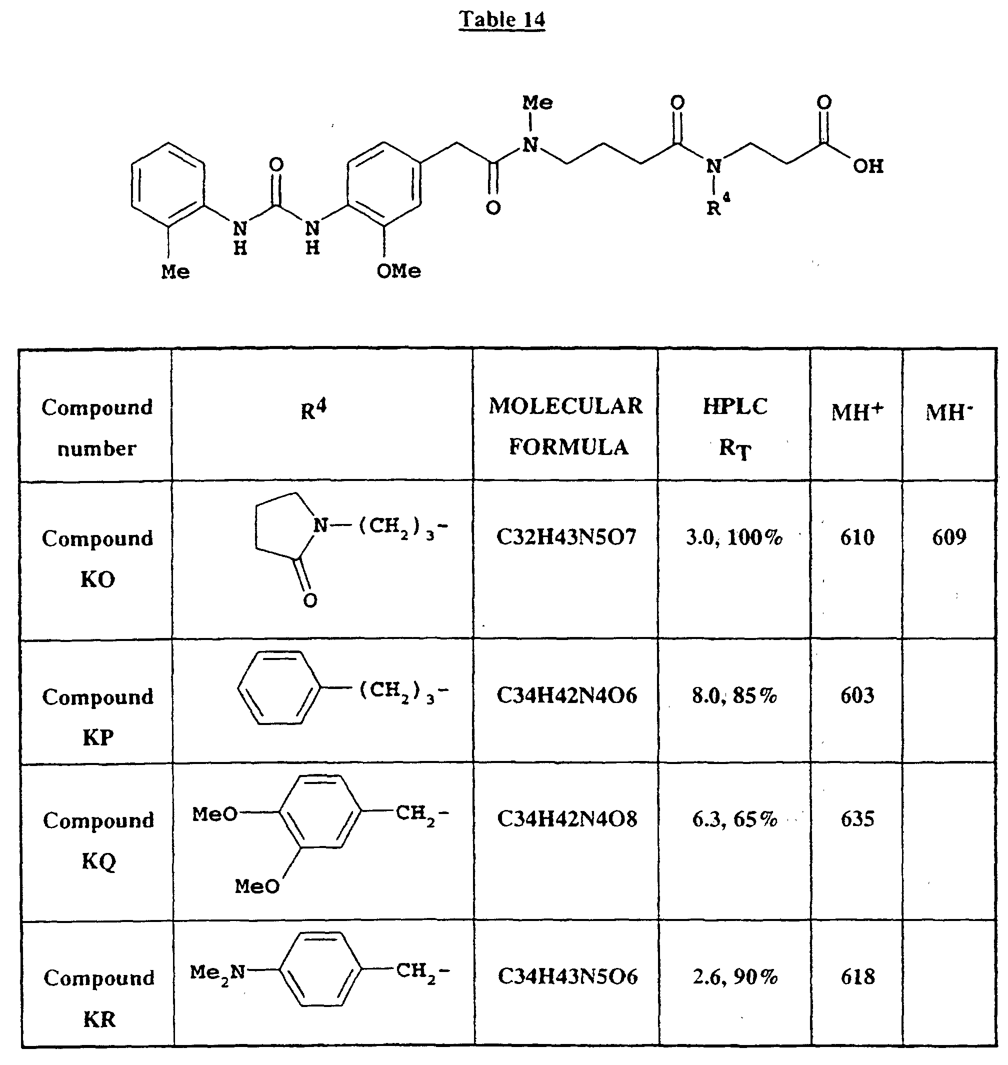

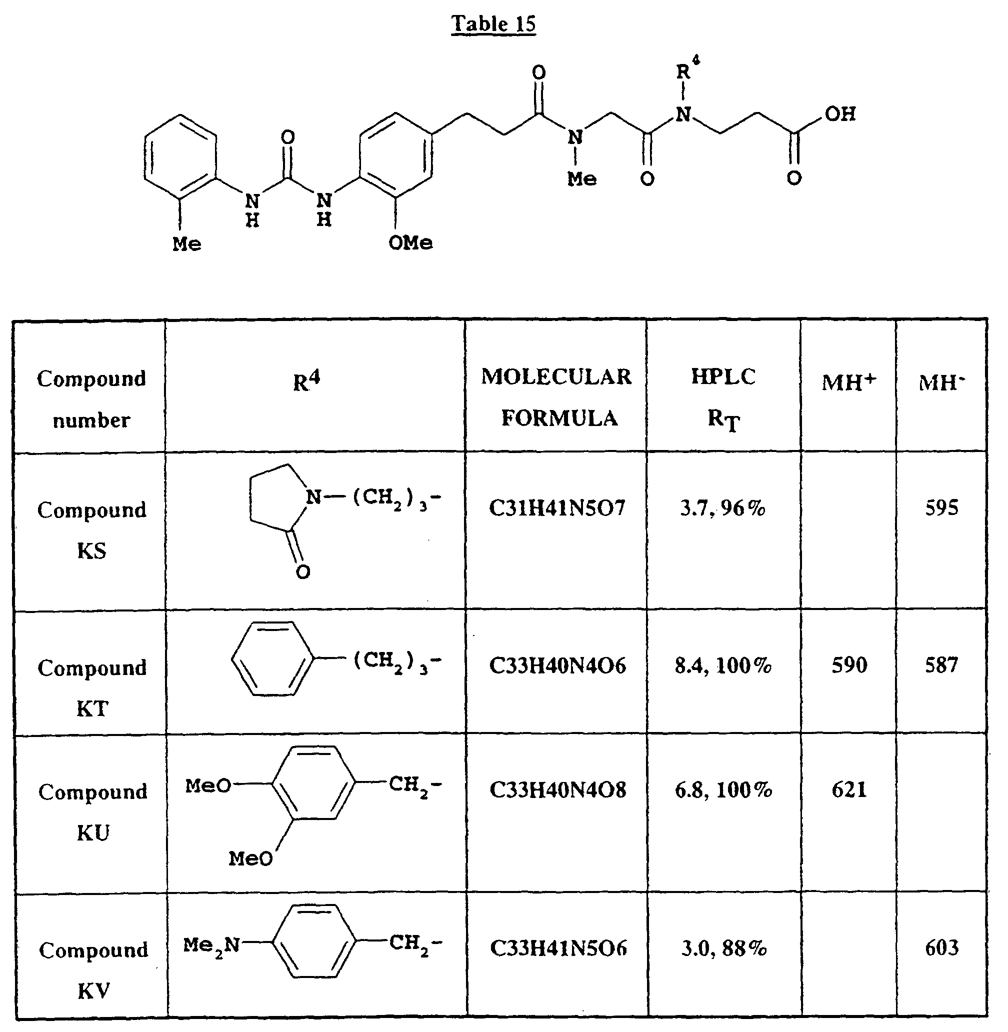

### EXAMPLE 5

### Compounds C, KW and KX

A solution of 3-{(3,4-dimethoxy-benzyl)-[({[3-methoxy-4-(3-o-tolylureido)phenyl]-acetyl}-N-methylamino)-acetyl]-amino}-propionic acid ethyl ester [2.0g. Reference Example 4(a)] in ethanol (50ml) was treated with sodium hydroxide (3.5ml, 1M). After stirring at room temperature for 3 hours the mixture was concentrated to dryness. The residue was dissolved in water (12ml) and the pH of the solution was adjusted to1.0 by addition of concentrated hydrochloric acid (0.25ml) and then extracted three times with dichloromethane. The resultant solid was collected and recrystallised twice from 20% aqueous isopropanol to give 3-{(3,4-dimethoxy-benzyl)-[({[3-methoxy-4-(3-o-tolylureido)phenyl]-acetyl}-N-methylamino)-acetyl]-amino}-propionic acid (Compound C) as a white solid (0.25g,), m.p. 183-187°C. [Elemental analysis:- C,63.2; H.6.3; N,9.2% Calculated for C₃₂H₃₈N₄O₈:- C,63.4; H,6.3; N,9.2 %].
MS: 605 [MH]⁻. HPLC: R_{T}=11.92 minutes (gradient elution using a mixture of acetonitrile and water 1:4 to 4:1).

(b) By proceeding in a manner similar to Example 5(a) but using 3-{[({[3-methoxy-4-(3-o-tolylureido)phenyl]-acetyl}-N-methylamino)-acetyl]-(3-carboxy-prop-1-yl)-amino}-propionic acid ethyl ester [Reference Example 4(b)] there was prepared 3-{[({[3-methoxy-4-(3-o-tolylureido)phenyl]-acetyl}-N-methylamino)-acetyl]-(3-carboxy-prop-1-yl)-amino}-propionic acid (Compound KW) as a white foam. [Elemental analysis:- C,57.2; H,6.5; N,9.9%. Calculated for C₂₇H₃₄N₄O₈ •H₂O:- C,57.2; H,6.3; N,9.8%]. MS: 543 [MH]⁺.

(c) By proceeding in a manner similar to Example 5(a) but 3-{[({[3-methoxy-4-(3-o-tolylureido)phenyl]-acetyl}-N-methylamino)-acetyl]-[2-(2-oxo-pyrrolidin-1-yl)-ethyl]-amino}-propionic acid ethyl ester [Reference Example 4(c)] there was prepared 3-{[({[3-methoxy-4-(3-o-tolylureido)phenyl]-acetyl}-N-methylamino)-acetyl]-[2-(2-oxo-pyrrolidin-1-yl)-ethyl]-amino}-propionic acid (Compound KX) as a yellow foam. [Elemental analysis:- C,59.5; H,6.3; N,11.9%. Calculated for C₂₉H₃₇N₅O₇•H₂O:- C,59.5; H,6.7; N,11.95%]. MS: 568 [MH]⁺.

### EXAMPLE 6

### Compounds BD, D, LA, LB, LC, AO, AC and LD to LH

(a) Step 1. A solution of ({[3-methoxy-4-(3-o-tolylureido)phenyl]-acetyl}-amino)-acetic acid [50g, Reference Example 3] and 3-{3,4-dimethoxy-benzylamino)-propionic acid ethyl ester [36g, Reference Example 2(a)] in dimethylformamide (500ml) was treated with [O-(7-azabenzotriazol-1-yl)-1,1,3,3,-tetramethyluronium hexafluorophosphate] (53.2g) and diisopropylethylamine (59ml). After stirring at ambient temperature for 3 hours the reaction mixture was evaporated to dryness and the residue was treated with water (3L). The mixture was extracted twice with ethyl acetate (1L) and then concentrated to dryness. The residue was subjected to flash chromatography on silica eluting initially with ethyl acetate and then with a mixture of ethyl acetate and methanol (9:1, v/v) to give 3-[N-(3,4-dimethoxybenzyl)-2-{2-[3-methoxy-4-(3-o-tolylureido)phenyl]acetylamino}acetamido]propionic acid ethyl ester as a yellow oil (49g). Step 2. This material was treated with methanol (IL) and sodium hydroxide (160ml, 1.0M) and the reaction mixture was heated at 40°C for 2 hours. After this time, the reaction mixture was cooled then evaporated and then treated with water (1.5L). The aqueous solution was washed twice with ethyl acetate (500ml) and then acidified to pH 1.0 by addition of concentrated hydrochloric acid. The resultant solid was collected, washed with water and dried under vacuum. This material was recrystallised from 10% aqueous methanol to give 3-[N-(3,4-dimethoxy-benzyl)-2-{2-[3-methoxy-4-(3-o-tolylureido)phenyl]acetylamino}-acetamido]propionic acid (Compound BD) as a white solid (33g), m.p. 172-17.4°C. [Elemental analysis:- C,62.4; H,6.2; N,9.5%. Calculated for C₃₁H₃₆N₄O₈:- C,62.8; H,6.1; N,9.45%].
   MS: 593 [MH]⁺.
(b) By proceeding in a manner similar to Example 6(a) but using 3-(3-imidazol-1-yl-prop-1-ylamino)-propionic acid ethyl ester [Reference Example 2(b)] to replace 3-(3,4-dimethoxybenzylamino)-propionic acid ethyl ester, there was prepared 3{(3-imidazol-1-yl-prop-1-yl)--[({[3-methoxy-4-(3-o-tolylureido)phenyl]-acetyl}-amino)-acetyl]-amino}-propionic acid (Compound BK) as a white foam. MS: 551 [MH]⁺.
(c) By proceeding in a manner similar to Example 6(a) but using 3-[3-(2-oxo-pyrrolidin-1-yl)-prop-1-ylamino]-propionic acid ethyl ester [Reference Example 2(c)] to replace 3-(3,4-dimethoxy-benzylamino)-propionic acid ethyl ester, there was prepared 3-{[({[3-methoxy-4-(3-o-tolylureido)phenyl]-acetyl}-amino)-acetyl]-[3-(2-oxo-pyrrolidin-1-yl)-prop-1-yl]-amino}-propionic acid (Compound D) as a white foam. [Elemental analysis:- C,58.9; H,6.2; N.11.5%. Calculated for C₂₉H₃₇N₅O₇ •H₂O:- C.59.5; H,6.7; N,11.95%]. MS: 586 [MH]⁺.
(d) By proceeding in a manner similar to Example 6(a) but using 3-(3-carboxy-prop-1-ylamino)-propionic acid di-ethyl ester [Reference Example 2(d)] to replace 3-(3.4-dimethoxybenzylamino)-propionic acid ethyl ester. there was prepared 3-{[({[3-methoxy-4-(3-o-tolylureido)phenyl]-acetyl}-amino)-acetyl]-(3-carboxy-prop-1-yl)-amino]-propionic acid (Compound LA) as a white solid, m.p. 179-181°C. [Elemental analysis:- C.59.0; H,6.1; N.10.55%. Calculated for C₂₆H₃₂N₄O₈:- C.59.1; H,6.1; N,10.6%]. MS: 529 [MH]⁺.
(e) By proceeding in a manner similar to Example 6(a) but using 3-[2-(2-oxo-pyrrolidin-1-yl)-ethylamino]-propionic acid ethyl ester [Reference Example 2(e)] to replace 3-(3,4-dimethoxybenzylamino)-propionic acid ethyl ester, there was prepared 3-{[({[3-methoxy-4-(3-o tolylureido)phenyl]-acetyl}-amino)-acetyl]-[2-(2-oxo-pyrrolidin-1-yl)-ethyl]-amino}-propionic acid (Compound LB) as a white foam. [Elemental analysis:- C,58.2; H,6.5; N,12.0% Calculated for C₂₈H₃₅N₅O₇•H₂O:- C,58.8; H,6.5; N,12.25%]. MS: 553 [MH]⁺.
(f) By proceeding in a manner similar to Example 6(a) but using 3-(2-carboxy-ethytamino)-propionic acid di-ethyl ester [Reference Example 2(f)] to replace 3-(3,4-dimethoxy-benzylamino)-propionic acid ethyl ester, there was prepared 3-{[({[3-methoxy-4-(3-o-tolylureido)phenyl]-acetyl}-amino)-acetyl]-(2-carboxy-ethyl)-amino}-propionic acid (Compound LC) as a white solid, m.p. 117-121°C. [Elemental analysis:- C,56.5; H,5.85; N,10.6% Calculated for C₂₅H₃₀N₄O₈•H₂O:- C,56.4; H,6.1; N,10.5%]. MS: 533 [MH]⁺.
(g) By proceeding in a manner similar to Example 6(a) but using 3-(2,3-dimethoxybenzylamino)-propionic acid ethyl ester [Reference Example 2(g)] to replace 3-(3,4-dimethoxybenzylamino)-propionic acid ethyl ester, there was prepared 3-{(2.3-dimethoxy-benzyl)-[({[3-methoxy-4-(3-o-tolylureido)phenyl]-acetyl}-amino)-acetyl]-amino}-propionic acid (Compound AO) as a white solid, m.p. 134-136°C. [Elemental analysis:- C,63.0; H,6.6; N,9.6% Calculated for C₃₁H₃₆N₄O₈:- C,62.8; H,6.1; N,9.45%]. MS: 593 [MH]⁺.
(h) By proceeding in a manner similar to Example 6(a) but using 3-(3-phenyl-prop-1-ylamino)-propionic acid ethyl ester [Reference Example 2(h)] to replace 3-(3,4-dimethoxybenzylamino)-propionic acid ethyl ester, there was prepared 3-{[({[3-methoxy-4-(3-o-tolylureido)phenyl]-acetyl}-amino)-acetyl]-(3-phenyl-prop-1-yl)-amino}-propionic acid (Compound AC) as a white solid. m.p. 149-150°C. [Elemental analysis:- C,65.65; H,6.1; N,9.7% Calculated for C₃₁H₃₆N₄O₆:- C.66.4; H,6.5; N,10.0%]. MS: 561 [MH]⁺.
(i) By proceeding in a manner similar to Example 6(a) but using 3-(phenylamino)-propionic acid ethyl ester (prepared according to the procedure described by Kano, Shinzo; Ebata, Tsutomu; Shibuya, Shiroshi. J. Chem. Soc., Perkin Trans. 1 (1980), Issue 10, 2105-11) to replace 3-(3,4-dimethoxy-benzylamino)-propionic acid ethyl ester, there was prepared 3-{[({[3-methoxy-4-(3-o-tolylureido)phenyl]-acetyl}-amino)-acetyl]-phenyl-amino}-propionic acid (Compound LD) as a beige solid. MS: 519 [MH]⁺.
(k) By proceeding in a manner similar to Example 6(a) but using 3-(3-ethoxy-4-methoxybenzylamino)-propionic acid ethyl ester [Reference Example 7(a)] to replace 3-(3.4-dimethoxybenzylamino)-propionic acid ethyl ester, there was prepared 3-{(3-ethoxy-4-methoxy-benzyl)-[({[3-methoxy-4-(3-o-tolylureido)phenyl]-acetyl}-amino)-acetyl]-amino}-propionic acid (Compound LE) as a white solid, m.p. 187-189°C. [Elemental analysis:- C,63.1; H,6.5; N,9.2% Calculated for C₃₂H₃₈N₄O₈:- C,63.35; H.6.3; N,9.2%]. MS: 607 [MH]⁺.
(I) By proceeding in a manner similar to Example 6(a) but using 3-(3,4-diethoxybenzylamino)-propionic acid ethyl ester [Reference Example 7(b)] to replace 3-(3,4-dimethoxybenzylamino)-propionic acid ethyl ester, there was prepared 3-{(3,4-diethoxy-benzyl)-[({[3-methoxy-4-(3-o-tolylureido)phenyl]acetyl}-amino)-acetyl]-amino}-propionic acid (Compound LF) as a white solid, m.p. 197-198°C. [Elemental analysis:- C,63.95; H,6.4; N,8.9% Calculated for C₃₃H₄₀N₄O₈:- C,63.9; H,6.5; N,9.0%]. MS: 621 [MH]⁺.
(m) By proceeding in a manner similar to Example 6(a) but using 3-(4-benzyloxy-3-methoxybenzylamino)-propionic acid ethyl ester [Reference Example 7(c)] to replace 3-(3,4-dimethoxybenzylamino)-propionic acid ethyl ester, there was prepared 3-{(4-benzyloxy-3-methoxybenzyl)-[({[3-methoxy-4-(3-o-tolylureido)phenyl]-acetyl}-amino)-acetyl]-amino}-propionic acid (Compound LG) as a white solid, m.p. 160-162°C. [Elemental analysis:- C,65.7; H,5.9; N,8.35% Calculated for C₃₇H₄₀N₄O₈•0.26H₂O:- C,66.0; H,6.1; N,8.3%]. MS: 669 [MH]⁺.
(n) By proceeding in a manner similar to Example 6(a) but using 3-[(1,4-benzodioxan-6-yl)-methylamino]-propionic acid ethyl ester [Reference Example 7(d)] to replace 3-(3,4-dimethoxybenzylamino)-propionic acid ethyl ester, there was prepared 3-{[(1,4-benzodioxan-6-yl)-methyl]-[({[3-methoxy-4-(3-o-tolylureido)phenyl]-acetyl}-amino)-acetyl]-amino}-propionic acid (Compound LH) as a white solid, m.p. 175-178°C (with decomposition). [Elemental analysis:-C,62.0; H,6.1; N.9.5%. Calculated for C₃₁H₃₄N₄O₈•0.55H₂O:- C,62.0; H.5.9; N,9.3%]. MS: 591 [MH]⁺.

### EXAMPLE 7

### Compound LI

Step1. By proceeding in a manner similar to step 1 of Example 6(a) but using 3-(3-tert-butoxycarbonylamino-prop-1-ylamino)-propionic acid ethyl ester [Reference Example 2(i)] to replace 3-(3,4-dirnethoxy-benzytamino)-propionic acid ethyl ester. there was prepared 3-{[({[3-methoxy-4-(3-o-tolylureido)phenyl]-acetyl}-amino)-acetyl]-(3-tert-butoxycarbonylamino-prop-1-yl)-amino}-propionic acid ethyl ester.

Step2. A solution of this material (0.75g) in dichloromethane (10ml) was treated with trinuoracetic acid (1.75ml) and stirred at ambient temperature for 2.5 hours. The reaction mixture was evaporated to give 3-{[({[3-methoxy-4-(3-o-tolylureido)phenyl]-acetyl)-amino)-acetyl]-(3-amino-prop-1-yl)-amino}-propionic acid ethyl ester trifluoroacetate salt (1.0g) which was treated with dichloromethane (10ml). The resulting solution was cooled to 0°C and then treated with triethylamine (1.8ml) followed by methanesulphonyl chloride (0.1ml). The reaction mixture was stirred for 3 hours at ambient temperature then diluted with dichloromethane (10ml) and washed with hydrochloric acid (10ml, 1M), then with water (10ml), then with saturated sodium bicarbonate solution (10ml) and then with brine. The organic layer was dried over magnesium sulphate and evaporated. The residue was subjected to flash chromatography on silica eluting with a mixture of ethyl acetate and methanol (9:1, v/v) to give a white foam (0.63g). This material was hydrolysed according to the procedure described in step 2 of Example 3(a) to give 3-{[({[3-methoxy-4-(3-o-tolylureido)phenyl]-acetyl}-amino)-acetyl]-(3-methanesulphonylamino-prop-1-yl)-amino}-propionic acid (Compound LI) as a colourless foam (0.43g). MS: 578 [MHJ⁺.

### EXAMPLE 8

### Compounds LJ to MD

By proceeding in a similar manner to Example 3, but using the appropriately substituted amines in step 2 and ({[3-methoxy-4-(3-o-tolylureido)phenyl]-acetyl}-N-methylamino)-acetic acid (Reference Example 1) or ({[3-methoxy-4-(3-o-tolylureido)phenyl]-acetyl}-amino)-acetic acid (Reference Example 3) in step 3 there were prepared Compounds LJ to MD depicted in Table 16.

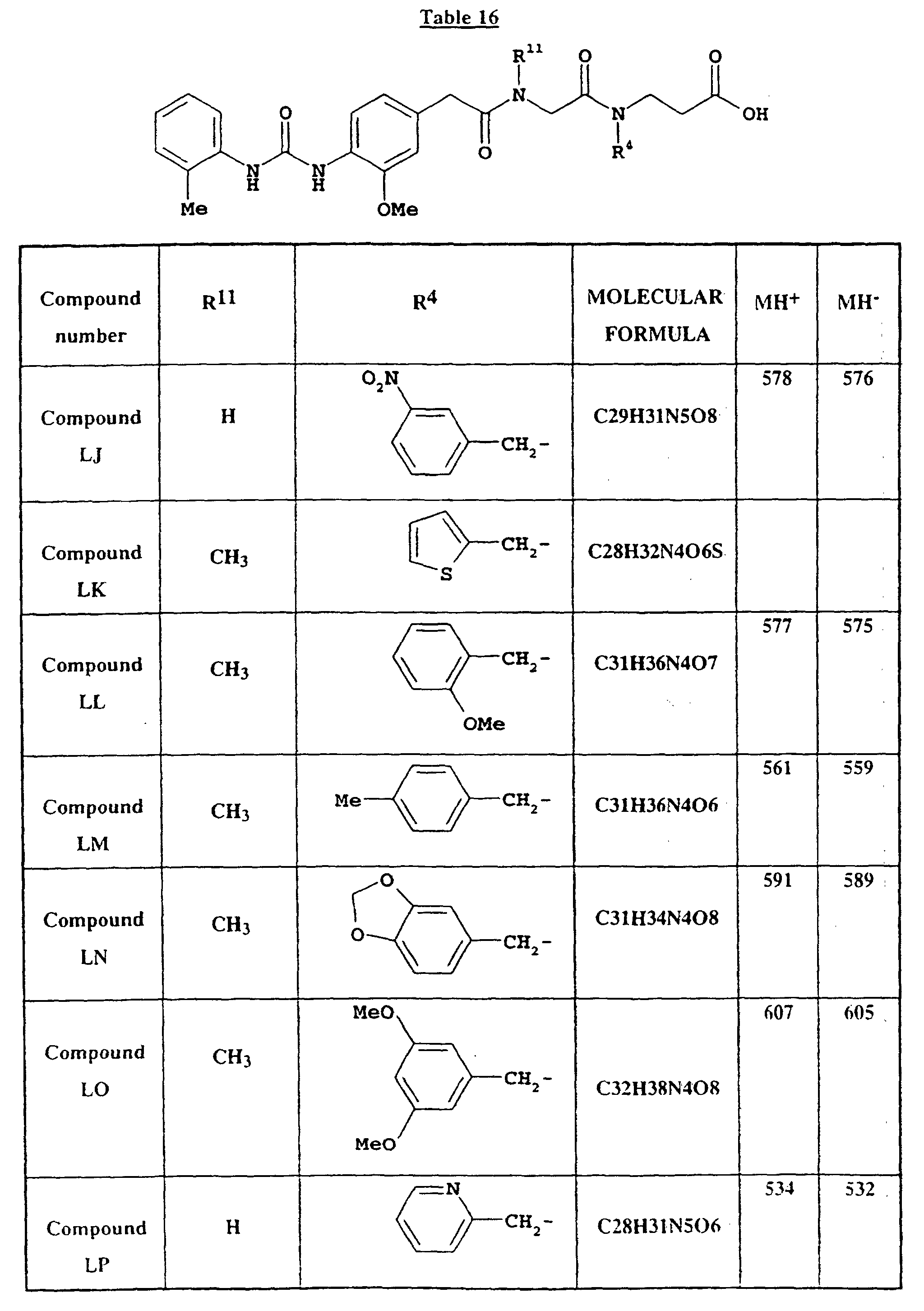

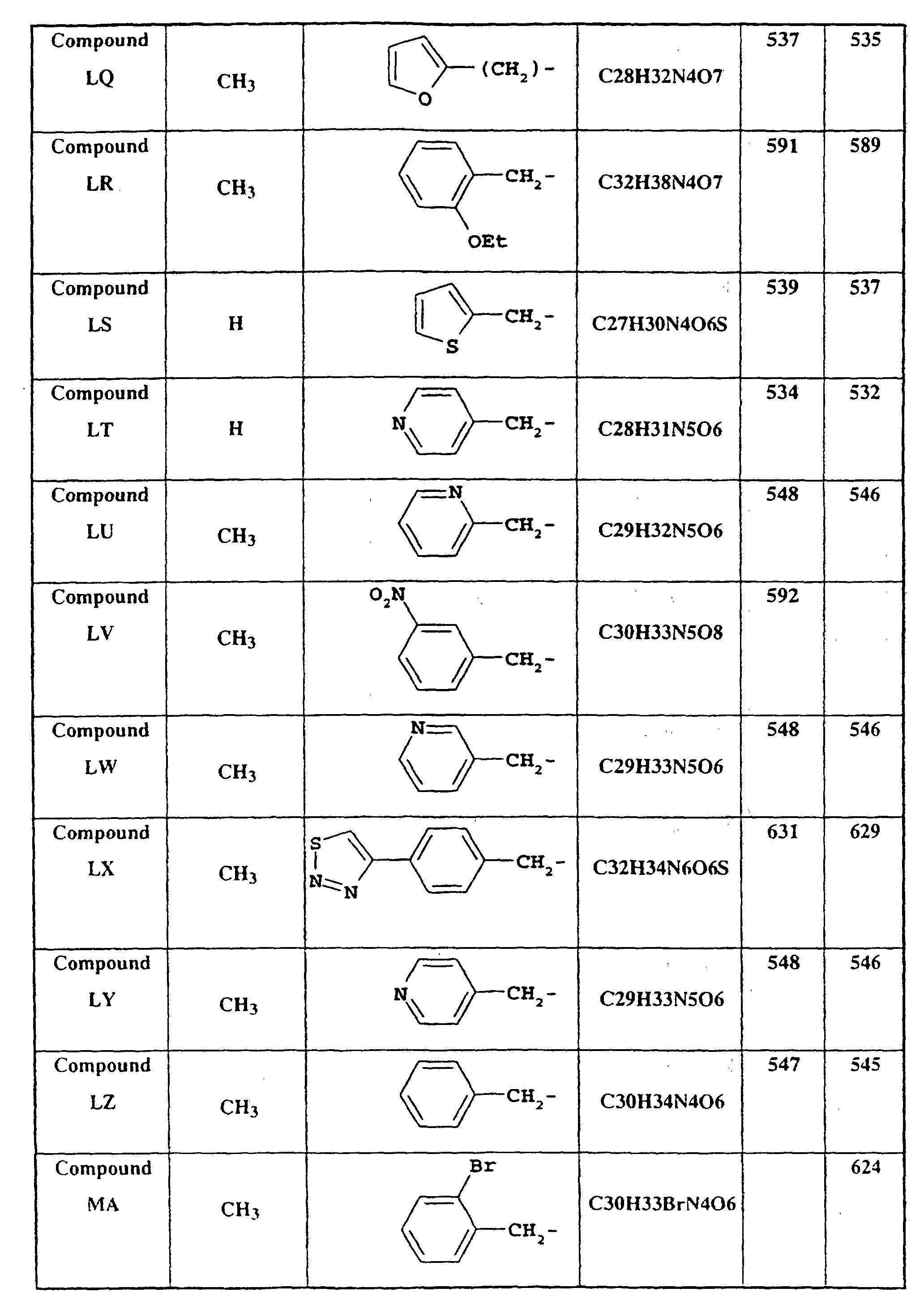

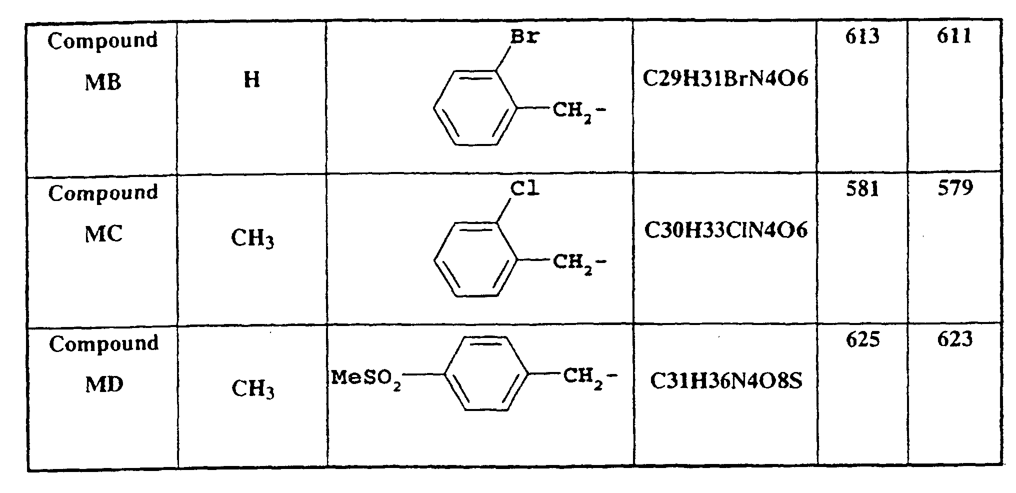

### EXAMPLE 9

(a) 3-{(3,4-Dimethoxy-benzyl)-[({[3-methoxy-4-(3-o-tolylureido)phenyl]-acetyl)-N-methylamino)-acetyl]-amino}-propionic acid sodium salt
   A solution of 3-{(3,4-dimethoxy-benzyl)-[({[3-methoxy-4-(3-o-tolylureido)phenyl]-acetyl}-N-methylamino)-acetyl]-amino}-propionic acid [17g, Example 5(a)] in ethanol (170ml) was treated with sodium hydroxide solution (23.3ml, IN). After stirring at ambient temperature for 24 hours the reaction mixture was filtered through a short pad of diatomaceous earth and then evaporated. The residue was triturated with hot ethyl acetate (200ml) and dried under vacuum. The resultant foam was dissolved in water (200ml) and freeze dried for 40 hours to yield the title compound as a white solid (15.4g), m.p. 225°C (with decomposition). [Elemental analysis:-C,57.8; H,6.05; N,8.2; Na,3.5%. Calculated for C₃₂H₃₇N₄NaO₈•2H₂O:- C,57.8; H,6.2; N,8.4; Na,3.5 % ].
(b) By proceeding in a manner similar to Example 9(a) but using 3-{[({[3-methoxy-4-(3-o-tolylureido)phenyl]-acetyl}-N-methylamino)-acetyl}-[3-(2-oxo-pyrrolidin-1-yl)-prop-1-yl}-amino}-propionic acid [Example 1(a)}, there was prepared 3-{[({[3-methoxy-4-(3-o-tolylureido)phenyl]-acetyl}-N-methylamino)-acetyl]-[3-(2-oxo-pyrrolidin-1-yl)-prop-1-yl]-amino}-propionic acid sodium salt. m.p. 213°C (with decomposition). [Elemental analysis:-C.57.55: H.6.4; N.10.85; Na.3.5% Calculated for C₃₀H₃₈N₅NaO₇•H₂O:- C.57.95: H,6.5; N,11.25; Na.3.7%].
(c) By proceeding in a manner similar to Example 9(a) but using 3-{(3,4-dimethoxy-benzyl)-[({[3-methoxy-4-(3-o-tolyl-ureido)-phenyl]-acetyl}-amino)-acetyl]-amino}-propionic acid [Example 6(a)], there was prepared 3-{[({[3-methoxy-4-(3-o-tolyl-ureido)-phenyl]-acetyl}-amino)-acetyl]-[3-(2-oxo-pyrrolidin-1-yl)-prop-1-yl]-amino}-propionic acid sodium salt, m.p. >250°C (with decomposition).

### REFERENCE EXAMPLE 1

### ({[3-Methoxy-4-(3-o-tolylureido)phenyl]-acetyl}-N-methylamino)-acetic acid

A solution of [3-methoxy-4-(3-o-tolylureido)phenyl]-acetic acid (2.50g, Reference Example 5) and sarcosine ethyl ester hydrochloride(1.23g) in dimethylformamide (75ml) was treated with [O-(7-azabenzotriazol-1-yl)-1,1,3,3,-tetramethyluronium hexafluorophosphate] (3.03g) and diisopropylethylamine (4.30ml). The reaction mixture was stirred at room temperature for 2 hours, then treated with water (200ml) and then filtered. The white solid was suspended in tetrahydrofuran (100ml) and the mixture was then treated with lithium hydroxide hydrate (0.45g) in water (20ml). After stirring for 45 minutes the mixture was concentrated to remove the tetrahydrofuran. The remaining aqueous phase was washed with ethyl acetate then acidified by addition of hydrochloric acid (1M), then extracted three times with ethyl acetate. The combined organic extracts were washed with brine, then dried over magnesium sulphate and then evaporated to yield the title compound (2.24g) as a white solid, m. p. 125-130°C (with decomposition). HPLC: R_{T}=10.83 minutes (gradient elution using a mixture of acetonitrile and water 1:4 to 4:1 v/v). MS(-ve) [M-1]⁻ 384.

### REFERENCE EXAMPLE 2

(a) 3-(3,4-dimethoxy-benzylamino)-propionic acid ethyl ester
   A mixture of 3,4-dimethoxy-benzylamine (100g) and ethyl acrylate (65ml) in ethanol (2L) and cylcohexane (1L) was stirred at room temperature for 20 hours then evaporated to give the title compound as a colourless oil (154g). MS: 268 [MH]⁺.
(b) By proceeding in a manner similar to Reference Example 2(a) but using 1-(3-aminoprop-1-yl)-imidazole there was prepared 3-(3-imidazol-1-yl-prop-1-ylamino)-propionic acid ethyl ester. MS: 226 [MH]⁺.
(c) By proceeding in a manner similar to Reference Example 2(a) but using 1-(3-aminoprop-1-yl)-2-pyrrolidinone there was prepared 3-[3-(2-oxo-pyrrolidin-1-yl)-prop-1-ylamino]-propionic acid ethyl ester. MS: 243 [MH]⁺.
(d) By proceeding in a manner similar to Reference Example 2(a) but using 4-amino-butanoic acid ethyl ester there was prepared 3-(3-carboxy-prop-1-ylamino)-propionic acid diethyl ester.
(e) By proceeding in a manner similar to Reference Example 2(a) but using 1-(2-aminoethyl)-2-pyrrolidinone there was prepared, 3-[2-(2-oxo-pyrrolidin-1-yl)-ethylamino]-propionic acid ethyl ester.
(f) By proceeding in a manner similar to Reference Example 2(a) but using β-alanine ethyl ester there was prepared 3-(2-carboxy-ethylamino)-propionic acid di-ethyl ester.
(g) By proceeding in a manner similar to Reference Example 2(a) but using 2,3-dimethoxy-benzylamine, there was prepared 3-(2.3-dimethoxy-benzylamino)-propionic acid ethyl ester.
(h) By proceeding in a manner similar to Reference Example 2(a) but using 3-phenyl-prop-1-ylamine, there was prepared 3-(3-phenyl-prop-1-ylamino)-propionic acid ethyl ester.
(i) By proceeding in a manner similar to Reference Example 2(a) but using 3-(tert-butoxycarbonytamino)prop-1-ylamine (prepared according to the procedure described by Muller, Dan; Zeltser, Irena; Bitan, Gal; Gilon, Chaim. J. Org. Chem. 1997,62, page 411-416), there was prepared 3-(3-tert-butoxycarbonylamino-prop-1-ylamino)-propionic acid ethyl ester.

### REFERENCE EXAMPLE 3

### ({[3-Methoxy-4-(3-o-tolylureido)phenyl]-acetyl}-amino)-acetic acid

A stirred solution of [3-methoxy-4-(3-o-tolylureido)phenyl]-acetic acid (7.53g, Reference Example 5) in a mixture of dimethylformamide (15ml) and dichloromethane (150ml) was treated with 1-(3-dimethylaminoprop-1-yl)-3-ethylcarbodiimide (4.98g), then with 1-hydroxybenzotriazole (3.57g), then with glycine methyl ester hydrochloride (3.01g) and then with diisopropylethylamine (4.10ml). After stirring at room temperature for 20 hours the reaction mixture was diluted with water (100ml). The organic phase was washed with saturated sodium bicarbonate (100ml), then with hydrochloric acid (1M) and then with brine. then dried over magnesium sulphate and then evaporated. The residue was triturated with petroleum ether and the resulting cream coloured solid (6.25g) was dissolved in tetrahydrofuran (200ml). The solution was treated with water (50ml) and then with lithium hydroxide hydrate (0.75g). The mixture was stirred at room temperature for 4 hours and then the tetrahydrnfuran was removed under vacuum. The aqueous phase was acidified by addition of hydrochloric acid (12M). The resulting solid was washed with diethyl ether and then dried to give the title compound (5.76g), m. p. 130-134°C (with decomposition). MS: 370 [M-1] -. HPLC: R_{T}=10.16 minutes (gradient elution using a mixture of acetonitrile and water 4:1 to 1:4 v/v).

### REFERENCE EXAMPLE 4

(a) 3-{(3,4-Dimethoxy-benzyl)-[({[3-methoxy-4-(3-o-tolylureido)phenyl]-acetyl)-N-methylamino)-acetyl]-amino}-propionic acid ethyl ester
   A solution of ({[3-methoxy-4-(3-o-tolylureido)phenyl]-acetyl}-N-methylamino)-acetic acid [18.4g, Reference Example 1] and 3-(3,4-dimethoxy-benzylamino)-propionic acid ethyl ester [13.4g, Reference Example 2(a)] in dimethylformamide (400ml) was treated with [O-(7-azabenzotriazol-1-yl)-1,1,3,3,-tetramethyluronium hexafluorophosphate] (19.1g) and diisopropylethylamine (10.5ml). After stirring at ambient temperature for 20 hours the reaction mixture was evaporated to dryness. The residue was treated with water (800ml) followed by hydrochloric acid (175ml, 1 M) and the mixture was extracted twice with ethyl acetate (500ml). The combined organic extracts were washed with hydrochloric acid (500ml, 1M), then with water (400ml), then with saturated aqueous sodium bicarbonate solution (500ml), then dried over magnesium sulphate and then evaporated. The residual oil was subjected to flash chromatography on silica eluting with a mixture of dichloromethane and methanol (49:1, v/v) to give the title compound as a fawn coloured foam (26.4g), m.p. 97-105°C. [Elemental analysis:- C,63.4; H,6.7; N,8.7% Calculated for C₃₄H₄₂N₄O₈•0.5H₂O:- C,63.3; H.6.8; N.8.8%].
(b) By proceeding in a manner similar to Reference Example 4(a) but using 3-(3-carboxy-prop-1-ylamino)-propionic acid di-ethyl ester [Reference Example 2(d)] to replace 3-(3,4-dimethoxy-benzylamino)-propionic acid ethyl ester, there was prepared 3-{[({[3-methoxy-4-(3-o-tolylureido)phenyl]-acetyl}-N-methylamino)-acetyl]-(3-carboxy-prop-1-yl)-amino}-propionic acid ethyl ester.
(c) By proceeding in a manner similar to Reference Example 4(a) but using 3-[2-(2-oxo-pyrrolidin-1-yl)-ethylamino]-propionic acid ethyl ester [Reference Example 2(e)] to replace 3-(3,4-dimethoxy-benzylamino)-propionic acid ethyl ester, there was prepared 3-{[({[3-methoxy-4-(3-o-tolylureido)phenyl]-acetyl}-N-methylamino)-acetyl]-[2-(2-oxo-pyrrolidin-1-yl)-ethyl]-amino}-propionic acid ethyl ester.

### REFERENCE EXAMPLE 5

### [3-Methoxy-4-(3-o-tolylureido)phenyl]acetic acid

A suspension of [3-methoxy-4-(3-o-tolylureido)phenyl]-acetic acid methyl ester (19.43g, Reference Example 6) in methanol (195ml) was treated with sodium hydroxide solution (65ml, IN) and the mixture was heated at reflux for 1 hour giving a clear solution. The reaction mixture was cooled to room temperature and then filtered. The filtrate was diluted to 390ml with water, then heated to 50°C and then acidified to pH 1 by the addition of hydrochloric acid (80ml, 1N) over 1 hour. The resulting suspension was stirred for a further 30 minutes at 50°C, then allowed to cool to room temperature and then filtered. The solid was washed with twice with water (200ml) then dried to give the title compound (15.72g) as a white solid, m. p. 179-181°C (with decomposition).

### REFERENCE EXAMPLE 6

### [3-methoxy-4-(3-o-tolylureido)phenyl]-acetic acid methyl ester

A suspension of potassium t-butoxide (1.44kg) in dimethylformamide(6.61), cooled to -5°C to -10°C, was treated with a mixture of 2-nitroanisole (690g) and methyl dichloroacetate (915g) over 4 hours, whilst maintaining the temperature below -5°C. The reaction mixture was then treated with acetic acid (770ml), then with water (6.6l) and then extracted three times with *tert*-butyl methyl ether (5.5l). The combined extracts were washed with water (5.5l), then with saturated sodium bicarbonate solution (5.5l), then with saturated brine (5.5l) and then dried over magnesium sulphate to give a solution of methyl α-chloro-3-methoxy-4-nitrophenylacetate. This solution was concentrated to half volume under reduced pressure and then treated with tetrahydrofuran (2l), followed by triethylamine (751ml), followed by 10% palladium on charcoal (58.4g) and the mixture was hydrogenated under a pressure of 50psi hydrogen at 50°C for 8 hours. The mixture was cooled to room temperature and filtered. The filtrate was dried over magnesium sulphate to give a solution of methyl 4-amino-3-methoxyphenytacetate which was heated to reflux and then treated with *o*-tolyl isocyanate (598.5g) over 30 minutes. After heating at reflux temperature for a further 3 hours, during which time a solid was deposited, the mixture was cooled to room temperature. The solid was collected, washed twice with *tert*-butyl methyl ether (4l), then dried in a vacuum oven at 60°C to give the title compound (764.8g) as a white solid, m.p. 167-168°C.

### REFERENCE EXAMPLE 7

(a) 3-(3-Ethoxy-4-methoxy-benzylamino)-propionic acid ethyl ester
   A mixture of β-alanine ethyl ester hydrochloride (1.6g), 3-ethoxy-4-methoxybenzaldehyde (1.8g), sodium cyanoborohydride (0.42g) and powdered 3Å molecular seives (2.5g) in ethanol (25ml) was stirred at ambient temperature for 24 hours. A further aliquot of sodium cyanoborohydride (0.42g) was added and stirring was continued for a further 24 hours. The reaction mixture was filtered and the filtrate was evaporated. The residue was treated with ethyl acetate (100ml) and the solution was washed with 10% aqueous potassium carbonate (50ml), then twice with water (25ml), then with brine (25ml), then dried over magnesium sulphate and then evaporated. The residual oil was subjected to flash chromatography on silica eluting with a mixture of dichloromethane and methanol (95:5, v/v) to give the title compound as a colourless oil (1.1g).
(b) By proceeding in a manner similar to Reference Example 7(a) but using 3,4-diethoxybenzaldehyde, there was prepared 3-(3,4-diethoxy-benzylamino)-propionic acid ethyl ester.
(c) By proceeding in a manner similar to Reference Example 7(a) but using 4-benzyloxy-3-methoxy-benzaldehyde, there was prepared 3-(4-benzyloxy-3-methoxy-benzylamino)-propionic acid ethyl ester.
(d) By proceeding in a manner similar to Reference Example 7(a) but using 1,4-benzodioxan-6-carboxaldehyde, there was prepared 3-[(1,4-benzodioxan-6-yl)-methylamino]-propionic acid ethyl ester.

### IN VITRO AND IN VIVO TEST PROCEDURES

### 1. Inhibitory effects of compounds on VLA4 dependent cell adhesion to Fibronectin and VCAM.

### 1.1 Metabolic labelling of RAMOS cells.

RAMOS cells (a pre-B cell line from ECACC, Porton Down. UK) are cultured in RPMI culture medium (Gibco, UK) supplemented with 5% foetal calf serum (FCS, Gibco, UK). Prior to assay the cells are suspended at a concentration of 0.5 X 10⁶ cells/ml RPMI and labelled with 400µCi/100mls of [³H]-methionine (Amersham, UK) for 18 hours at 37°C.

### 1.2 96 well plate preparation for adhesion assay.

Cytostar plates (Amersham, UK) were coated with 50µl/well of either 3µg/ml human soluble VCAM-1 (R&D Systems Ltd, UK) or 28.8µg/ml human tissue Fibronectin (Sigma, UK). In control non-specific binding wells 50µl phosphate buffered saline was added. The plates were then left to dry in an incubator at 25°C, overnight. The next day the plates were blocked with 200µl/well of Pucks buffer (Gibco, UK) supplemented with 1% BSA (Sigma, UK). The plates were left at room temperature in the dark for 2 hours. The blocking buffer was then disposed of and the plates dried by inverting the plate and gently tapping it on a paper tissue. 50µl/well of 3.6% dimethyl sulphoxide in Pucks buffer supplemented with 5mM manganese chloride (to activate the integrin receptor Sigma, UK) and 0.2% BSA (Sigma, UK), was added to the appropriate control test binding and non-specific binding assay wells in the plate. 50µl/well of the test compounds at the appropriate concentrations diluted in 3.6% dimethyl sulphoxide in Pucks buffer supplemented with 5mM manganese chloride and 0.2% BSA, was added to the test wells.
Metabolically labelled cells were suspended at 4 x 10⁶ cells/ml in Pucks buffer that was supplemented with manganese chloride and BSA as above. 50µl/well of cells in 1.8% dimethyl sulphoxide in Pucks buffer and supplements was added to all plate wells.
The same procedure exists for plates coated with either VCAM-1 or fibronectin and data is determined for compound inhibition of cell binding to both substrates.

### 1.3 Performance of assay and data analysis.

The plates containing cells in control or compound test wells are incubated in the dark at room temperature for 1 hour.
The plates are then counted on a Wallac Microbeta scintillation counter (Wallac, UK) and the captured data processed in Microsoft Excel (Microsoft. US). The data was expressed as an IC50, namely the concentration of inhibitor at which 50% of control binding occurs. The percentage binding is determined from the equation:${\text{{[(C}}_{\text{TB}} {\text{- C}}_{\text{NS}} {\text{)-(C}}_{\text{I}} {\text{- C}}_{\text{NS}} {\text{)] / (C}}_{\text{TB}} {\text{- C}}_{\text{NS}} \text{)}X 100 = % binding}$ where C_{TB} are the counts bound to fibronectin (or VCAM-1) coated wells without inhibitor present, C_{NS} are the counts present in wells without substrate, and C_{I} are the counts present in wells containing a cell adhesion inhibitor.

Compound data of this invention is expressed for IC₅₀s for inhibition of cell adhesion to both fibronectin and VCAM-1.

Compound data of this invention is expressed as IC₅₀s for inhibition of cell adhesion to both fibronectin and VCAM-1. Particular compounds of the invention inhibit cell adhesion to fibronectin and VCAM-1 with IC50s in the range 100 micromolar to 0.01 nanomolar. Preferred compounds of the invention inhibit cell adhesion to fibronectin and VCAM-1 with IC50s in the range 1.0 micromolar to 0.01 nanomolar. Especially preferred compounds of the invention inhibit cell adhesion to fibronectin and VCAM-1 with IC50s in the range 100 nanomolar to 0.01 nanomolar.

### 2. Inhibition of antigen-induced airway inflammation in the mouse and rat.

### 2.1 Sensitization of the animals.

Rats (Brown Norway, Harland Olac, UK) are sensitized on days 0, 12 and 21 with ovalbumin (100 µg, intraperitoneally [i.p], Sigma, UK) administered with aluminium hydroxide adjuvant (100mg, i.p., Sigma, UK) in saline (1ml, i.p.).
In addition mice (C57) are sensitized on days 0 and 12 with ovalbumin (10µg, i.p.) administered with aluminium hydroxide adjuvant (20mg, i.p.) in saline (0.2ml, i.p.).

### 2.2 Antigen challenge.

Rats are challenged on any one day between days 28-38, while mice are challenged on any one day between days 20-30.
The animals are challenged by exposure for 30 minutes (rats) or 1 hour (mice) to an aerosol of ovalbumin (10g / 1) generated by an ultrasonic nebulizer (deVilbiss Ultraneb, US) and passed into an exposure chamber.

### 2.3 Treatment protocols.

### 2.3 Treatment protocols.

Animals are treated as required before or after antigen challenge. The aqueous-soluble compounds of this invention can be prepared in water (for oral, p.o. dosing) or saline (for intratracheal, i.t. dosing). Non-soluble compounds are prepared as suspensions by grinding and sonicating the solid in 0.5 % methyl cellulose / 0.2 % polysorbate 80 in water (for p.o. dosing, both Merck UK Ltd., UK) or saline (for i.t. dosing). Dose volumes are: for rats 1ml / kg, p.o. or 0.5mg / kg, i.t.; for mice 10ml / kg, p.o. or 1ml / kg, i.t.

### 2.4 Assessment of airway inflammation.

The cell accumulation in the lung is assessed 24 hours after challenge (rats) or 48-72 hours after challenge (mice). The animals are euthanized with sodium pentobarbitone (200mg/kg, i.p., Pasteur Merieux, France) and the trachea is immediately cannulated. Cells are recovered from the airway lumen by bronchoalveolar tavage (BAL) and from the lung tissue by enzymatic (collagenase, Sigma, UK) disaggregation as follows.
BAL is performed by flushing the airways with 2 aliquots (each 10 mt/kg) RPMI 1640 medium (Gibco, UK) containing 10 % fetal calf serum (FCS, Serotec Ltd., UK). The recovered BAL aliquots are pooled and cell counts made as described below.
Immediately after BAL, the lung vasculature is flushed with RPMI 1640 / FCS to remove the blood pool of cells. The lung lobes are removed and cut into 0.5 mm pieces. Samples (rats: 400mg; mice: 150mg) of homogenous lung tissue are incubated in RPMI 1640 / FCS with collagenase (20 U/ml for 2 hours, then 60 U/ml for 1 hour, 37°C) to disaggregate cells from the tissue. Recovered cells are washed in RPMI 1640 / FCS.
Counts of total leukocytes recovered from the airway lumen and the lung tissue are made with an automated cell counter (Cobas Argos, US). Differential counts of eosinophils, neutrophils and mononuclear cells are made by light microscopy of cytocentrifuge preparations stained with Wright-Giemza stain (Sigma, UK). T cells are counted by flow cytometry (EPICS XL, Coulter Electronics, US) using fluophore-labelled antibodies against CD2 (a pan-T cell marker used to quantify total T cells), CD4, CD8 and CD25 (a marker of activated T cells). All antibodies were supplied by Serotec Ltd., UK)

### 2.5 Data analysis.

The cell data was expressed as mean cell numbers in unchallenged, challenged and vehicle treated, and challenged and compound treated groups, including the standard error of the means. Statistical analysis of the difference among treatment groups was evaluated using one-way analysis of variance via the Mann-Whitney test. Where p < 0.05 no statistical significance existed. The inhibitors of the invention caused a statistically significant reduction in cosinophil and lymphocyte numbers in the BAL and airway tissue. The inhibitors of the invention caused a statistically significant reduction in eosinophil and lymphocyte numbers in the BAL and airway tissue at doses within the range 100 mg/kg to 0.01 mg/kg.

### 3. Inhibition of Antigen Induced Airway Sensitivity in Allergic Sheep

The experiment was performed essentially as described in W. M. Abraham et al. J. Clin. invest.. (1994) Vol 93, 776-787. The experiment used allergic sheep which had been previously shown to develop early and late phase responses to inhaled challenge with Ascaris suum antigen. The inhibitors of the invention were delivered as an aerosol to the sheep and caused a statistically significant reduction of Ascaris suum induced airway responses when dosed at 1mg/kg.

## Claims

1. A compound of general formula (I):- 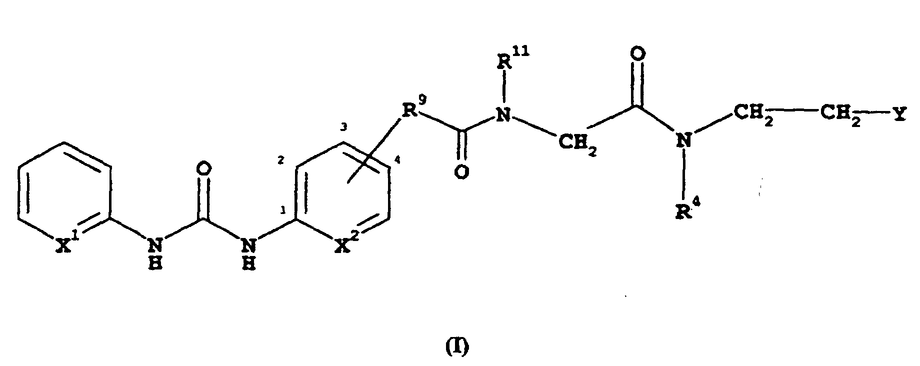 in which:
R⁴ is aryl or heteroaryl, or R⁴ is alkyl, alkenyl, alkynyl each optionally substituted by one or more groups chosen from halo, oxo, R⁵, -C(=O)-R⁷, -NH-C(=O)-R⁷ or -C(=O)NY¹Y², or R⁴ is cycloalkenyl, cycloalkyl or heterocycloalkyl each optionally substituted by one or more groups chosen from oxo, R⁶ or -L²-R⁶ {where R⁵ is a carboxyl (or corresponding protected derivative), aryl, cycloalkyl, cycloalkenyl, heteroaryl, heterocycloalkyl, -ZR⁷ or -NY¹Y²; R⁶ is a carboxyl (or corresponding protected derivative), aryl, heteroaryl, heterocycloalkyl, -ZH, -Z¹R⁷ or -NY¹Y²; R⁷ is alkyl, aryl, arylalkyl, cycloalkyl, cycloalkylalkyl, heteroaryl, heteroarylalkyl, heterocycloalkyl or heterocycloalkylalkyl;
L² is alkylene; Y¹ and Y² are independently hydrogen, acyl, alkyl [optionally substituted by hydroxy, heterocycloalkyl, or one or more carboxy or -C(=O)-NHR⁸ groups (where R⁸ is hydrogen or C₁₋₄alkyl)], alkylsulphonyl, aryl, arylalkyloxycarbonyl, arylsulphonyl, arylalkyl, heteroaryl, heteroarylalkyl, heterocycloalkyl or heterocycloalkylalkyl; or the group -NY¹Y² may form a 5-7 membered cyclic amine which (i) may be optionally substituted with one or more substituents selected from carboxamido, carboxy, hydroxy, oxo, hydroxyalkyl, HOCH₂CH₂-(OCH₂CH₂)ₘ- (where m is zero, or an integer one or two), or alkyl optionally substituted by carboxy or carboxamido (ii) may also contain a further heteroatom selected from O, N, S or SO₂ and (iii) may also be fused to additional aromatic, heteroaromatic, heterocycloalkyl or cycloalkyl rings to form a bicyclic or tricyclic ring system; Z is O or S; and Z¹ is O or S(O)ₘ};
R⁹ is a straight or branched C₁₋₆alkylene chain, a straight or branched C₂₋₆alkenylene chain or a straight or branched C₂₋₆alkynylene chain;
R¹¹ is a hydrogen atom or a group R⁴;
Y is carboy;
X¹ and X² each independently represent CR² (wherein R² is hydrogen, halogen, C₁₋₄alkyl or C₁₋₄alkoxy);
and -R⁹-CON(R¹¹)-CH₂-CON(R⁴)-CH₂-CH₂-Y is attached at the ring 3 or 4 position;
wherein said aryl and heteroaryl, whether as a group or part of a group, may be optionally substituted by aryl substituents selected from the group consisting of acyl, acylamino, alkoxy, alkoxycarbonyl, alkylsulphinyl, alkylsulphonyl, alkylthio, aroyl, aroylamino, aryl, arylalkyloxy, arylalkyloxycarbonyl, arylalkylthio, aryloxy, aryloaycarbonyl, arylsulphinyl, arylsulphonyl, arylthio, carboxy, cyano, halo, heteroaroyl, heteroaryl, heteroarylalkylory, heteroarylamino, heteroaryloxy, hydroxy, nitro, trifluoromethyl, Y³Y⁴N-, Y³Y⁴NCO-, Y³Y⁴NSO₂-(where Y³ and Y⁴ are independently hydrogen, alkyl, aryl, and arylalkyl), Y³Y⁴N-L³-Z²- (where L³ is C₂₋₆alkylene and Z² is O, NR⁸ or S(O)ₘ), alkylC(=O)-Y³N-, alkylSO₂-Y³N- or alkyl optionally substituted with aryl, heteroaryl, hydroxy, or Y³Y⁴N-,
and their ester prodrugs and pharmaceutically acceptable salts, and solvates of compounds of formula (I) and their ester prodrugs.

2. A compound according to claim 1 in which R⁹ represents straight or branched C₁₋₄alkylene.

3. A compound according to claim 1 in which R⁹ represents methylene.

4. A compound according to any preceding claim in which R¹¹ represents hydrogen, straight or branched C₁₋₄alkyl, straight or branched C₁₋₃alkyl substituted by aryl, heteroaryl, C₃₋₈cycloalkyl or carboxy, or straight or branched C₂₋₃alkyl substituted by -NY¹Y².

5. A compound according to any preceding claim in which R¹¹ represents hydrogen.

6. A compound according to any preceding claim in which R⁴ represents straight or branched C₁₋₆alkyl substituted by R⁵, where R⁵ is aryl.

7. A compound according to claim 6 in which R⁴ represents 3,4-diC₁₋₃alkoxybenzyl.

8. A compound according to any preceding claim in which the group -R⁹-C(=O)-N(R¹¹)-CH₂-C(=O)-NR⁴-CH₂-CH₂-Y is attached at the ring 4 position.

9. A compound according to claim 1 in which R⁴ represents C₁₋₁₀alkyl, C₁₋₆alkyl substituted by aryl, heteroaryl, cycloalkyl, heterocycloalkyl, C₁₋₆alkoxy, halo or -NY¹Y², or R⁴ represents C₁₋₄alkenyl; R¹¹ represents hydrogen, C₁₋₄alkyl, C₁₋₃alkyl substituted by aryl, heteroaryl, C₃₋₈cycloalkyl or carboxy, or R¹¹ represents C₂₋₃alkyl substituted by -NY¹Y²; R⁹ represents C₁₋₄alkylene; X¹ represents CR² where R² is C₁₋₄alkyl; X² represent CR² where R² is C₁₋₄alkoxy; Y represents carboxy; and the group -R⁹-C(=O)-N(R¹¹)-CH₂-C(=O)-NR⁴-CH₂-CH₂-Y is attached at the ring 4 position; and their ester prodrugs, and pharmaceutically acceptable salts and solvates of such compounds and their ester prodrugs.

10. A compound according to claim 1 selected from the following:
3-{[({[3-methoxy-4-(3-o-tolylureido)phenyl]-acetyl}-N-methylamino)-acetyl)-[3-(2-oxo-pyrrolidin-1-yl)-prop-1-yl]-amino}-propionic acid, Compound A;
3-{(3,4-dimethoxy-benzyl)-[({[3-methoxy-4-(3-o-tolylureido)phenyl]-acetyl}-N-methylamino)-acetyl]-amino}-propionic acid, Compound C;
3-{[({[3-methoxy-4-(3-o-tolylureido)phenyl]-acetyl}-amino)-aceryl]-[3-(2-oxo-pyrrolidin-1-yl)-prop-1-yl]-amino}-propionic acid, Compound D;
3-[(2,3-dimethoxy-benzyl)-({2-[3-methoxy-4-(3-o-tolylureido)phenyl]-acetylamino}-acetyl)-amino]-propionic acid, Compound AO;
3-[N-(3,4-dimethoxybenzyl)-2-{2-[3-methoxy-4-(3-o-tolylureido)phenyl]acetylamino}acetamido]-propionic acid, Compound BD;
3-{[({[3-methoxy-4-(3-o-tolylureido)phenyl]-acetyl}-N-methylamiao)-acetyl]-(3-carboxy-prop-1-yl)-amino}-propionic acid; Compound KW;
3-{(3-ethoxy-4-methoxy-benzyl)-[({[3-methoxy-4-(3-o-tolylureido)phenyl]-acetyl}-amino)-acetyl]-amino}-propionic acid; Compound LE;
3-{(3,4-diethoxy-benzyl)-[({[3-metboxy-4-(3-o-tolylureido)phenyl]-acetyl}-amino)-acetyl]-amino}-propionic acid; Compound LF;
and their ester prodrugs, and pharmaceutically acceptable salts and solvates of such compounds and their ester prodrugs.

11. 3-[N-(3,4-Dimethoxybenzyl)-2-{2-[3-methoxy-4-(3-o-tolylureido)phenyl]acetylamino}-acetamido]propionic acid, Compound BD, and its pharmaceutically acceptable salts and solvates.

12. A pharmaceutical composition comprising an effective amount of a compound according to claim 1 or a corresponding ester prodrug, or a pharmaceutically acceptable salt or solvate of such a compound or an ester prodrug thereof, in association with a pharmaceutically acceptable carrier or excipient.

13. A composition according to claim 12, for use in the treatment of a patient suffering from, or subject to, conditions which can be ameliorated by the administration of an inhibitor of α4β1 mediated cell adhesion.

14. A composition according to claim 12, for use in the treatment of inflammatory diseases.

15. A composition according to claim 12, for use in the treatment of asthma.

16. A compound according to claim 1 or a corresponding ester prodrug, or a pharmaceutically acceptable salt or solvate of such a compound or an ester prodrug thereof, for use in therapy.

17. A compound according to claim 16, wherein the therapy involves the treatment of a patient suffering from, or subject to, conditions which can be ameliorated by the administration of an inhibitor of α4β1 mediated cell adhesion.

18. A compound according to claim 16, wherein the therapy involves the treatment of inflammatory diseases.

19. A compound according to claim 16, wherein the therapy involves the treatment of asthma.

20. Use of a compound according to claim 1 or a corresponding ester prodrug, or a pharmaceutically acceptable salt or solvate of such a compound or an ester prodrug thereof, in the manufacture of a medicament for the treatment of a patient suffering from, or subject to, conditions which can be ameliorated by the administration of an inhibitor of α4β1 mediated cell adhesion.

21. Use of a compound according to claim I or a corresponding ester prodrug, or a pharmaceutically acceptable salt or solvate of such a compound or an ester prodrug thereof, in the manufacture of a medicament for the treatment of asthma.

22. Use of a compound according to claim 1 or a corresponding ester prodrug, or a pharmaceutically acceptable salt or solvate of such a compound or an ester prodrug thereof, in the manufacture of a medicament for the treatment of inflammatory diseases.

23. A resin selected from Resin 1, Resin 2, Resin 3, Resin 4, Resin 6, Resin 7 and Resin 8. 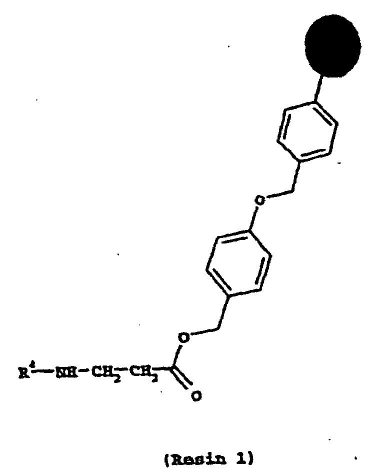 The residue R⁴ is an defined in claim 1 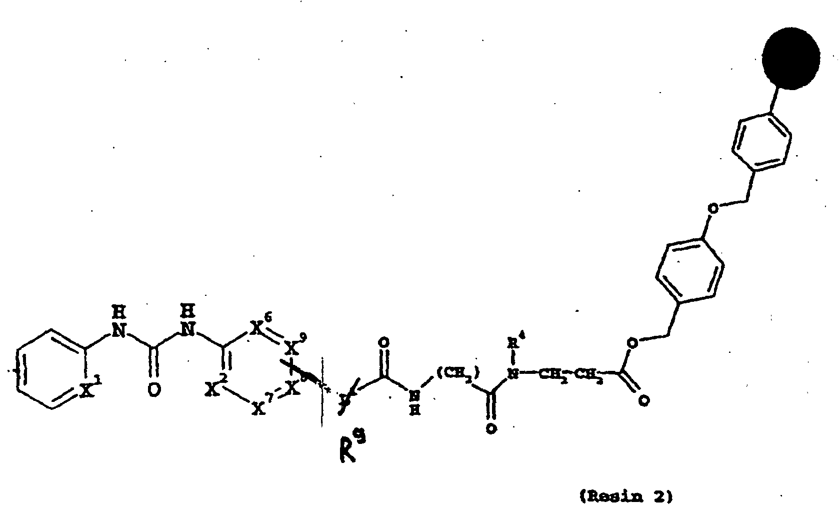 The residues X¹, X² , R⁴ and R⁹ are as defined in claim 1. X⁶ and X⁷ represent CH. One of X⁷ or X⁸ represents CH the other represent a carbon atom, which is attached to R⁹. 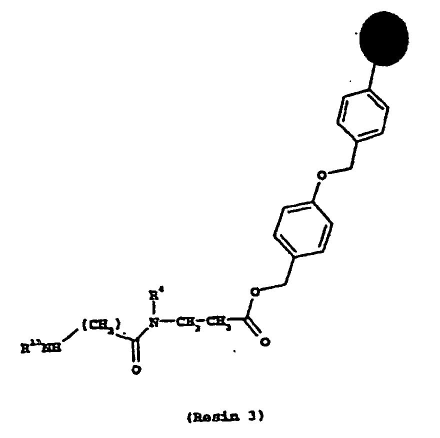 The resiclue R⁴ is as defined in claim 1, the residue R¹³ is a suitable amino protecting group. 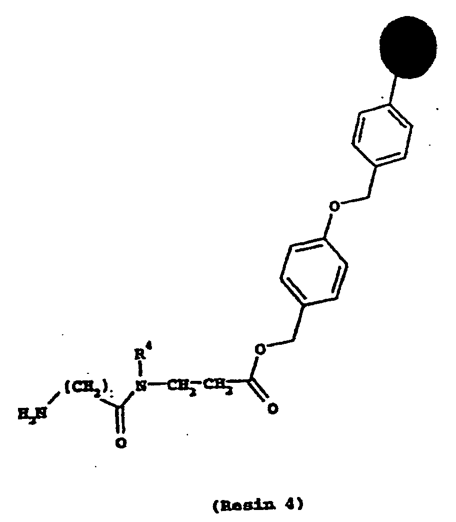 The residue R⁴ is as defined in claim 1. 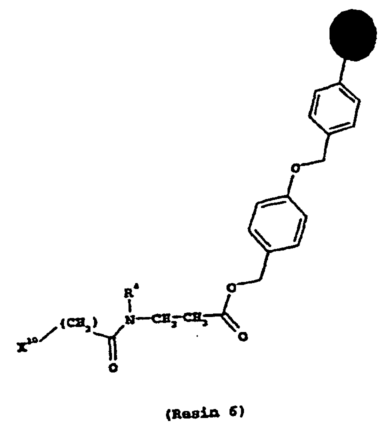 The residue R⁴ is as defined in claim 4. The residue X¹⁰ represents a halogen atom. 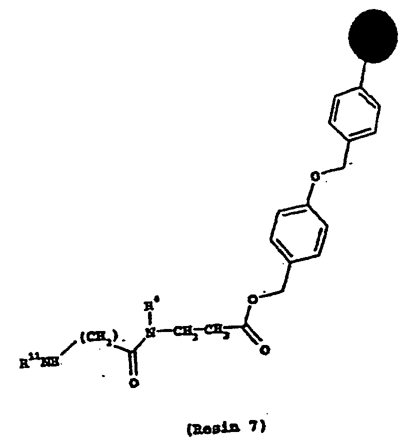 The residues R⁴ and R¹¹ are as defined in claim 1. 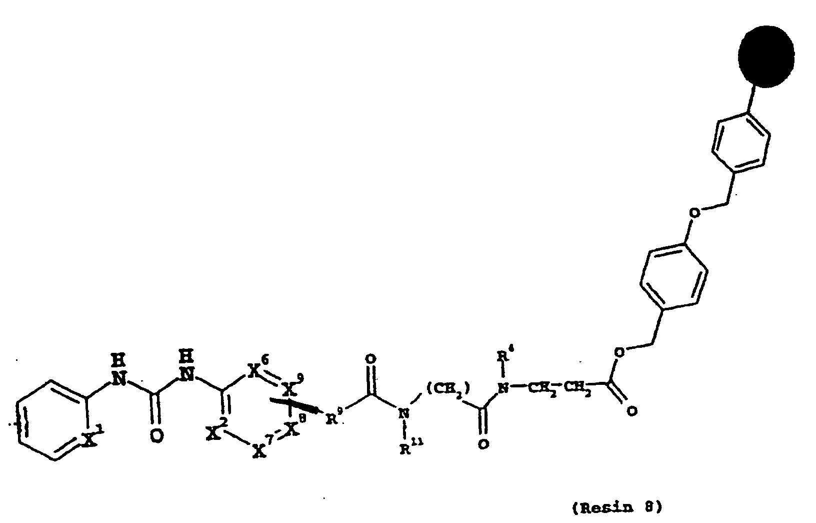 The residues are as defined for resin 2; R¹¹ is defined as in claim 1.

## Patentansprüche

1. Verbindung der allgemeinen Formel (I): 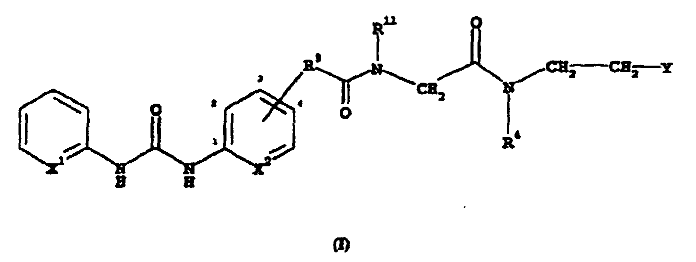 worin:
R⁴ Aryl oder Heteroaryl darstellt oder R⁴ Alkyl, Alkenyl, Alkinyl, jedes gegebenenfalls substituiert mit einer oder mehreren Gruppen, ausgewählt aus Halogen, Oxo, R⁵, -C(=O)-R⁷, -NH-C(=O)-R⁷ oder -C(=O)NY¹Y², darstellt oder R⁴ Cycloalkenyl, Cycloalkyl oder Heterocycloalkyl, jedes gegebenenfalls substituiert mit einer oder mehreren Gruppen, ausgewählt aus Oxo, R⁶ oder -L²-R⁶, darstellt (wobei R⁵ ein Carboxyl (oder entsprechend geschütztes Derivat), Aryl, Cycloalkyl, Cycloalkenyl, Heteroaryl, Heterocycloalkyl, -ZR⁷ oder -NY¹Y² darstellt; R⁶ ein Carboxyl (oder entsprechend geschütztes Derivat), Aryl, Heteroaryl, Heterocycloalkyl, -ZH, -Z¹R⁷ oder -NY¹Y² darstellt; R⁷ Alkyl, Aryl, Arylalkyl, Cycloalkyl, Cycloalkylalkyl, Heteroaryl, Heteroarylalkyl, Heterocycloalkyl oder Heterocycloalkylalkyl darstellt;
L² Alkylen darstellt; Y¹ und Y² unabhängig Wasserstoff, Acyl, Alkyl [gegebenenfalls substituiert mit Hydroxy, Heterocycloalkyl oder einer oder mehreren Gruppen Carboxy oder -C(=O)-NHR⁸ (worin R⁸ Wasserstoff oder C₁₋₄-Alkyl darstellt)], Alkylsulfonyl, Aryl, Arylalkyloxycarbonyl, Arylsulfonyl, Arylalkyl, Heteroaryl, Heteroarylalkyl, Heterocycloalkyl oder Heterocycloalkylalkyl darstellen; oder die Gruppe -NY¹Y² ein 5-7-gliedriges cyclisches Amin bilden kann, das (i) gegebenenfalls mit einem oder mehreren Substituenten, ausgewählt aus Carboxamido, Carboxy, Hydroxy, Oxo, Hydroxyalkyl, HOCH₂CH₂-(OCH₂CH₂)ₘ-(worin m null oder eine ganze Zahl eins oder zwei ist), oder Alkyl, gegebenenfalls substituiert mit Carboxy oder Carboxamido, substituiert sein kann, (ii) auch ein weiteres Heteroatom, ausgewählt aus O, N, S oder SO₂, enthalten kann und (iii) auch an weitere aromatische, heteroaromatische, Heterocycloalkyloder Cycloalkyl-Ringe zur Bildung eines bicyclischen oder tricyclischen Ringsystems kondensiert sein kann; Z O oder S darstellt; und Z¹ O oder S(O)ₘ darstellt};
R⁹ eine gerade oder verzweigte C₁₋₆-Alkylenkette, eine gerade oder verzweigte C₂₋₆-Alkenylenkette oder eine gerade oder verzweigte C₂₋₆-Alkinylenkette darstellt;
R¹¹ ein Wasserstoffatom oder eine Gruppe R⁴ darstellt;
Y Carboxy darstellt;
X¹ und X² jeweils unabhängig CR² wiedergeben (worin R² Wasserstoff, Halogen, C₁₋₄-Alkyl oder C₁₋₄-Alkoxy darstellt);
und -R⁹-CON(R¹¹)-CH₂-CON(R⁴)-CH₂-CH₂-Y an die Ring-3-oder 4-Position gebunden ist;
worin das Aryl und Heteroaryl, ob als eine Gruppe oder Teil einer Gruppe, gegebenenfalls mit Aryl-Substituenten substituiert sein kann, ausgewählt aus der Gruppe, bestehend aus Acyl, Acylamino, Alkoxy, Alkoxycarbonyl, Alkylsulfinyl, Alkylsulfonyl, Alkylthio, Aroyl, Aroylamino, Aryl, Arylalkyloxy, Arylalkyloxycarbonyl, Arylalkylthio, Aryloxy, Aryloxycarbonyl, Arylsulfinyl, Arylsulfonyl, Arylthio, Carboxy, Cyano, Halogen, Heteroaroyl, Heteroaryl, Heteroarylalkyloxy, Heteroarylamino, Heteroaryloxy, Hydroxy, Nitro, Trifluormethyl, Y³Y⁴N-, Y³Y⁴NCO-, Y³Y⁴NSO₂- (worin Y³ und Y⁴ unabhängig Wasserstoff, Alkyl, Aryl und Arylalkyl darstellen), Y³Y⁴N-L³-Z²- (worin L³ C₂₋₆-Alkylen darstellt und Z² O, NR⁸ oder S(O)ₘ darstellt), AlkylC(=O)-Y³N-, AlkylSO₂-Y³N- oder Alkyl, gegebenenfalls substituiert mit Aryl, Heteroaryl, Hydroxy oder Y³Y⁴N-,
und deren Esterprodrugs und pharmazeutisch verträgliche Salze und Solvate von Verbindungen der Formel (I) und deren Esterprodrugs.

2. Verbindung nach Anspruch 1, worin R⁹ gerades oder verzweigtes C₁₋₄-Alkylen wiedergibt.

3. Verbindung nach Anspruch 1, worin R⁹ Methylen wiedergibt.

4. Verbindung nach einem vorangehenden Anspruch, worin R¹¹ Wasserstoff, gerades oder verzweigtes C₁₋₄-Alkyl, gerades oder verzweigtes C₁₋₃-Alkyl, substituiert mit Aryl, Heteroaryl, C₃₋₈-Cycloalkyl oder Carboxy, oder gerades oder verzweigtes C₂₋₃-Alkyl, substituiert mit -NY¹Y², wiedergibt.

5. Verbindung nach einem vorangehenden Anspruch, worin R¹¹ Wasserstoff wiedergibt.

6. Verbindung nach einem vorangehenden Anspruch, worin R⁴ gerades oder verzweigtes C₁₋₆-Alkyl, substituiert mit R⁵, worin R⁵ Aryl darstellt, wiedergibt.

7. Verbindung nach Anspruch 6, worin R⁴ 3,4-Di-C₁₋₃-alkoxybenzyl wiedergibt.

8. Verbindung nach einem vorangehenden Anspruch, worin die Gruppe -R⁹-C(=O)-N(R¹¹)-CH₂-C(=O)-NR⁴-CH₂-CH₂-Y an die Ring-4-Position gebunden ist.

9. Verbindung nach Anspruch 1, worin R⁴ C₁₋₁₀-Alkyl, C₁₋₆-Alkyl, substituiert mit Aryl, Heteroaryl, Cycloalkyl, Heterocycloalkyl, C₁₋₆-Alkoxy, Halogen oder -NY¹Y², wiedergibt oder R⁴ C₁₋₄-Alkenyl wiedergibt; R¹¹ Wasserstoff, C₁₋₄-Alkyl, C₁₋₃-Alkyl, substituiert mit Aryl, Heteroaryl, C₃₋₈-Cycloalkyl oder Carboxy, wiedergibt oder R¹¹ C₂₋₃-Alkyl, substituiert mit -NY¹Y², wiedergibt; R⁹ C₁₋₄-Alkylen wiedergibt; X¹ CR² wiedergibt, worin R² C₁₋₄-Alkyl darstellt; X² CR² wiedergibt, worin R² C₁₋₄-Alkoxy darstellt; Y Carboxy wiedergibt; und die Gruppe -R⁹-C(=O)-N(R¹¹)-CH₂-C(=O)-NR⁴-CH₂-CH₂-Y an die Ring-4-Position gebunden ist; und deren Esterprodrugs und pharmazeutisch verträgliche Salze und Solvate von solchen Verbindungen und deren Esterprodrugs.

10. Verbindung nach Anspruch 1, ausgewählt aus den Nachstehenden:
3-{[({[3-Methoxy-4-(3-o-tolylureido)phenyl]-acetyl}-N-methylamino)-acetyl]-[3-(2-oxo-pyrrolidin-1-yl)-prop-1-yl]-amino}propionsäure, Verbindung A;
3-{(3,4-Dimethoxy-benzyl)-[({[3-methoxy-4-(3-o-tolylureido)-phenyl]-acetyl}-N-methylamino)-acetyl]-amino]-propionsäure, Verbindung C;
3-{[({[3-Methoxy-4-(3-o-tolylureido)phenyl]-acetyl}-amino)-acetyl]-[3-(2-oxo-pyrrolidin-1-yl)-prop-1-yl]-amino}-propionsäure, Verbindung D;
3-[(2,3-Dimethoxy-benzyl)-({2-[3-methoxy-4-(3-o-tolylureido)-phenyl]-acetylamino}-acetyl)-amino]-propionsäure, Verbindung AO;
3-[N-(3,4-Dimethoxybenzyl)-2-(2-[3-methoxy-4-(3-o-tolylureido)phenyl]acetylamino}acetamido]-propionsäure, Verbindung BD;
3-{[({[3-Methoxy-4-(3-o-tolylureido)phenyl]-acetyl}-N-methylamino)-acetyl]-(3-carboxy-prop-1-yl)-amino)-propionsäure, Verbindung KW;
3-{(3-Ethoxy-4-methoxy-benzyl)-[({[3-methoxy-4-(3-o-tolylureido)phenyl]-acetyl}-amino)-acetyl]-amino}-propionsäure, Verbindung LE;
3-{(3,4-Diethoxy-benzyl)-[({[3-methoxy-4-(3-o-tolylureido)phenyl]-acetyl)-amino)-acetyl]amino}-propionsäure, Verbindung LF;
und deren Esterprodrugs und pharmazeutisch verträgliche Salze und Solvate von solchen Verbindungen und deren Esterprodrugs.

11. 3-[N-(3,4-Dimethoxybenzyl)-2-{2-[3-methoxy-4-(3-o-tolylureido)phenyl]acetylamino}-acetamido]propionsäure, Verbindung BD und deren pharmazeutisch verträgliche Salze und Solvate.

12. Pharmazeutische Zusammensetzung, umfassend eine wirksame Menge einer Verbindung nach Anspruch 1 oder ein entsprechendes Esterprodrug oder ein pharmazeutisch verträgliches Salz oder Solvat einer solchen Verbindung oder eines Esterprodrugs davon in Verbindung mit einem pharmazeutisch verträglichen Träger oder Exipienten.

13. Zusammensetzung nach Anspruch 12 zur Verwendung bei der Behandlung eines Patienten, der unter Zuständen, die durch die Verabreichung eines Inhibitors von α4β1-vermittelter Zelladhäsion gemildert werden können, leidet oder dafür anfällig ist.

14. Zusammensetzung nach Anspruch 12 zur Verwendung bei der Behandlung von entzündlichen Erkrankungen.

15. Zusammensetzung nach Anspruch 12 zur Verwendung bei der Behandlung von Asthma.

16. Verbindung nach Anspruch 1 oder ein entsprechendes Esterprodrug oder ein pharmazeutisch verträgliches Salz oder Solvat von einer solchen Verbindung oder ein Esterprodrug davon zur Verwendung bei der Therapie.

17. Verbindung nach Anspruch 16, worin die Therapie die Behandlung eines Patienten beinhaltet, der unter Zuständen, die durch die Verabreichung eines Inhibitors von α4β1-vermittelter Zelladhäsion gemildert werden können, leidet oder dafür anfällig ist.

18. Verbindung nach Anspruch 16, worin die Therapie die Behandlung von entzündlichen Erkrankungen beinhaltet.

19. Verbindung nach Anspruch 16, worin die Therapie die Behandlung von Asthma beinhaltet.

20. Verwendung einer Verbindung nach Anspruch 1 oder eines entsprechenden Esterprodrugs oder eines pharmazeutisch verträglichen Salzes oder Solvats einer solchen Verbindung oder eines Esterprodrugs davon bei der Herstellung eines Arzneimittels für die Behandlung eines Patienten, der unter Zuständen, die durch die Verabreichung eines Inhibitors von α4β1-vermittelter Zelladhäsion gelindert werden können, leidet oder dafür anfällig ist.

21. Verwendung einer Verbindung nach Anspruch 1 oder eines entsprechenden Esterprodrugs oder eines pharmazeutisch verträglichen Salzes oder Solvats einer solchen Verbindung oder eines Esterprodrugs davon bei der Herstellung eines Arzneimittels für die Behandlung von Asthma.

22. Verwendung einer Verbindung nach Anspruch 1 oder eines entsprechenden Esterprodrugs oder eines pharmazeutisch verträglichen Salzes oder Solvats einer solchen Verbindung oder eines Esterprodrugs davon bei der Herstellung eines Arzneimittels für die Behandlung von entzündlichen Erkrankungen.

23. Harz, ausgewählt aus Harz 1, Harz 2, Harz 3, Harz 4, Harz 6, Harz 7 und Harz 8 wie folgt: 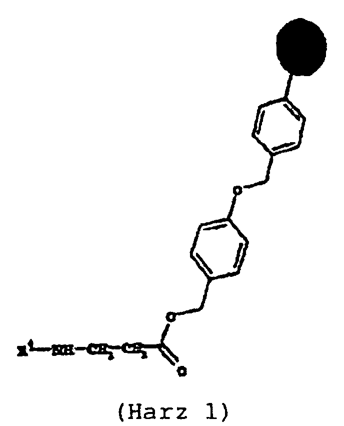 Der Rest R⁴ ist wie in Anspruch 1 definiert. 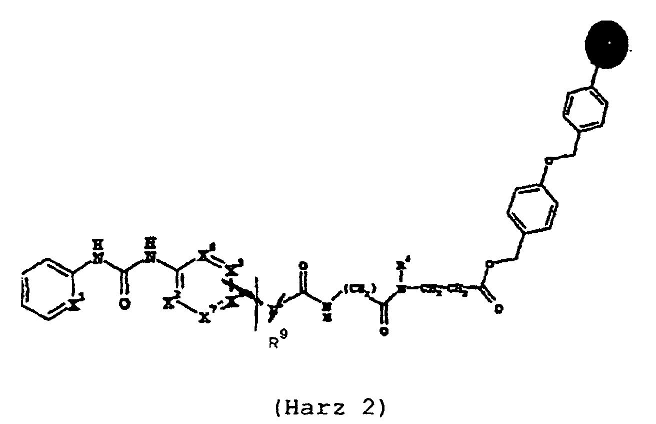 Die Reste X¹, X², R⁴ und R⁹ sind wie in Anspruch 1 definiert. X⁶ und X⁷ geben CH wieder. Einer von X⁷ oder X⁸ gibt CH wieder, der andere gibt ein Kohlenstoffatom wieder, das an R⁹ gebunden ist. 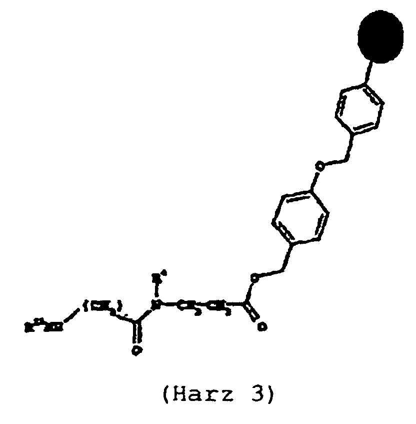 Der Rest R⁴ ist wie in Anspruch 1 definiert, der Rest R¹³ ist eine geeignete Amino-Schutzgruppe. 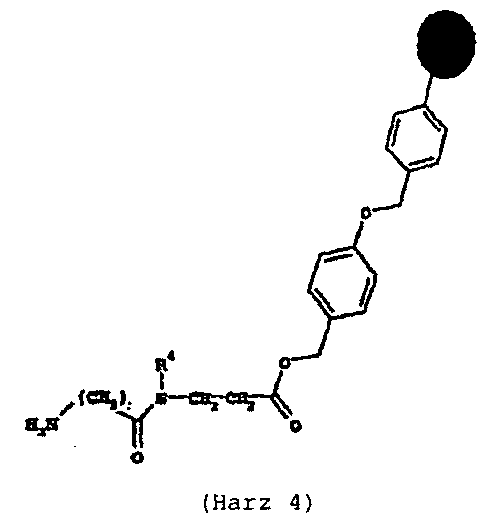 Der Rest R⁴ ist wie in Anspruch 1 definiert. 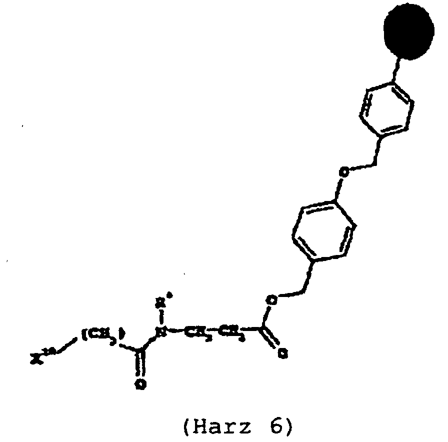 Der Rest R⁴ ist wie in Anspruch 4 definiert. Der Rest X¹⁰ gibt ein Halogenatom wieder. 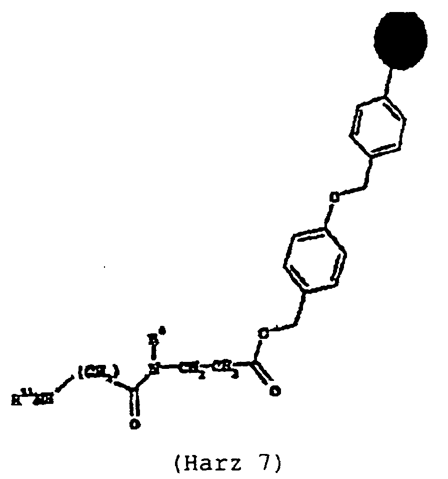 Die Reste R⁴ und R¹¹ sind wie in Anspruch 1 definiert. 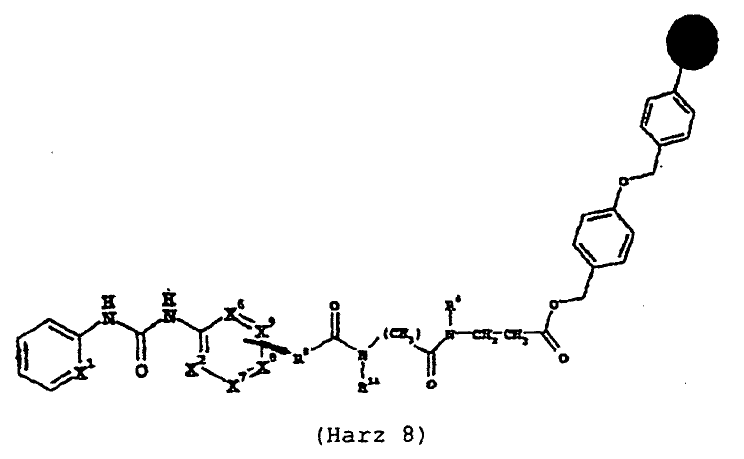 Die Reste sind wie für Harz 2 definiert; R¹¹ ist wie in Anspruch 1 definiert.

## Revendications

1. Composé de formule générale (I): 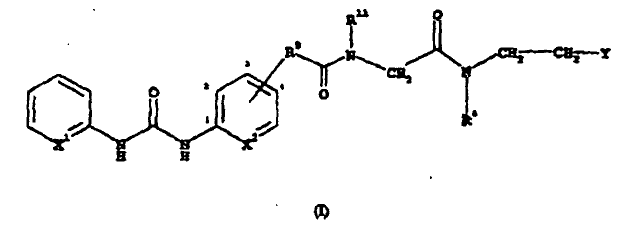 dans lequel:
R⁴ est un groupe aryle ou hétéroaryle ou dans lequel R⁴ est un groupe alkyle, alcényle, alcynyle, chaque groupe étant éventuellement substitué avec un ou plusieurs groupes sélectionnés parmi un halogène, un groupe oxo, R⁵, -C(=O)-R⁷, -NH-C(=O)-R⁷ ou -C(=O)-NY¹Y², ou dans lequel R⁴ est un groupe cycloalcényle, cycloalkyle ou hétérocycloalkyle, chaque groupe étant éventuellement substitué avec un ou plusieurs groupes sélectionnés parmi un groupe oxo, R⁶ ou -L²-R⁶, R⁵ étant un groupe carboxyle (ou un dérivé protégé correspondant), aryle, cycloalkyle, cycloalcényle, hétéroaryle, hétérocycloalkyle, -ZR⁷ ou -NY¹Y², R⁶ étant un groupe carboxyle (ou un dérivé protégé correspondant), aryle, hétéroaryle, hétérocycloalkyle, -ZH, -Z¹R⁷ ou -NY¹Y², R⁷ étant un groupe alkyle, aryle, arylalkyle, cycloalkyle, cycloalkylalkyle, hétéroaryle, hétéroarylalkyle, hétérocycloalkyle ou hétérocycloalkylalkyle,
L² est un groupe alkylène, Y¹ et Y² représentent indépendamment l'hydrogène, un groupe acyle, alkyle éventuellement substitué avec un groupe hydroxy, hétérocycloalkyle ou avec un ou plusieurs groupes carboxy ou -C(=O)-NHR⁸, R⁸ représentant l'hydrogène ou un groupe alkyle en C₁₋₄, alkylsulfonyle, aryle, arylalkyloxycarbonyle, arylsulfonyle, arylalkyle, hétéroaryle, hétéroarylalkyle, hétérocycloalkyle ou hétérocycloalkylalkyle; ou le groupe -NY¹Y² peut former une amine cyclique à 5 ou 7 chaînons qui (i) peut être éventuellement substituée avec un ou plusieurs substituants sélectionnés parmi les groupes carboxamido, carboxy, hydroxy, oxo, hydroxyalkyle, HOCH₂CH₂-(OCH₂CH₂)m- (m étant égal à zéro ou un nombre entier valant un ou deux) ou alkyle, éventuellement substitué avec un groupe carboxy ou carboxamido, (ii) peut également comprendre un autre hétéroatome sélectionné parmi O, N, S ou SO₂ et (iii) peut également être fusionnée à des composés cycliques aromatiques, hétéroaromatiques, hétérocycloalkyle ou cycloalkyle supplémentaires pour former un système bicyclique ou tricyclique, Z représentant 0 ou S et Z¹ étant O ou S(O)ₘ},
R⁹ est une chaîne linéaire ou ramifiée d'alcylène en C₁₋₆, une chaîne linéaire ou ramifiée d'alcénylène en C₂₋₆ ou une chaîne linéaire ou ramifiée d'alcynylène en C₂₋₆,
R¹¹ est un atome d'hydrogène ou un groupe R⁴,
Y est un groupe carboxy,
X¹ et X² représentent chacun indépendamment CR² (R² étant l'hydrogène, un halogène, un alkyle en C₁₋₄ ou un alcoxy en C₁₋₄),
et -R⁹-CON(R¹¹)-CH₂-CON(R⁴)-CH₂-CH₂-Y est fixé en position 3 ou 4 du cycle,
lesdits groupes aryle et hétéroaryle, qu'ils forment un groupe ou une partie d'un groupe, peuvent être éventuellement substitués avec des substituants aryles sélectionnés parmi le groupe constitué des groupes acyle, acylamino, alcoxy, alcoxycarbonyle, alkylsulfinyle, alkylsulfonyle, alkylthio, aroyle, aroylamino, aryle, arylalkyloxy, arylalkyloxycarbonyle, arylalkylthio, aryloxy, aryloxycarbonyle, arylsulfinyle, arylsulfonyle, arylthio, carboxy, cyano, des halogènes, des groupes hétéroaroyle, hétéroaryle, hétéroarylalkyloxy, hétéroarylamino, hétéroaryloxy, hydroxy, nitro, trifluorométhyle, Y³Y⁴N-, Y³Y⁴NCO-, Y³Y⁴NSO₂- (Y³ et Y⁴ représentant indépendamment l'hydrogène, un groupe alkyle, aryle et arylalkyle), Y³Y⁴N-L³-Z²- (L³ étant un groupe alkylène en C₂₋₆ et Z² représentant O, NR⁸ ou S(O)ₘ), un groupe alkyl-C(=O)-Y³N-, alkyl-SO₂-Y³N- ou alkyle éventuellement substitué avec un groupe aryle, hétéroaryle, hydroxy ou Y³Y⁴N-, ainsi que leurs précurseurs de médicament du type ester, et les sels et les solvates pharmaceutiquement acceptables des composés de formule (I) et de leurs précurseurs de médicament du type ester.

2. Composé selon la revendication 1, dans lequel R⁹ représente une chaîne linéaire ou ramifiée d'alkylène en C₁₋₄.

3. Composé selon la revendication 1, dans lequel R9 représente un groupe méthylène.

4. Composé selon l'une quelconque des revendications précédentes, dans lequel R¹¹ représente l'hydrogène, une chaîne linéaire ou ramifiée d'alkyle en C₁₋₄, une chaîne linéaire ou ramifiée d'alkyle en C₁₋₃ substitué avec un groupe aryle, hétéroaryle, cycloalkyle en C₃₋₈ ou carboxy ou une chaîne linéaire ou ramifiée d'alkyle en C₂₋₃ substitué avec -NY¹Y².

5. Composé selon l'une quelconque des revendications précédentes, dans lequel R¹¹ représente l'hydrogène.

6. Composé selon l'une quelconque des revendications précédentes, dans lequel R⁴ représente une chaîne linéaire ou ramifiée d'alkyle en C₁₋₆ substitué avec R⁵, R⁵ étant un groupe aryle.

7. Composé selon la revendication 6, dans lequel R⁴ représente un groupe 3,4-di((alcoxy en C₁₋₃)benzyle).

8. Composé selon l'une quelconque des revendications précédentes, dans lequel le groupe -R⁹-C(=O)-N(R¹¹)-CH₂-C(=O)-NR⁴-CH₂-CH₂-Y est fixé en position 4 du cycle.

9. Composé selon la revendication 1, dans lequel R⁴ représente un groupe alkyle en C₁₋₁₀, alkyle en C₁₋₆ substitué avec un groupe aryle, hétéroaryle, cycloalkyle, hétérocycloalkyle, alcoxy en C₁₋₆, un halogène ou -NY¹Y² ou dans lequel R⁴ représente un groupe alcényle en C₁₋₄, R¹¹ représentant l'hydrogène, un groupe alkyle en C₁₋₄, alkyle en C₁₋₃ substitué avec un groupe aryle, hétéroaryle, cycloalkyle en C₃₋₈ ou carboxy, ou R¹¹ représente un groupe alkyle en C₂₋₃ substitué avec -NY¹Y², R⁹ représentant un groupe alkylène en C₁₋₄, X¹ représentant CR² dans lequel R² est un groupe alkyle en C₁₋₄, X² représentant CR² dans lequel R² est un groupe alcoxy en C₁₋₄, Y représentant un groupe carboxy et le groupe -R⁹-C(=O)-N(R¹¹)-CH₂-C(=O)-NR⁴-CH₂-CH₂-Y étant fixé en position 4 du cycle, ainsi que leurs précurseurs de médicament du type ester, les sels et les solvates pharmaceutiquement acceptables de ces composés et de leurs précurseurs de médicament du type ester.

10. Composé selon la revendication 1, qui est sélectionné parmi :
l'acide 3-{[({[3-méthoxy-4-(3-o-tolyluréido)phényl]-acétyl}-N-méthylamino)-acétyle]-[3-(2-oxo-pyrrolidine-1-yl)-prop-1-yl]-amino}-propionique, composé A,
l'acide 3-{(3,4-diméthoxy-benzyl)-[({[3-méthoxy-4-(3-o-tolyluréido)phényl]-acétyl}-N-méthylamino)-acétyl]-amino}-propionique, composé C,
l'acide 3-{[({[3-méthoxy-4-(3-o-tolyluréido)phényl]-acétyl}-amino)-acétyl]-[3-(2-oxo-pyrrolidine-1-yl)-prop-1-yl]-amino}-propionique, composé D,
l'acide 3-[(2,3-diméthoxy-benzyl)-({2-[3-méthoxy-4-(3-o-tolyluréido)phényl]-acétylamino}-acétyl)-amino]-propionique, composé AO,
l'acide 3-[N-(3,4-diméthoxybenzyl)-2-{2-[3-méthoxy-4-(3-o-tolyluréido)phényl]acétylamino}acétamido]-propionique, composé BD,
l'acide 3-{[({[3-méthoxy-4-(3-o-tolyluréido)phényl]-acétyle}-N-méthylamino)-acétyl]-(3-carboxy-prop-1-yl)-amino}-propionique, composé KW,
l'acide 3-{(3-éthoxy-4-méthoxy-benzyl)-[({(3-méthoxy-4-(3-o-tolyluréido)phényl]-acétyl}-amino)-acétyl]-amino}- propionique, composé LE,
l'acide 3-{(3,4-diéthoxy-benzyl)-[({[3-méthoxy-4-(3-o-tolyluréido)phényl]-acétyl}-amino)-acétyl]-amino}-propionique, composé LF,
et leurs précurseurs de médicament du type ester, les sels et les solvates pharmaceutiquement acceptables de ces composés et de leurs précurseurs de médicament du type ester.

11. Acide 3-[N-(3,4-diméthoxybenzyl)-2-{2-[3-méthoxy-4-(3-o-tolyluréido)phényl]acétylamino}-acétamido]-propionique, composé BD, ses sels et ses solvates pharmaceutiquement acceptables.

12. Composition pharmaceutique qui comprend une quantité efficace d'un composé selon la revendication 1, ou d'un précurseur de médicament correspondant du type ester, ou d'un sel pharmaceutiquement acceptable, ou solvate d'un tel composé ou de son précurseur de médicament du type ester, en association avec un porteur ou un excipient pharmaceutiquement acceptables.

13. Composition selon la revendication 12, destinée à être utilisée dans le traitement d'un patient qui souffre d'états ou qui est sujet à des états qui peuvent être améliorés par l'administration d'un inhibiteur d'adhérence cellulaire médiée par α4β1.

14. Composition selon la revendication 12, destinée à être utilisée dans le traitement de maladies inflammatoires.

15. Composition selon la revendication 12, destinée à être utilisée dans le traitement de l'asthme.

16. Composé selon la revendication 1 ou précurseur de médicament du type ester correspondant, ou sel pharmaceutiquement acceptable ou solvate d'un tel composé ou de son précurseur de médicament du type ester, destiné à être utilisé en thérapie.

17. Composé selon la revendication 16, dans lequel la thérapie consiste en le traitement d'un patient qui souffre d'états ou qui est sujet à des états qui peuvent être améliorés par l'administration d'un inhibiteur d'adhérence cellulaire médiée par α4β1.

18. Composé selon la revendication 16, dans lequel la thérapie consiste en le traitement des maladies inflammatoires.

19. Composé selon la revendication 16, dans lequel la thérapie consiste en le traitement de l'asthme.

20. Utilisation d'un composé selon la revendication 1, ou d'un précurseur de médicament du type ester correspondant, ou d'un sel pharmaceutiquement acceptable ou solvate d'un tel composé ou de son précurseur de médicament du type ester, dans la préparation d'un médicament destiné au traitement d'un patient qui souffre d'états ou qui est sujet à des états qui peuvent être améliorés par l'administration d'un inhibiteur d'adhérence cellulaire médiée par α4β1.

21. Utilisation d'un composé selon la revendication 1, ou d'un précurseur de médicament du type ester correspondant, ou d'un sel pharmaceutiquement acceptable ou solvate d'un tel composé ou de son précurseur de médicament du type ester, dans la préparation d'un médicament destiné au traitement de l'asthme.

22. Utilisation d'un composé selon la revendication 1, ou d'un précurseur de médicament du type ester correspondant, ou d'un sel pharmaceutiquement acceptable ou solvate d'un tel composé ou de son précurseur de médicament du type ester, dans la préparation d'un médicament destiné au traitement des maladies inflammatoires.

23. Résine sélectionnée parmi la résine 1, la résine 2, la résine 3, la résine 4, la résine 6, la résine 7 et la résine 8. 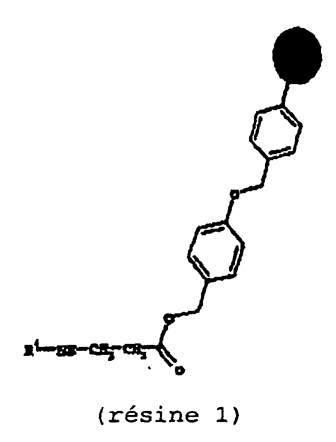 Le groupe R⁴ est défini dans la revendication 1 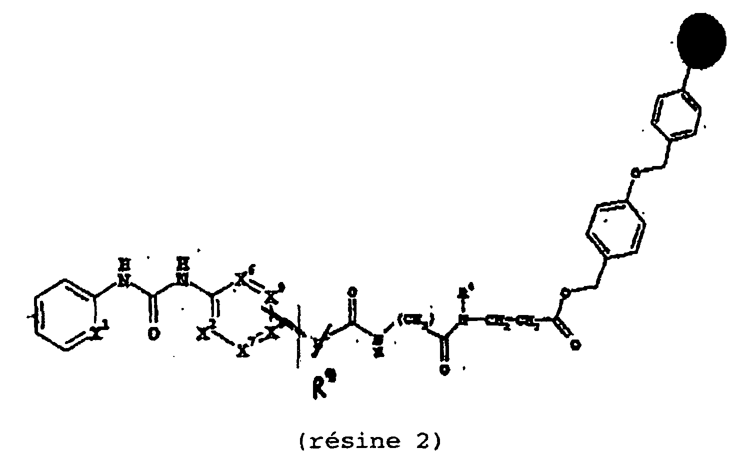 Les groupes X¹, X², R⁴ et R⁹ sont définis dans la revendication 1. X⁶ et X⁷ représentent CH. L'un parmi X⁷ ou X⁸ représente CH et l'autre représente un atome de carbone qui est fixé à R⁹. 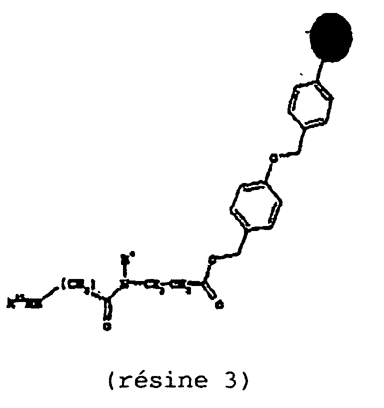 Le groupe R⁴ est défini dans la revendication 1, le groupe R¹³ étant un groupe protecteur amino approprié. 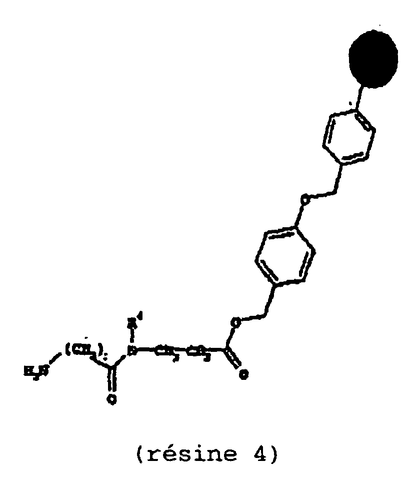 Le groupe R⁴ est défini dans la revendication 1. 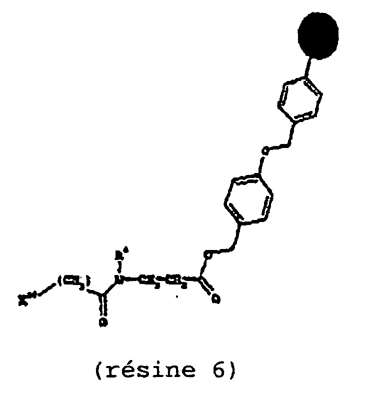 Le groupe R⁴ est défini dans la revendication 4. Le groupe X¹⁰ représente un atome d'halogène. 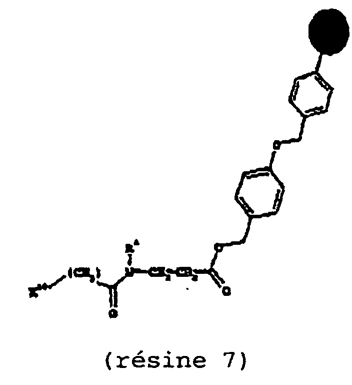 Les groupes R⁴ et R¹¹ sont définis dans la revendication 1. 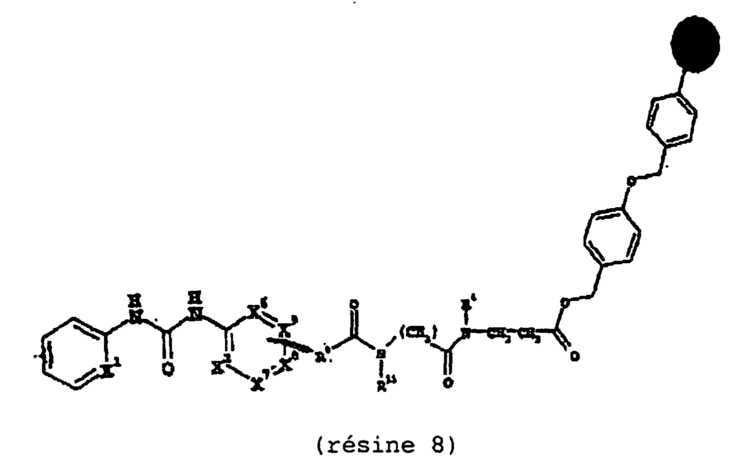 Les groupes sont définis pour la résine 2, R¹¹ étant défini dans la revendication 1.
